# NEW EUROPEAN PATENT SPECIFICATION

(11) **EP 3 254 113 B2**
(45) Date of publication and mention of the opposition decision: **28.10.2026**
(45) Mention of the grant of the patent: 09.10.2024
(21) Application number: 16747366.9
(22) Date of filing: 05.02.2016
(51) Int. Cl.: G01N 33/58, G01N 33/566

(54) **LUCIFERASE-BASED THERMAL SHIFT ASSAYS**
AUF LUCIFERASE BASIERENDE WÄRMEVERSCHIEBUNGSASSAYS
ANALYSES À VARIATION THERMIQUE FONDÉES SUR LA LUCIFÉRASE

(30) Priority: 05.02.2015 US 201562112518 P
(43) Date of publication of application: 13.12.2017
(60) Divisional application: 24198006.9 / 4 455 668
(73) Proprietor: Promega Corporation, Madison, WI 53711 (US)
(72) Inventor: DART, Melanie, Madison, Wisconsin 53711 (US); ENCELL, Lance P., Madison, Wisconsin 53711 (US); KIRKLAND, Thomas, Madison, Wisconsin 53711 (US); MACHLEIDT, Thomas, Madison, Wisconsin 53711 (US); ROBERS, Matthew, Madison, Wisconsin 53711 (US); BINKOWSKI, Brock F., Madison, Wisconsin 53711 (US); WOOD, Keith, Madison, Wisconsin 53711 (US); SHI, Ce, Madison, Wisconsin 53711 (US)
(74) Representative: Potter Clarkson
(86) International application number: PCT/US2016/016846
(87) International publication number: WO 2016/127100

(56) References cited:
- WO-A1-2014/151736
- WO-A1-2016/028804
- US-A- 5 585 277
- US-A1- 2002 068 298
- US-A1- 2006 211 007
- US-A1- 2007 086 949
- US-A1- 2010 281 552
- US-A1- 2014 057 368
- US-A1- 2014 194 307
- ROZBEH JAFARI ET AL: "The cellular thermal shift assay for evaluating drug target interactions in cells", NATURE PROTOCOLS, vol. 9, no. 9, 7 August 2014 (2014-08-07), pages 2100 - 2122, XP055193929, ISSN: 1754-2189, DOI: 10.1038/nprot.2014.138
- DANIEL MARTINEZ MOLINA ET AL: "Monitoring drug target engagement in cells and tissues using the cellular thermal shift assay", SCIENCE, vol. 341, no. 6141, 23 May 2013 (2013-05-23), US, pages 84 - 87, XP055471723, ISSN: 0036-8075, DOI: 10.1126/science.1233606
- MATTHEW B. ROBERS ET AL: "Target engagement and drug residence time can be observed in living cells with BRET", NATURE COMMUNICATIONS, vol. 6, 3 December 2015 (2015-12-03), pages 10091, XP055271302, DOI: 10.1038/ncomms10091
- MELANIE L. DART ET AL: "Homogeneous Assay for Target Engagement Utilizing Bioluminescent Thermal Shift", ACS MEDICINAL CHEMISTRY LETTERS, 19 April 2018 (2018-04-19), XP055475102, ISSN: 1948-5875, DOI: 10.1021/acsmedchemlett.8b00081

## Description

### FIELD

Provided herein are methods for characterizing target/ligand engagement In particular, luciferase-labeled polypeptide targets are used to detect or quantify target/ligand engagement (e.g., within a cell or cell lysate).

### BACKGROUND

Multiple challenges face drug development today including high costs and long development cycles for new therapeutics. Methods that promote accelerated drug development are urgently needed. The efficacy of therapeutics is dependent on a drug binding to its target. Due to its simplicity, a protein thermal shift assay (TSA) is a commonly used method for screening libraries and validating hits in drug discovery programs. The most common method of TSA in use today can only be performed using purified protein, which has several disadvantages. Recently, the cellular thermal shift assay (CETSA) was developed to detect endogenous target protein within cells or cell lysates, thereby alleviating the need to create purified proteins and allowing for target engagement analysis in complex environments that are more biologically relevant. However, the CETSA is a multi-step assay in which the analysis of target engagement relies upon western blot or AlphaScreen (Perkin Elmer) technologies, both of which have several disadvantages, e.g., cost, insensitive, multi-stepped protocols, requires cell lysis and spin steps, low throughput (e.g., Western blot), dependent upon antibody recognition of native protein state, cell line optimization, requires a large amount of cells, etc. What is needed is a simple, homogeneous, rapid, and inexpensive TSA, whereby target engagement can be characterized in complex environments, such as cells and cell lysate.

### SUMMARY

Provided herein are methods for characterizing target/ligand engagement. In particular, luciferase-labeled polypeptide targets are used to detect or quantify target/ligand engagement (e.g., within a cell, e.g., in an intact live cell, or cell lysate).

The invention provides a method to detect a target/ligand interaction, comprising the steps of: (a) incubating a fusion of a target protein and a bioluminescent reporter polypeptide: (i) in the presence of a ligand to produce a test sample and (ii) in the absence of a ligand to produce a control sample; (b) treating said test and control samples under conditions that cause the target protein to unfold to an appropriate extent; (c) measuring signal from the reporter polypeptide in said test and control samples; and (d) comparing the measurement made in step (c) between the test and control samples, wherein increase of the signal from said reporter polypeptide in the test sample compared to the control sample indicates the presence of a target/ligand interaction, and wherein the bioluminescent reporter has 70% or greater sequence identity with SEQ ID NO: 1. In some embodiments, the fusion is within a cell, e.g., a live, intact cell, cell lysate, or reaction mixture. In some embodiments, the fusion is expressed within the cell, e.g., a live intact cell, cell lysate, or reaction mixture. In some embodiments, the ligand is added exogenously to the cell, e.g., a live intact cell, cell lysate, or reaction mixture. In some embodiments, the luciferase is a variant *Oplophorus gracilirostris* luciferase (OgLuc) with 70% or greater sequence identity with SEQ ID NO: 1 (e.g., >60%, 70%, 80%, 90%, or 95% identity with SEQ ID NO: 1, SEQ ID NO: 2, etc.). In some embodiments, the bioluminescent reporter is a peptide or polypeptide tag that forms a bioluminescent complex upon interaction with a complement polypeptide or peptide (See, e.g., U.S. Pub. No. 2014/0348747). In some embodiments, the complement polypeptide or peptide is added exogenously to the test and control samples prior to step (c). In some embodiments, the complement polypeptide or peptide is expressed within the cell, cell lysate, or reaction mixture. In some embodiments, the conditions that cause the target protein to unfold to an appropriate extent comprise elevated temperature, increased pressure, and/or a denaturant. In some embodiments, elevated temperature comprises one or more temperatures above physiologic temperature for the protein in question. In some embodiments, elevated temperature comprises one or more temperatures near (e.g., +/- 1, 2, 3, 4, 5, 10, 15, 20°C) the approximate melting temperature of the target protein. In some embodiments, a plurality of test samples is produced using a plurality of test ligands.

The invention provides a method to detect a target/ligand interaction, comprising the steps of: (a) incubating a fusion of a target protein and a bioluminescent reporter: (i) in the presence of a ligand to produce a test sample, and (ii) in the absence of a ligand, to produce a control sample; (b) contacting the test and control samples with a hydrophobic dye that binds nonspecifically to aggregated, unfolded, or denatured proteins and/or hydrophobic peptide segments, wherein the emission spectra of the bioluminescent reporter upon interaction with a substrate and the excitation spectra of the hydrophobic dye that binds nonspecifically to aggregated, unfolded, or denatured proteins and/or hydrophobic peptide segments overlap; (c) treating said test and control samples under conditions that cause the target protein to unfold to an appropriate extent; (d) exposing the test and control samples to the substrate of the bioluminescent reporter; (e) measuring signal from the hydrophobic dye that binds nonspecifically to aggregated, unfolded, or denatured proteins and/or hydrophobic peptide segments in said test and control samples (e.g., emission resulting from energy transfer from the active bioluminescent reporter after addition of substrate); and (f) comparing the measurement made in step (e) between the test and control samples, wherein decrease of the signal from said hydrophobic dye that binds nonspecifically to aggregated, unfolded, or denatured proteins and/or hydrophobic peptide segments in the test sample compared to the control sample indicates the presence of a target/ligand interaction, and wherein the bioluminescent reporter has 70% or greater sequence identity with SEQ ID NO: 1. In some embodiments, steps (a), (b), and (c) are performed in any suitable order (e.g., a-b-c, a-c-b, b-a-c, b-c-a, c-a-b, or c-b-a). In some embodiments, the fusion is within a cell, e.g., a live intact cell, cell lysate, or reaction mixture. In some embodiments, the fusion is expressed within the cell, e.g., a live intact cell, cell lysate, or reaction mixture. In some embodiments, the ligand is added exogenously to the cell, e.g., a live intact cell, cell lysate, or reaction mixture. In some embodiments, the hydrophobic dye is added exogenously to the cell, e.g., a live intact cell, cell lysate, or reaction mixture. In some embodiments, the luciferase is a variant *Oplophorus gracilirostris* luciferase (OgLuc) with 70% or greater sequence identity with SEQ ID NO: 1 (e.g., >60%, 70%, 80%, 90%, or 95% identity with SEQ ID NO: 1, SEQ ID NO: 2, etc.). In some embodiments, the bioluminescent reporter is a peptide or polypeptide tag that forms a bioluminescent complex upon interaction with a complement polypeptide or peptide. In some embodiments, the complement polypeptide or peptide is added exogenously to the test and control samples prior to step (d). In some embodiments, the complement polypeptide or peptide is expressed within the cell, e.g., a live intact cell, cell lysate, or reaction mixture. In some embodiments, the conditions that cause the target protein to unfold to an appropriate extent comprise elevated temperature, increased pressure, and/or a denaturant. In some embodiments, elevated temperature comprises one or more temperatures above physiologic temperature. In some embodiments, elevated temperature comprises one or more temperatures near the approximate melting temperature of the target protein. In some embodiments, the hydrophobic dye binds nonspecifically to hydrophobic surfaces. In some embodiments, the hydrophobic dye binds preferentially to the folded, unfolded, or molten globule states of the protein. In some embodiments, the fluorescence of the hydrophobic dye is quenched by water. In some embodiments, the hydrophobic dye is Sypro Orange, SYPRO Red, Nile Red, ANS, Bis-ANS, SYPRO Ruby, SYPRO Tangerine, and/or Dapoxyl Sulfonic Acid Sodium Salt, Protein Thermal Shift^{™} Dye (Life Technologies), Proteostat^{™} Dye (Enzo), or combinations thereof. Other suitable dyes for use in embodiments described herein include, but are not limited to: styryl dyes, asymmetric cyanines, oxazole dyes (Dapoxyls), azo dyes, and other dyes such as Thioflavin T and (Dicyanovinyl)julolidine (DCVJ). Suitable dyes for use in embodiments described herein are also described in, for example: Kovalska et al. Dyes and Pigments 67 (2005) 47-54.; Volkova et al. Bioorganic & Medicinal Chemistry 16 (2008) 1452-1459.; Volkova et al. J. Biochem. Biophys. Methods 70 (2007) 727-733.; Hawe et al. Pharmaceutical Research, Vol. 25, No. 7, July 2008.; U.S. Pub. No. 2011/0130305; PCT Pub. WO 2006/079334; Diwu et al. Photochemistry and Photobiology, 1997, 66(4): 424-431..

The invention provides a method of screening a group of test ligands for binding to a target protein comprising: (a) creating a plurality of test samples each comprising at least one test ligand and a fusion of a target protein and a bioluminescent reporter; (b) creating at least one control sample comprising a fusion of a target protein and a bioluminescent reporter in the absence of a test ligand; (c) contacting the test and control samples with a hydrophobic dye that binds nonspecifically to aggregated, unfolded, or denatured proteins and/or hydrophobic peptide segments, wherein the emission spectra of the bioluminescent reporter upon interaction with a substrate and the excitation spectra of the hydrophobic dye that binds nonspecifically to aggregated, unfolded, or denatured proteins and/or hydrophobic peptide segments overlap; (d) treating the test and control samples under conditions that cause the target protein to unfold to an appropriate extent; (e) exposing the test and control samples to the substrate of the bioluminescent reporter; (f) measuring signal from the hydrophobic dye that binds nonspecifically to aggregated, unfolded, or denatured proteins and/or hydrophobic peptide segments in said test and control samples; and (g) comparing the measurement made in step (f) between the test and control samples, wherein decrease of the signal from said hydrophobic dye that binds nonspecifically to aggregated, unfolded, or denatured proteins and/or hydrophobic peptide segments in one or more test samples compared to the control sample indicates the presence of a target/ligand interaction, and wherein the bioluminescent reporter has 70% or greater sequence identity with SEQ ID NO: 1. In some embodiments, multiple assays, each comprising a different test ligand of set of test ligands are performed in parallel (e.g., in a high throughput method). Fluorescence emission from the fluorescent dye as a result of BRET from the bioluminescent reporter is detected. Decrease of BRET-induced fluorescence in the presence of the one or more test ligands, and/or an increase in the temperature or amount of denaturant required to generate a BRET-induced fluorescent signal, indicates interaction of one or more of the test ligands with the target protein.

The invention provides a method to detect a target/ligand interaction, comprising the steps of: (a) incubating a fusion of a target protein and a bioluminescent reporter polypeptide in the presence of a ligand to produce a test sample; (b) treating said test sample under conditions that cause the target protein to unfold to an appropriate extent; (c) measuring signal from the reporter polypeptide in said test sample; and (d) detecting target/ligand interaction based on the signal from said reporter polypeptide in the test sample, and wherein the difference or ratio of the signal is compared to a difference or ratio of signal in a control sample without the ligand, and wherein the bioluminescent reporter has 70% or greater sequence identity with SEQ ID NO: 1.

The invention provides a method to detect a target/ligand interaction, comprising the steps of: (a) incubating a fusion of a target protein and a bioluminescent reporter in the presence of a ligand to produce a test sample; (b) contacting the test sample with a hydrophobic dye that binds nonspecifically to aggregated, unfolded, or denatured proteins and/or hydrophobic peptide segments, wherein the emission spectra of the bioluminescent reporter and the excitation spectra of the hydrophobic dye that binds nonspecifically to aggregated, unfolded, or denatured proteins and/or hydrophobic peptide segments overlap; (c) treating the test sample under conditions that cause the target protein to unfold to an appropriate extent; (d) exposing the test sample to the substrate of the bioluminescent reporter; (e) measuring signal from the hydrophobic dye that binds nonspecifically to aggregated, unfolded, or denatured proteins and/or hydrophobic peptide segments in said test sample; and (f) detecting target/ligand interaction based on the signal from said hydrophobic dye that binds nonspecifically to aggregated, unfolded, or denatured proteins and/or hydrophobic peptide segments in the test sample, and wherein the difference or ratio of the signal is compared to a difference or ratio of signal in a control sample without the ligand, and wherein the bioluminescent reporter has 70% or greater sequence identity with SEQ ID NO: 1.

Provided herein are methods for the detection of target/ligand interactions within a cell. In some embodiments, methods allow detection of target/ligand interactions within live, intact cells. In some embodiments, all steps (e.g., target/reporter expression, reagent (e.g., ligand, substrate, dye, etc.) addition, target denaturation, signal detection, etc.) are performed to or within the live, intact cells. In some embodiments, the target/ligand interaction is initiated within the live, intact cells, but one or more steps of the assay (e.g., substrate addition, target denaturation, signal detection, etc.) are performed in a lysate of the cells (e.g., following lysis of the live, intact cells to produce a cell lysate). In some embodiments, all steps (e.g., target/reporter expression, reagent (e.g., ligand, substrate, dye, etc.) addition, target denaturation, signal detection, etc.) are performed to or within the cell lysate.

The invention provides a method to detect a target/ligand interaction within a live, intact cell, comprising the steps of: (a) providing a live, intact cell expressing a fusion of a target protein and a bioluminescent reporter; (b) allowing time for the target protein to interact with the ligand within the live, intact cell; (c) contacting the live, intact cell with an hydrophobic dye that binds nonspecifically to aggregated, unfolded, or denatured proteins and/or hydrophobic peptide segments, wherein the emission spectra of the bioluminescent reporter and the excitation spectra of the hydrophobic dye that binds nonspecifically to aggregated, unfolded, or denatured proteins and/or hydrophobic peptide segments overlap; (d) treating the live, intact cell under conditions that cause the target protein to unfold to an appropriate extent; (e) exposing the live, intact cell to the substrate of the bioluminescent reporter; (f) measuring signal from the hydrophobic dye that binds nonspecifically to aggregated, unfolded, or denatured proteins and/or hydrophobic peptide segments and bioluminescent reporter in the live, intact cell; and (g) detecting target/ligand interaction based on the signals from said hydrophobic dye that binds nonspecifically to aggregated, unfolded, or denatured proteins and/or hydrophobic peptide segments and bioluminescent reporter in the live, intact cell, and wherein the signal from said hydrophobic dye in the live, intact cell is compared to a control sample without the ligand, and wherein the bioluminescent reporter has 70% or greater sequence identity with SEQ ID NO: 1. In some embodiments, methods further comprise a step of adding the ligand to the live, intact cell. In some embodiments, the ligand is endogenous to the live, intact cell.

The invention provides a method to detect a target/ligand interaction within a cell, comprising the steps of: (a) providing a live, intact cell expressing a fusion of a target protein and a bioluminescent reporter; (b) allowing time for the target protein to interact with the ligand within the live, intact cell; (c) lysing the cell to produce a cell lysate; (d) contacting the cell lysate with a hydrophobic dye that binds nonspecifically to aggregated, unfolded, or denatured proteins and/or hydrophobic peptide segments, wherein the emission spectra of the bioluminescent reporter and the excitation spectra of the hydrophobic dye that binds nonspecifically to aggregated, unfolded, or denatured proteins and/or hydrophobic peptide segments overlap; (e) treating the cell lysate under conditions that cause the target protein to unfold to an appropriate extent; (f) exposing the cell lysate to the substrate of the bioluminescent reporter; (g) measuring signal from the hydrophobic dye that binds nonspecifically to aggregated, unfolded, or denatured proteins and/or hydrophobic peptide segments and bioluminescent reporter in the cell lysate; and (h) detecting target/ligand interaction based on the signals from said hydrophobic dye that binds nonspecifically to aggregated, unfolded, or denatured proteins and/or hydrophobic peptide segments and bioluminescent reporter in the cell lysate, and wherein the signal from said hydrophobic dye in the cell lysate is compared to a control sample without the ligand, and wherein the bioluminescent reporter has 70% or greater sequence identity with SEQ ID NO: 1 . In some embodiments, methods further comprise a step of adding the ligand to the live, intact cell. In some embodiments, the ligand is endogenous to the live, intact cell.

The invention provides a method to detect a target/ligand interaction within a cell, comprising the steps of: (a) providing a live, intact cell expressing a fusion of a target protein and a bioluminescent reporter; (b) allowing time for the target protein to interact with the ligand within the live, intact cell; (c) contacting the live, intact cell with an hydrophobic dye that binds nonspecifically to aggregated, unfolded, or denatured proteins and/or hydrophobic peptide segments, wherein the emission spectra of the bioluminescent reporter upon interaction with a substrate and the excitation spectra of the hydrophobic dye that binds nonspecifically to aggregated, unfolded, or denatured proteins and/or hydrophobic peptide segments overlap; (d) lysing the cell to produce a cell lysate; (e) treating the cell lysate under conditions that cause the target protein to unfold to an appropriate extent; (f) exposing the cell lysate to the substrate of the bioluminescent reporter; (g) measuring signal from the hydrophobic dye that binds nonspecifically to aggregated, unfolded, or denatured proteins and/or hydrophobic peptide segments and bioluminescent reporter in the cell lysate; and (h) detecting target/ligand interaction based on the r signals from said hydrophobic dye that binds nonspecifically to aggregated, unfolded, or denatured proteins and/or hydrophobic peptide segments and bioluminescent reporter in the cell lysate, and wherein the signal from said hydrophobic dye in the cell lysate is compared to a control sample without the ligand, and wherein the bioluminescent reporter has 70% or greater sequence identity with SEQ ID NO: 1. In some embodiments, methods further comprise a step of adding the ligand to the live, intact cell. In some embodiments, the ligand is endogenous to the live, intact cell.

The invention provides a method to detect a target/ligand interaction within a live, intact cell, comprising the steps of: (a) providing a live, intact cell expressing a fusion of a target protein and a bioluminescent reporter; (b) allowing time for the target protein to interact with the ligand within the live, intact cell; (c) contacting the live, intact cell with an hydrophobic dye that binds nonspecifically to aggregated, unfolded, or denatured proteins and/or hydrophobic peptide segments, wherein the emission spectra of the bioluminescent reporter upon interaction with a substrate and the excitation spectra of the hydrophobic dye that binds nonspecifically to aggregated, unfolded, or denatured proteins and/or hydrophobic peptide segments overlap; (d) exposing the live, intact cell to the substrate of the bioluminescent reporter; (e) measuring signal from the hydrophobic dye that binds nonspecifically to aggregated, unfolded, or denatured proteins and/or hydrophobic peptide segments and bioluminescent reporter in the live, intact cell under pre-denaturing conditions; (f) treating the live, intact cell under conditions that cause the target protein to unfold to an appropriate extent; (g) measuring signal from the hydrophobic dye that binds nonspecifically to aggregated, unfolded, or denatured proteins and/or hydrophobic peptide segments and bioluminescent reporter in the live, intact cell under post-denaturing conditions; and (h) detecting target/ligand interaction based on the difference or ratio in signals from said hydrophobic dye and bioluminescent reporter under the pre-denaturing and post-denaturing conditions in the live, intact cell, wherein the hydrophobic dye binds nonspecifically to aggregated, unfolded, or denatured proteins and/or hydrophobic peptide segments, and wherein the difference or ratio of the signal is compared to a difference or ratio of signal in a control sample without the ligand, and wherein the bioluminescent reporter has 70% or greater sequence identity with SEQ ID NO: 1. In some embodiments, methods further comprise a step of adding the ligand to the live, intact cell prior to step (e). In some embodiments, the ligand is endogenous to the live, intact cell.

Provided herein are methods to detect target/ligand interactions within live, intact cells using luminescence-based readout. The invention provides a method to detect a target/ligand interaction within a live, intact cell comprising the steps of: (a) providing a live, intact cell expressing a fusion of a target protein and a bioluminescent reporter; (b) allowing time for the target protein to interact with the ligand within the live, intact cell; (c) treating the live, intact cell under conditions that cause the target protein to unfold to an appropriate extent; (d) exposing the live, intact cell to the substrate of the bioluminescent reporter; (e) measuring signal from the bioluminescent reporter in the live, intact cell; and (f) detecting target/ligand interaction based on the signal from said bioluminescent reporter in the live, intact cell, and wherein the difference or ratio of the signal is compared to a difference or ratio of signal in a control sample without the ligand, and wherein the bioluminescent reporter has 70% or greater sequence identity with SEQ ID NO: 1.

Provided herein are methods to detect target/ligand interactions in live cells using lytic endpoint luminescence-based readout. The invention provides a method to detect a target/ligand interaction within a live, intact cell comprising the steps of: (a) providing a live, intact cell expressing a fusion of a target protein and a bioluminescent reporter; (b) allowing time for the target protein to interact with the ligand within the live, intact cell; (c) treating the live, intact cell under conditions that cause the target protein to unfold to an appropriate extent; (d) lysing the cell to produce a cell lysate; (e) exposing the cell lysate to the substrate of the bioluminescent reporter; (f) measuring signal from the bioluminescent reporter in the lysate; and (g) detecting target/ligand interaction based on the signal from said bioluminescent reporter in the lysate, and wherein the difference or ratio of the signal is compared to a difference or ratio of signal in a control sample without the ligand, and wherein the bioluminescent reporter has 70% or greater sequence identity with SEQ ID NO: 1.

Provided herein are methods to detect target/ligand interactions within a cell lysate using luminescence-based readout. The invention provides a method to detect a target/ligand interaction within a live, intact cell comprising the steps of: (a) providing a live, intact cell expressing a fusion of a target protein and a bioluminescent reporter; (b) lysing the cell to produce a cell lysate; (c) allowing time for the target protein to interact with the ligand within the cell lysate; (d) treating the cell lysate under conditions that cause the target protein to unfold to an appropriate extent; (e) exposing the cell lysate to the substrate of the bioluminescent reporter; (f) measuring signal from the bioluminescent reporter in the lysate; and (g) detecting target/ligand interaction based on the signal from said bioluminescent reporter in the lysate, and wherein the difference or ratio of the signal is compared to a difference or ratio of signal in a control sample without the ligand, and wherein the bioluminescent reporter has 70% or greater sequence identity with SEQ ID NO: 1.

Provided herein are methods to detect target/ligand interactions in live, intact cells using luminescence-based readout with ratiometric analysis. The invention provides a method to detect a target/ligand interaction within a live, intact cell comprising the steps of: (a) providing a live, intact cell expressing a fusion of a target protein and a bioluminescent reporter; (b) allowing time for the target protein to interact with the ligand within the live, intact cell; (c) exposing the live, intact cell to the substrate of the bioluminescent reporter; (d) measuring signal from the bioluminescent reporter in the live, intact cell under pre-denaturing conditions; (e) treating the live, intact cell under conditions that cause the target protein to unfold to an appropriate extent; (f) measuring signal from the bioluminescent reporter in the live, intact cell under post-denaturing conditions; and (g) detecting target/ligand interaction based on the difference or ratio in signal from the bioluminescent reporter under the pre-denaturing and post-denaturing conditions in the live, intact cell, and wherein the difference or ratio of the signal is compared to a difference or ratio of signal in a control sample without the ligand, and wherein the bioluminescent reporter has 70% or greater sequence identity with SEQ ID NO: 1.

Provided herein are methods to detect target/ligand interactions in a cell lysate using luminescence-based readout with ratiometric analysis. The invention provides a method to detect a target/ligand interaction within a live, intact cell comprising the steps of: (a) providing a live, intact cell expressing a fusion of a target protein and a bioluminescent reporter; (b) lysing the cell to produce a cell lysate; (c) allowing time for the target protein to interact with the ligand within the cell lysate; (d) exposing the cell lysate to the substrate of the bioluminescent reporter; (e) measuring signal from the bioluminescent reporter in the cell lysate under pre-denaturing conditions; (f) treating the cell lysate under conditions that cause the target protein to unfold to an appropriate extent; (g) measuring signal from the bioluminescent reporter in the cell lysate under post-denaturing conditions; and (h) detecting target/ligand interaction based on the difference or ratio in signal from the bioluminescent reporter under the pre-denaturing and post-denaturing conditions in the cell lysate, and wherein the difference or ratio of the signal is compared to a difference or ratio of signal in a control sample without the ligand, and wherein the bioluminescent reporter has 70% or greater sequence identity with SEQ ID NO: 1.

Provided herein are methods to detect target/ligand interactions in live, intact cells using BRET readout with dye addition following a partial denaturation step (e.g., post-heat). The invention provides a method to detect a target/ligand interaction within a live, intact cell comprising the steps of: (a) providing a live, intact cell expressing a fusion of a target protein and a bioluminescent reporter; (b) allowing time for the target protein to interact with the ligand within the live, intact cell; (c) treating the live, intact cell under conditions that cause the target protein to unfold to an appropriate extent; (d) contacting the live, intact cell with an hydrophobic dye that binds nonspecifically to aggregated, unfolded, or denatured proteins and/or hydrophobic peptide segments, wherein the emission spectra of the bioluminescent reporter and the excitation spectra of the hydrophobic dye that binds nonspecifically to aggregated, unfolded, or denatured proteins and/or hydrophobic peptide segments overlap; (e) exposing the live, intact cell to the substrate of the bioluminescent reporter; (f) measuring signal from the hydrophobic dye that binds nonspecifically to aggregated, unfolded, or denatured proteins and/or hydrophobic peptide segments and bioluminescent reporter in the live, intact cell; and (g) detecting target/ligand interaction based on the ratiometric signal from said hydrophobic dye and bioluminescent reporter in the live, intact cell wherein the hydrophobic dye binds nonspecifically to aggregated, unfolded, or denatured proteins and/or hydrophobic peptide segments, and wherein the difference or ratio of the signal is compared to a difference or ratio of signal in a control sample without the ligand, and wherein the bioluminescent reporter has 70% or greater sequence identity with SEQ ID NO: 1.

Provided herein are methods to detect target/ligand interactions in live cells using lytic endpoint BRET readout with dye addition. The invention provides a method to detect a target/ligand interaction within a live, intact cell comprising the steps of: (a) providing a live, intact cell expressing a fusion of a target protein and a bioluminescent reporter; (b) allowing time for the target protein to interact with the ligand within the live, intact cell; (c) treating the live, intact cell under conditions that cause the target protein to unfold to an appropriate extent; (d) lysing the cell to produce a cell lysate; (e) contacting the cell lysate with an hydrophobic dye that binds nonspecifically to aggregated, unfolded, or denatured proteins and/or hydrophobic peptide segments, wherein the emission spectra of the bioluminescent reporter and the excitation spectra of the hydrophobic dye that binds nonspecifically to aggregated, unfolded, or denatured proteins and/or hydrophobic peptide segments overlap; (f) exposing the cell lysate to the substrate of the bioluminescent reporter; (g) measuring signal from the hydrophobic dye that binds nonspecifically to aggregated, unfolded, or denatured proteins and/or hydrophobic peptide segments and bioluminescent reporter in the cell lysate; and (h) detecting target/ligand interaction based on the ratiometric signal from said hydrophobic dye and bioluminescent reporter in the cell lysate, wherein the hydrophobic dye binds nonspecifically to aggregated, unfolded, or denatured proteins and/or hydrophobic peptide segments, and wherein the difference or ratio of the signal is compared to a difference or ratio of signal in a control sample without the ligand, and wherein the bioluminescent reporter has 70% or greater sequence identity with SEQ ID NO: 1.

Provided herein are methods to detect target/ligand interactions in cell lysate using BRET readout with dye addition. The invention provides a method to detect a target/ligand interaction within a live, intact cell comprising the steps of: (a) providing a live, intact cell expressing a fusion of a target protein and a bioluminescent reporter; (b) lysing the cell to produce a cell lysate; (c) contacting the cell lysate with an hydrophobic dye that binds nonspecifically to aggregated, unfolded, or denatured proteins and/or hydrophobic peptide segments, wherein the emission spectra of the bioluminescent reporter and the excitation spectra of the hydrophobic dye that binds nonspecifically to aggregated, unfolded, or denatured proteins and/or hydrophobic peptide segments overlap; (d) allowing time for the target protein to interact with the ligand within the cell lysate; (e) treating the cell lysate under conditions that cause the target protein to unfold to an appropriate extent; (f) exposing the cell lysate to the substrate of the bioluminescent reporter; (g) measuring signal from the hydrophobic dye that binds nonspecifically to aggregated, unfolded, or denatured proteins and/or hydrophobic peptide segments and bioluminescent reporter in the cell lysate; and (h) detecting target/ligand interaction based on the ratiometric signal from said hydrophobic dye and bioluminescent reporter in the cell lysate, wherein the hydrophobic dye binds nonspecifically to aggregated, unfolded, or denatured proteins and/or hydrophobic peptide segments, and wherein the difference or ratio of the signal is compared to a difference or ratio of signal in a control sample without the ligand, and wherein the bioluminescent reporter has 70% or greater sequence identity with SEQ ID NO: 1.

### BRIEF DESCRIPTION OF THE DRAWINGS

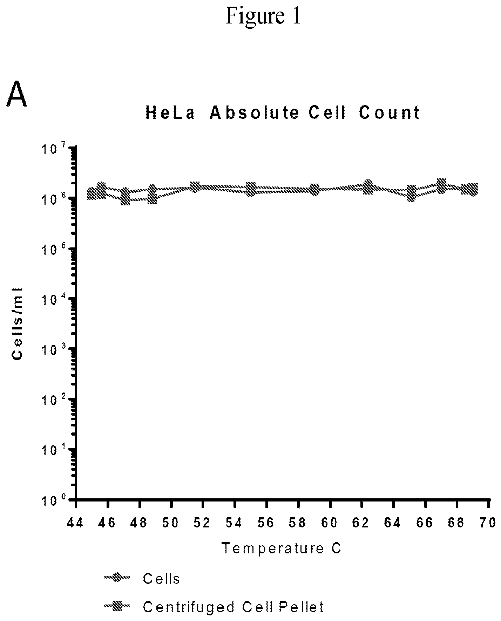

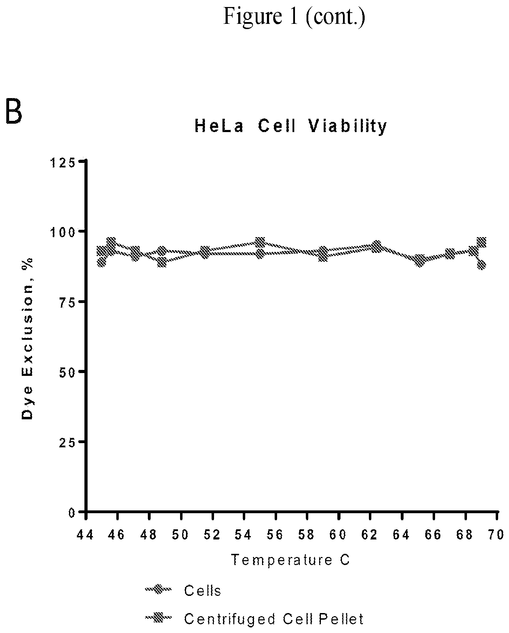

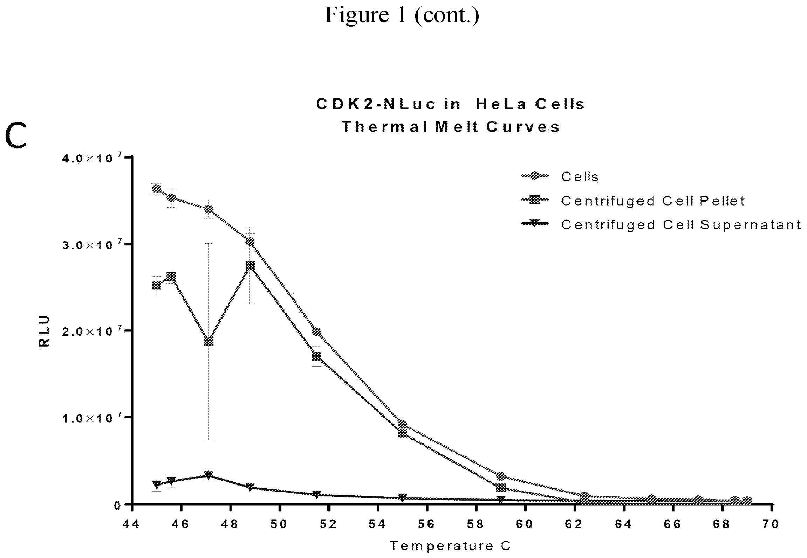

Figure 1 shows the effects of heat treatment on HeLa cell viability (Fig 1A and 1B) and NanoLuc^{®} activity (RLU) within HeLa cells after exposure to a temperature gradient (Fig. 1C).

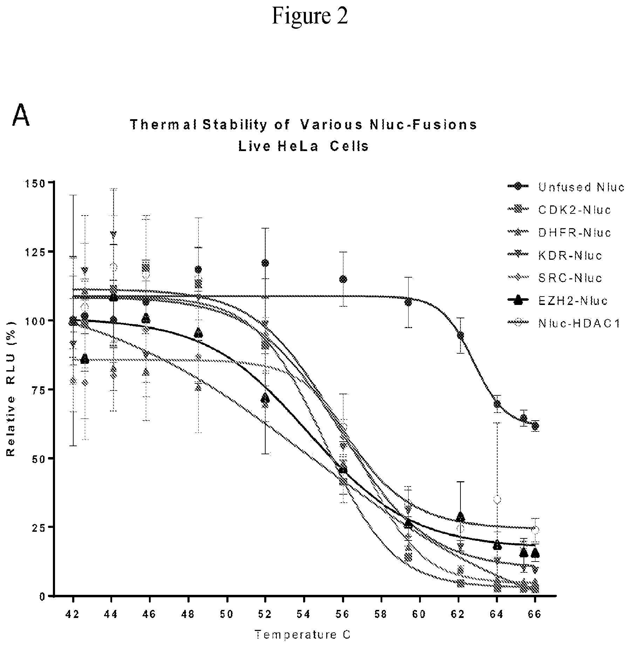

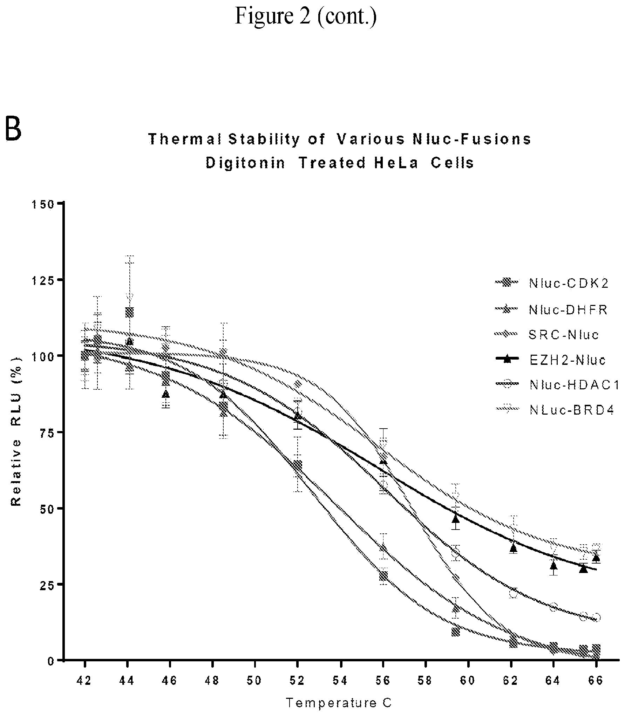

Figure 2 shows analysis of melting temperatures of several example target-NanoLuc^{®} (Nluc) fusions across a range of target classes as determined by NanoLuc^{®} activity (RLU) in live, intact cells (Fig. 2A) and digitonin-treated (Fig 2B) mammalian cells when exposed to a temperature gradient.

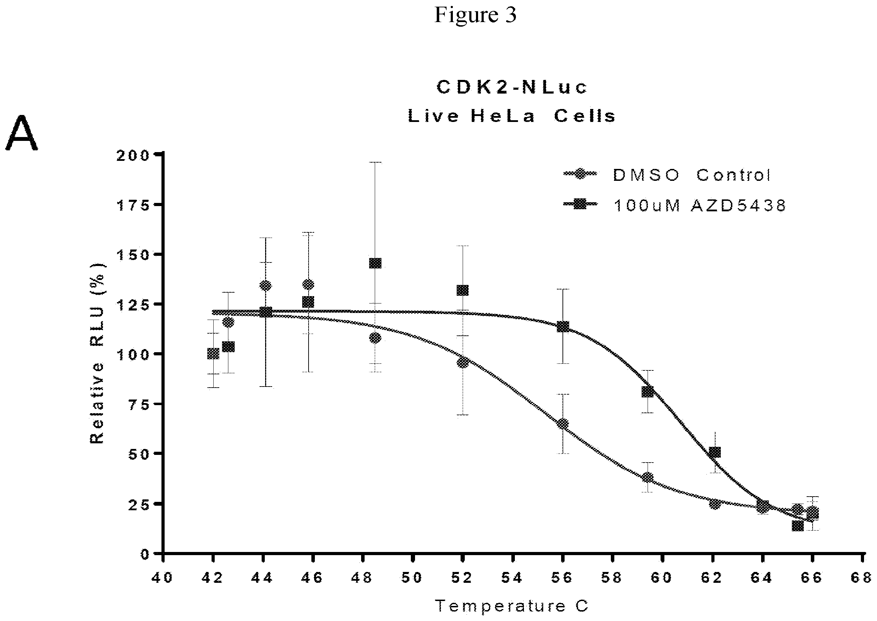

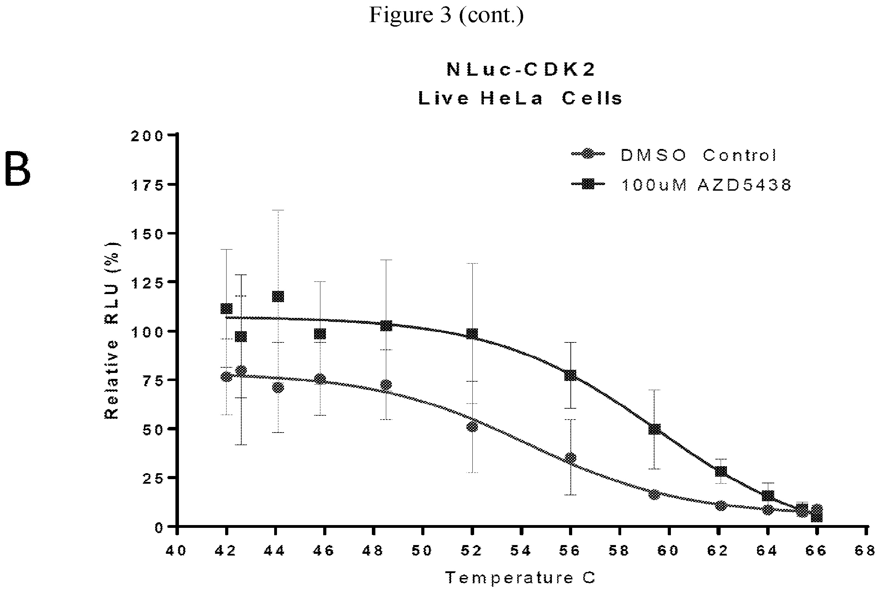

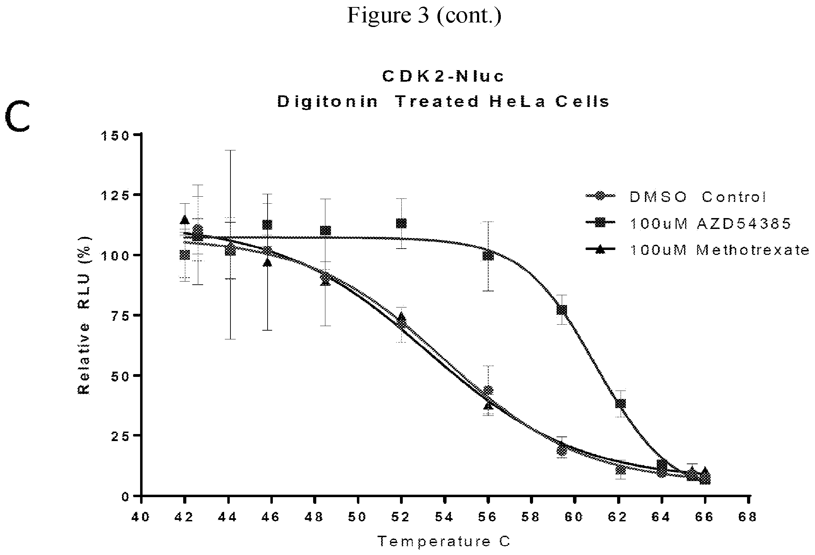

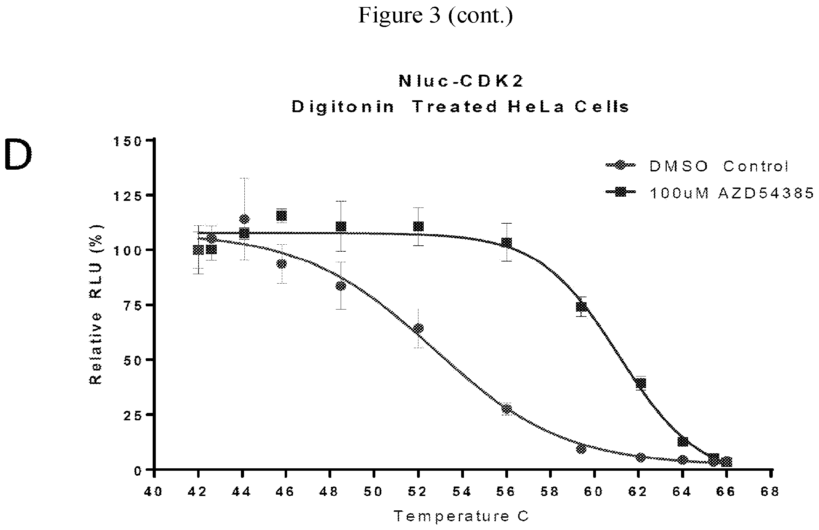

Figure 3 shows a detection of an increase in melting temperature for CDK2-Nluc and Nluc-CDK2 as determined by NanoLuc activity (RLU) after binding to a stabilizing ligand in live, intact cells (Fig. 3A) and digitonin-treated mammalian cells (Fig. 3B) and subsequently exposed to a temperature gradient.

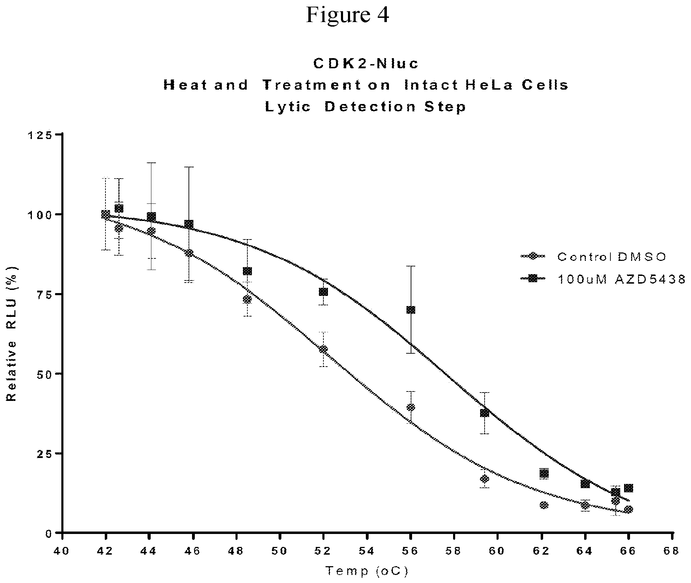

Figure 4 shows a detection of an increase in melting temperature for CDK2-Nluc as determined by NanoLuc^{®} activity (RLU) after binding to a stabilizing ligand in live mammalian cells subsequently lysed with lytic NanoGlo^{®} Detection Reagent (Promega) and subsequently exposed to a temperature gradient.

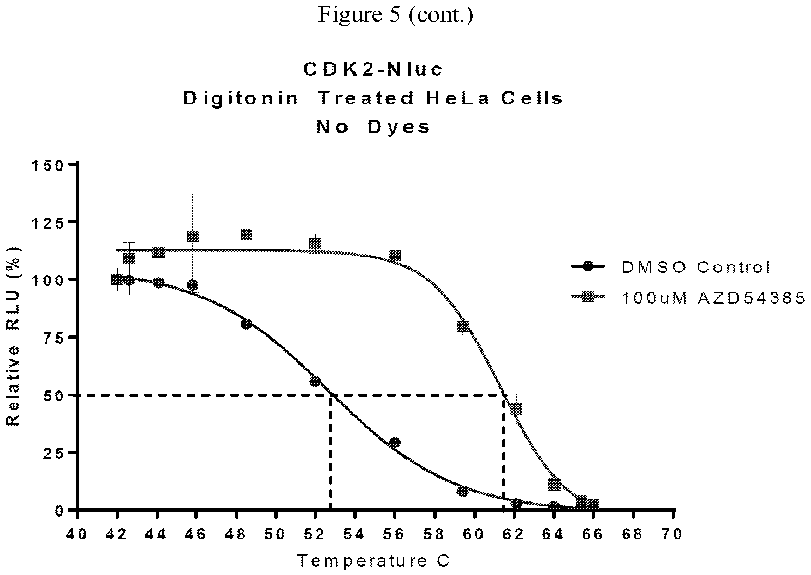

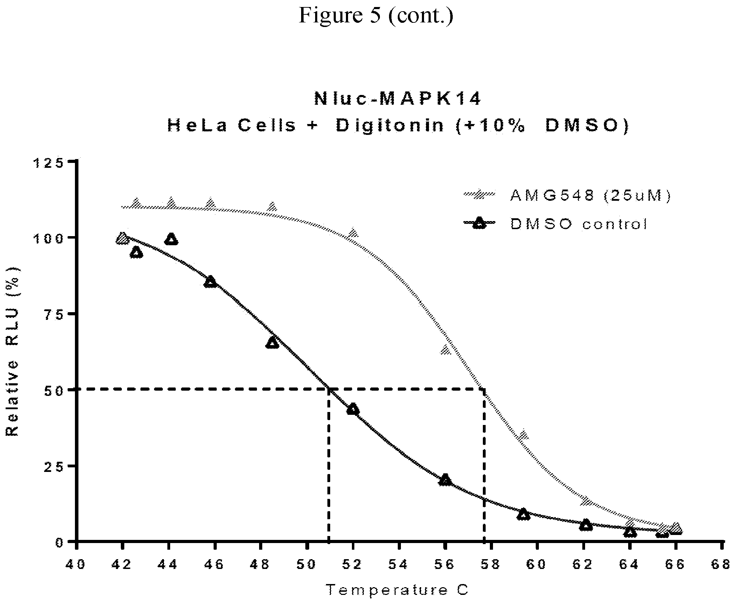

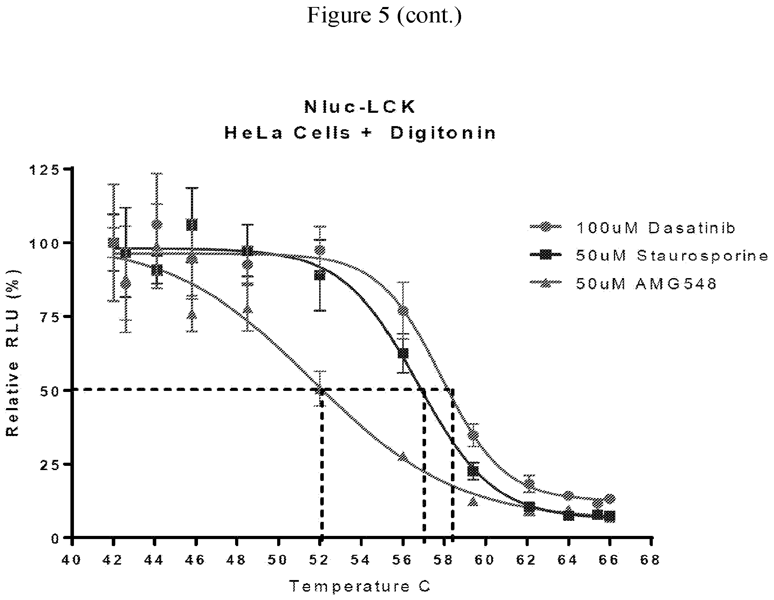

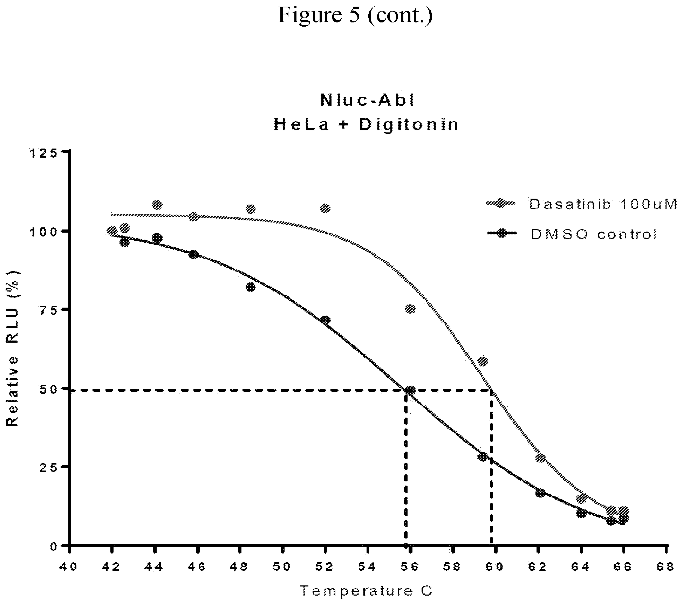

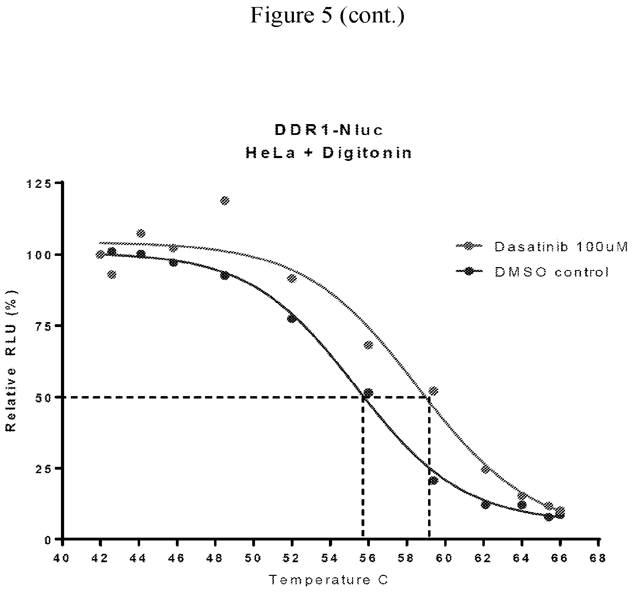

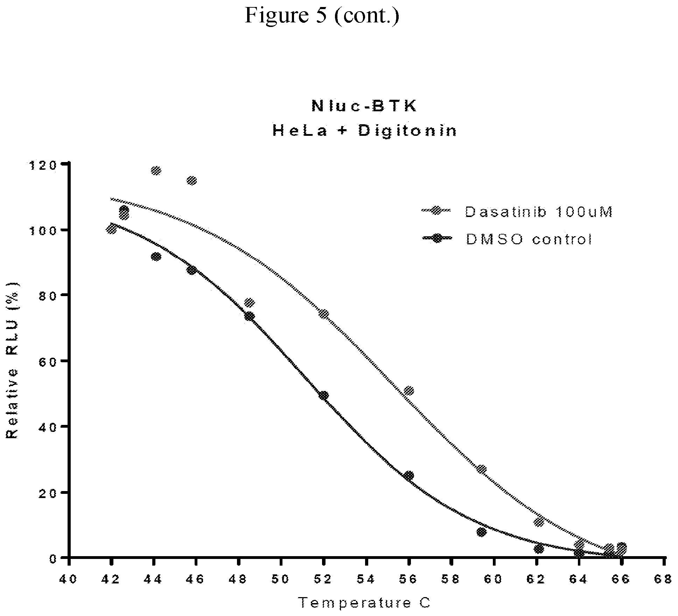

Figure 5 shows detection of an increase in melting temperature for a panel of kinases as determined by NanoLuc^{®} activity (RLU) after binding to stabilizing ligands in digitonin treated mammalian cells and subsequently exposed to a temperature gradient.

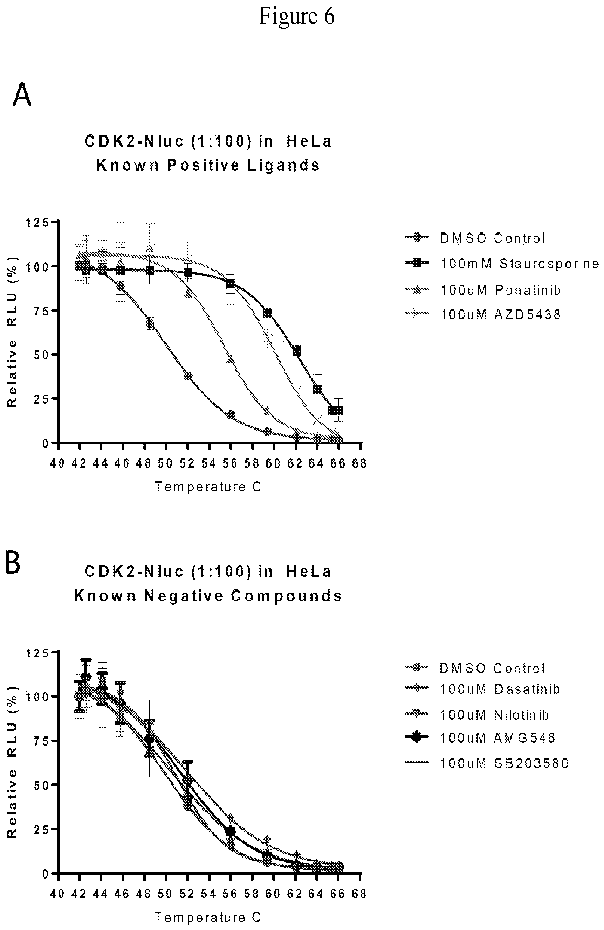

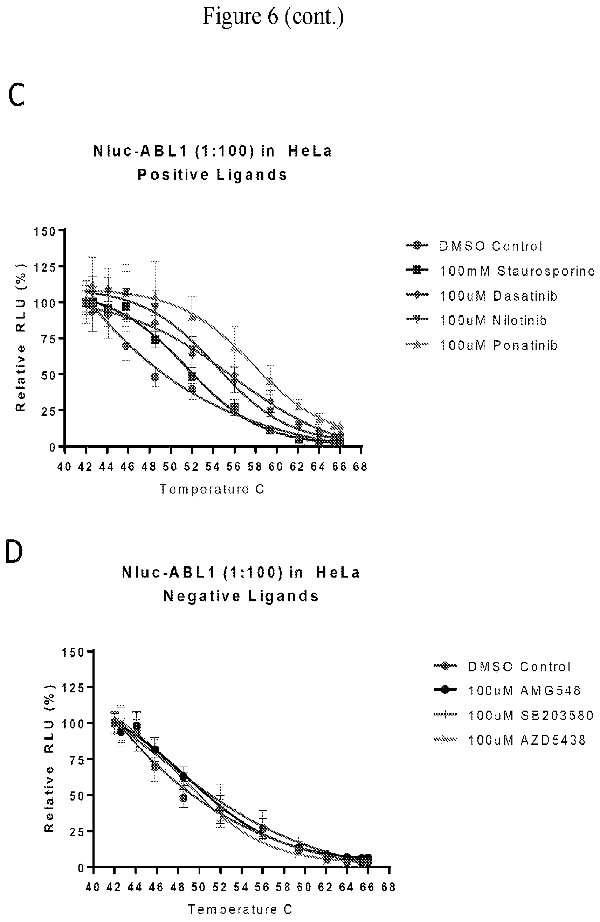

Figure 6 shows detection of an increase in melting temperatures or no change in melting temperatures for CDK2-Nluc and Nluc-ABL1 as determined by NanoLuc^{®} activity (RLU) after incubation with a panel of compounds thus displaying compound selectivity for the cytoplasmic targets in mammalian cells subsequently exposed to digitonin and a temperature gradient.

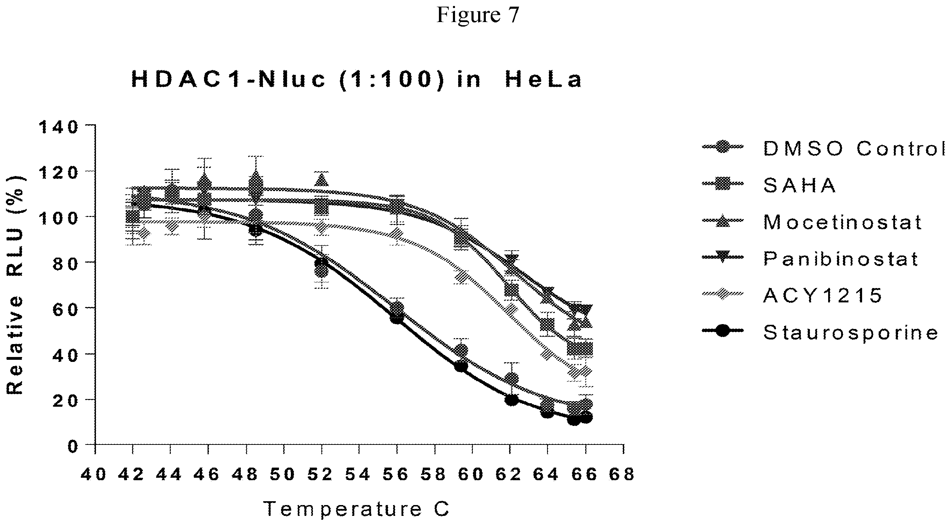

Figure 7 shows detection of an increase in melting temperatures or no change in melting temperatures for HDAC1-Nluc as determined by NanoLuc^{®} activity (RLU) after incubation with a panel of compounds thus displaying compound selectivity for the nuclear target in mammalian cells and subsequently exposed to digitonin and a temperature gradient.

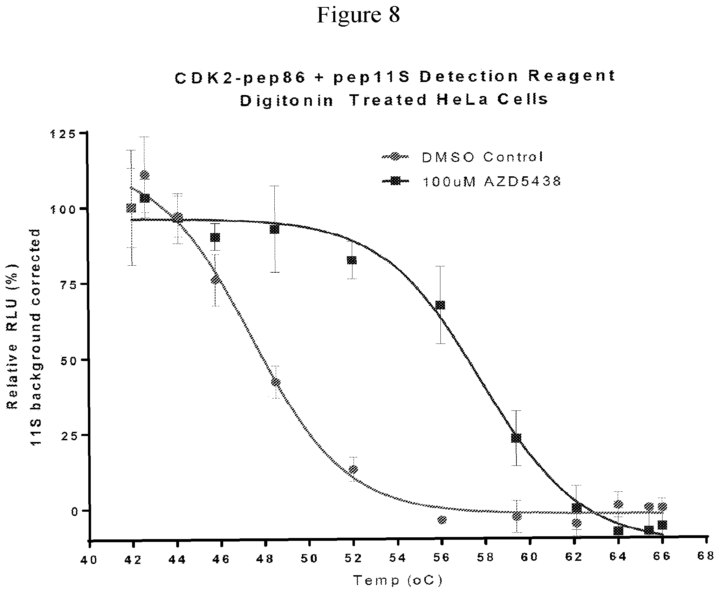

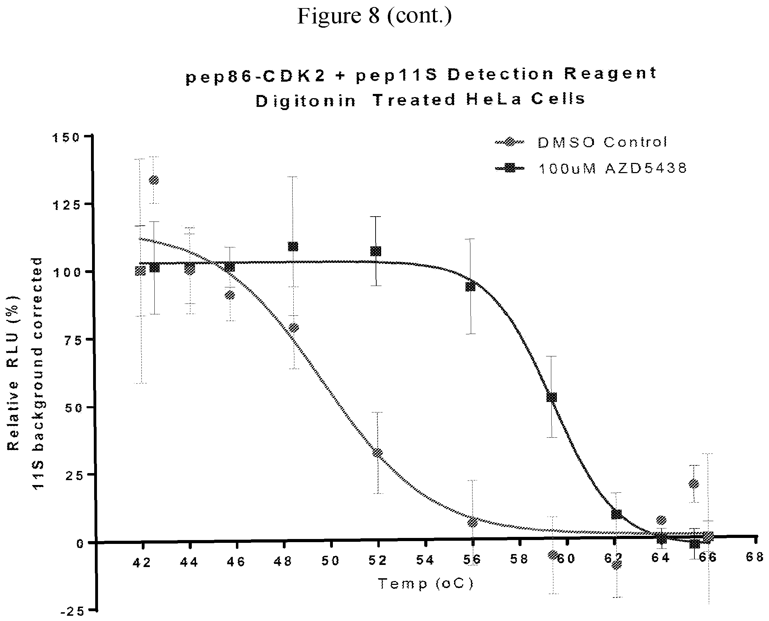

Figure 8 shows detection of an increase in melting temperature for the targets KDR-Nluc and DHFR-Nluc as determined by NanoLuc^{®} activity (RLU) after incubation with stabilizing ligand in mammalian cells subsequently harvested with the compound being washed out and then subjected to a temperature. With this compound washout step, the experiment is analyzed not under compound equilibrium conditions and has the ability to analyze binding kinetics. KDR is a membrane protein highlighting another subcellular location that the assay is able to monitor. This assay was performed with live, intact cells throughout the whole assay conditions.

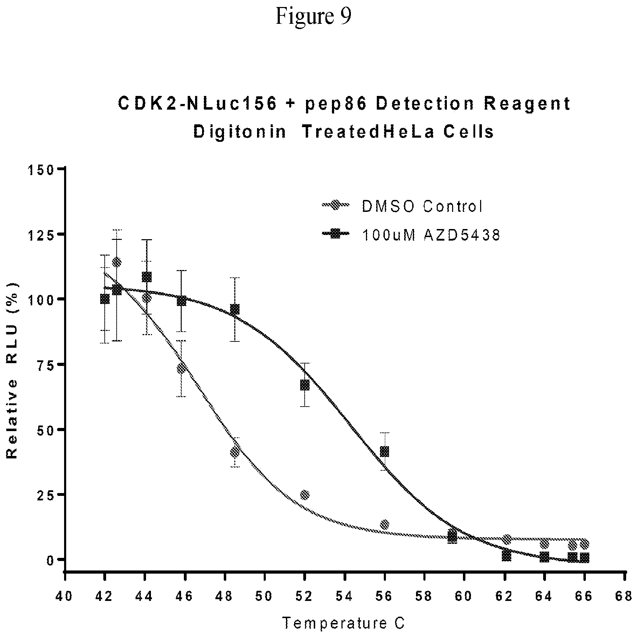

Figure 9 shows detection of an increase in melting temperature for CDK2-pep86 and pep86-CDK2 as determined by luciferase activity through spontaneous binary complementation after incubation in the presence of a stabilizing ligand in digitonin-treated mammalian cells subsequently exposed to a temperature gradient.

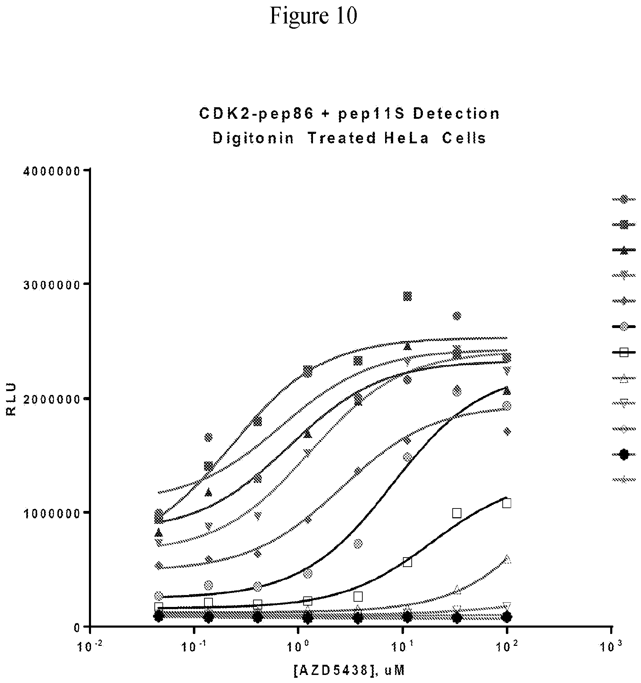

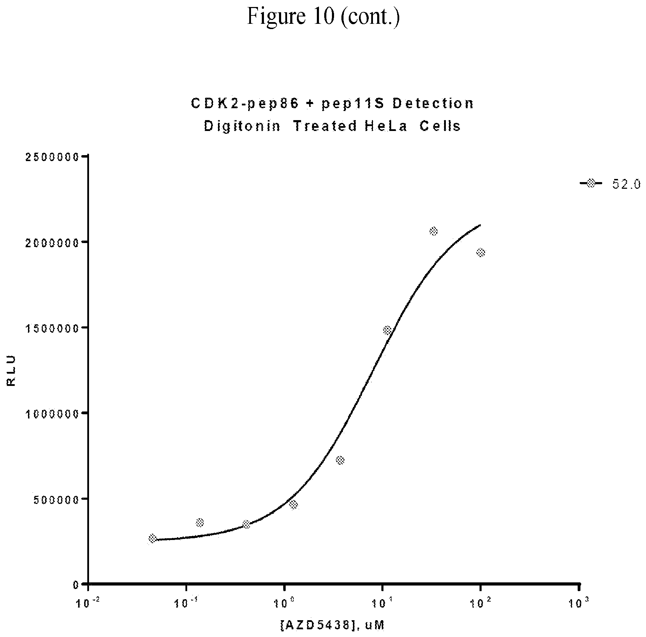

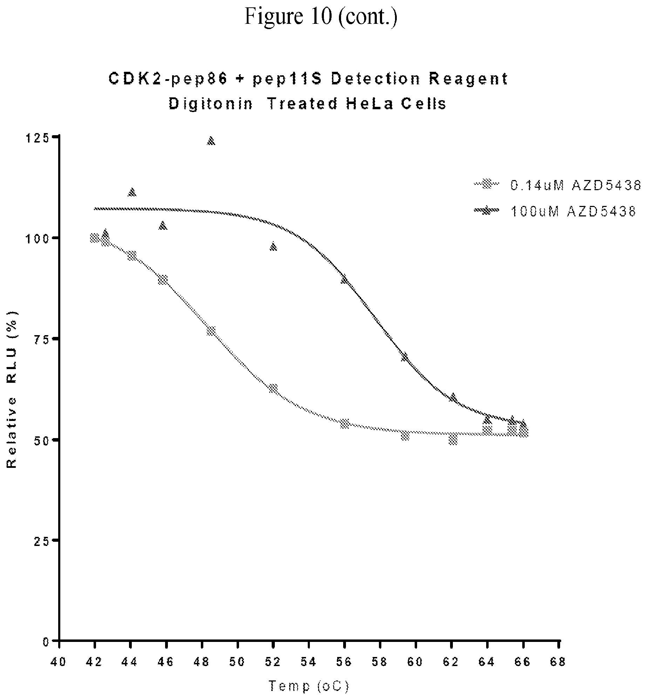

Figure 10 shows detection of an increase in melting temperature for CDK2-NLuc156 as determined by luciferase activity through spontaneous binary complementation after incubation in the presence of a stabilizing ligand in digitonin-treated mammalian cells subsequently exposed to a temperature gradient.

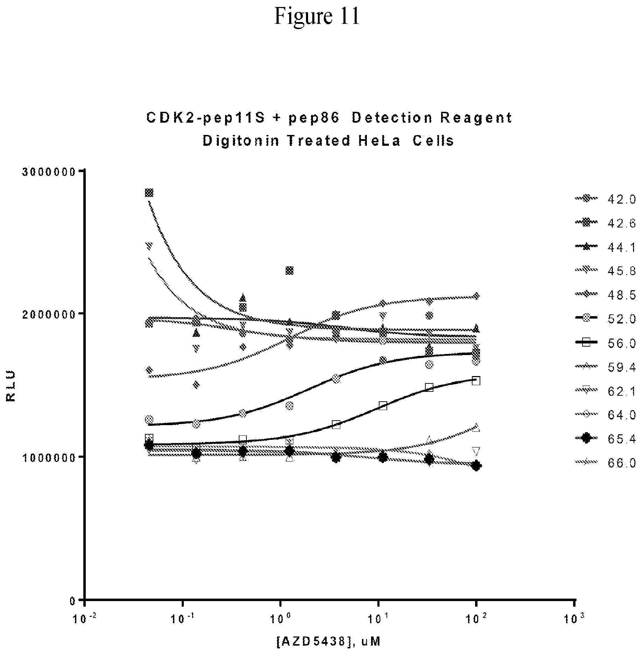

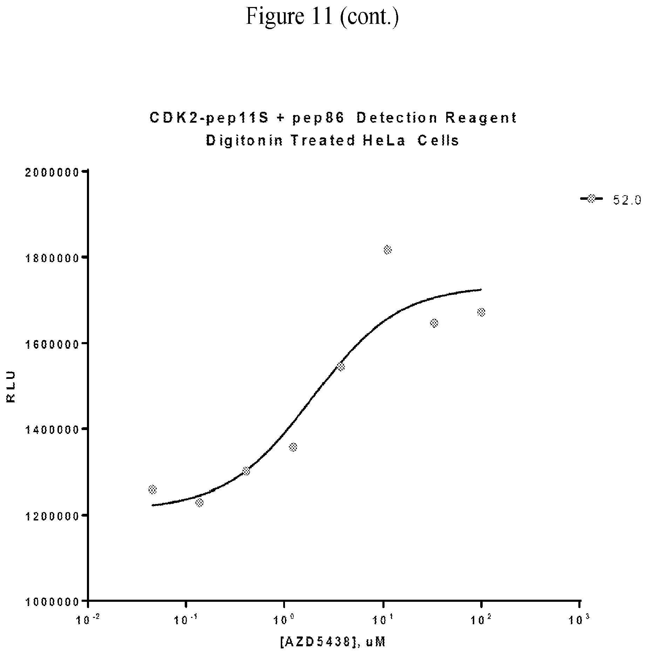

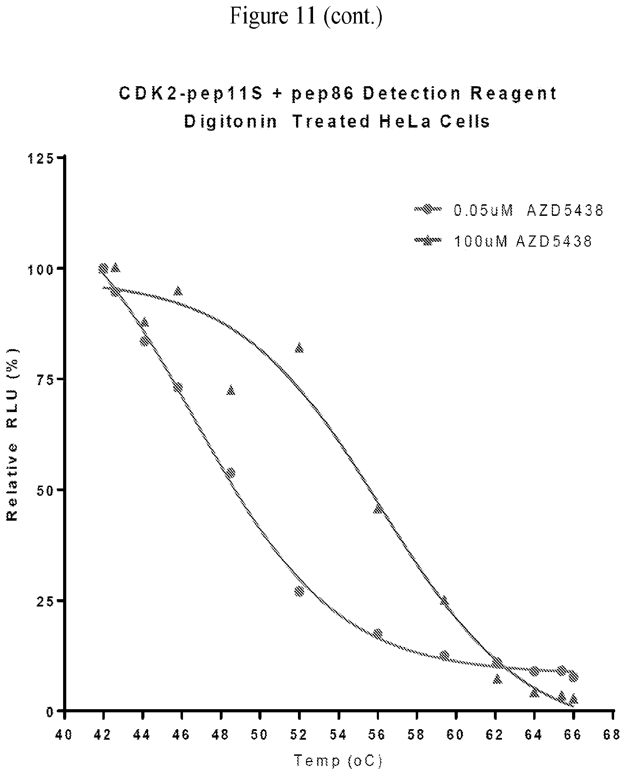

Figure 11 shows temperature and stabilizing ligand concentration effects on melting temperature of CDK2-pep86 as determined by luciferase activity through spontaneous binary complementation after incubation in the presence of stabilizing ligand in digitonin-treated mammalian cells subsequently exposed to a temperature gradient.

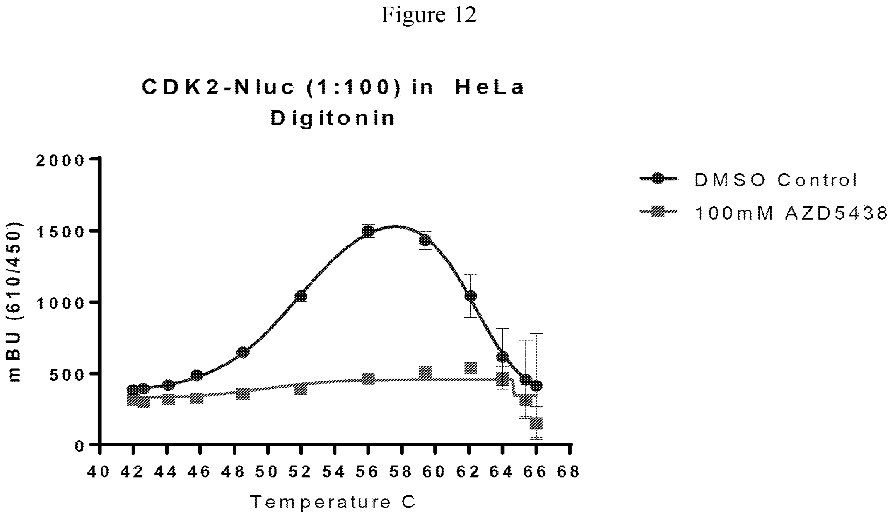

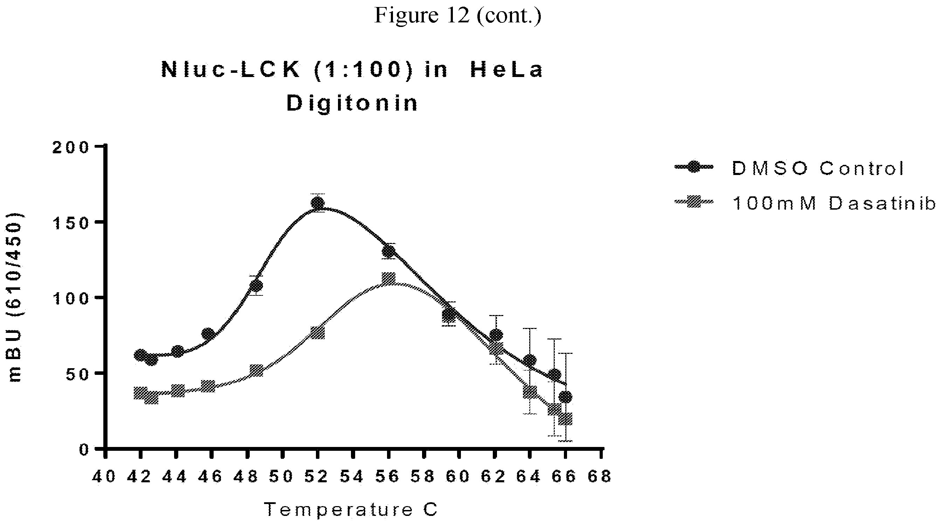

Figure 12 shows temperature and stabilizing ligand concentration effects on melting temperature of CDK2-11S as determined by luciferase activity through spontaneous binary complementation after incubation in the presence of stabilizing ligand in digitonin-treated mammalian cells subsequently exposed to a temperature gradient.

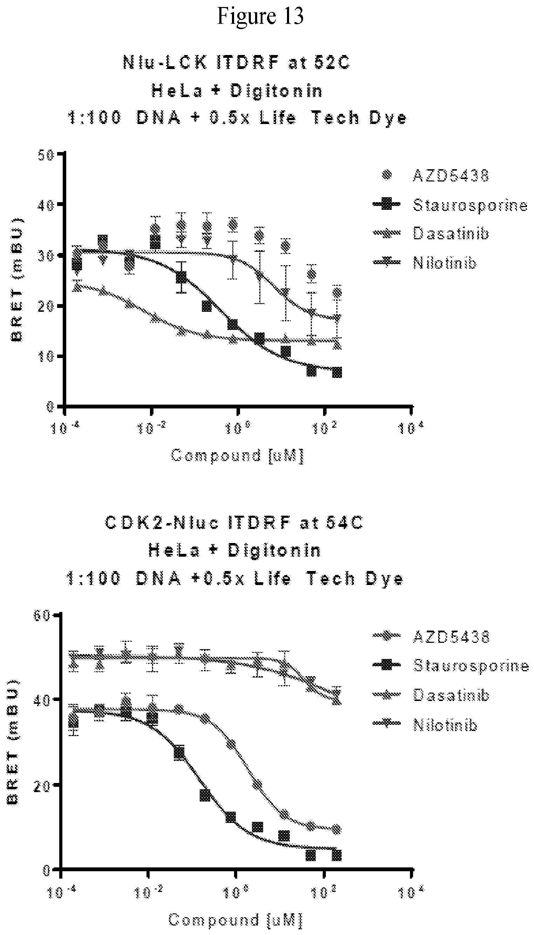

Figure 13 shows detection of ligand binding through a change in melting temperature for CDK2-Nluc and LCK-Nluc as determined by bioluminescence resonance energy transfer (BRET) after incubation in the presence of stabilizing ligand in digitonin-treated mammalian cells subsequently exposed to a temperature gradient.

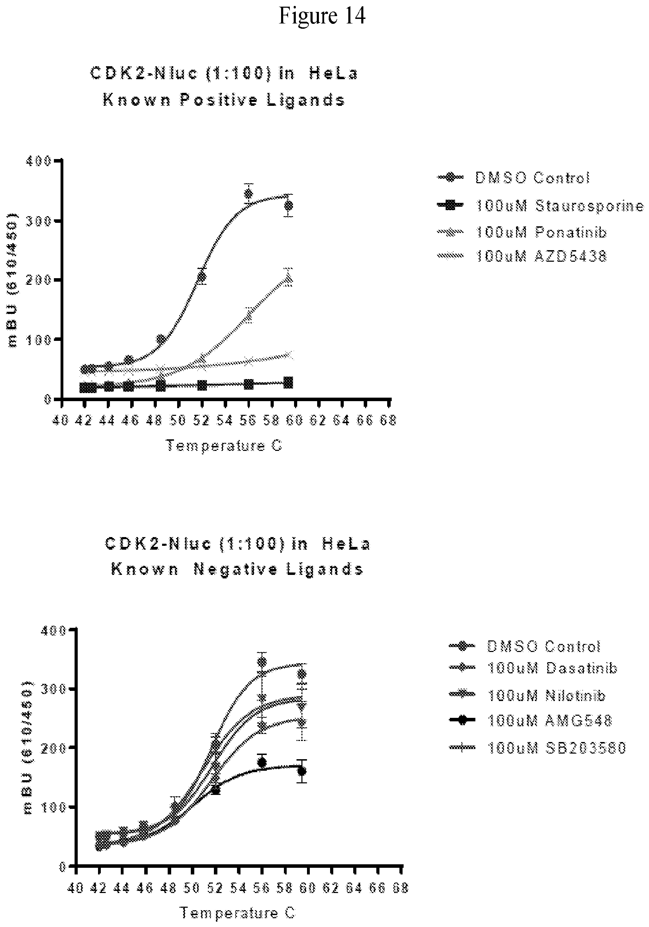

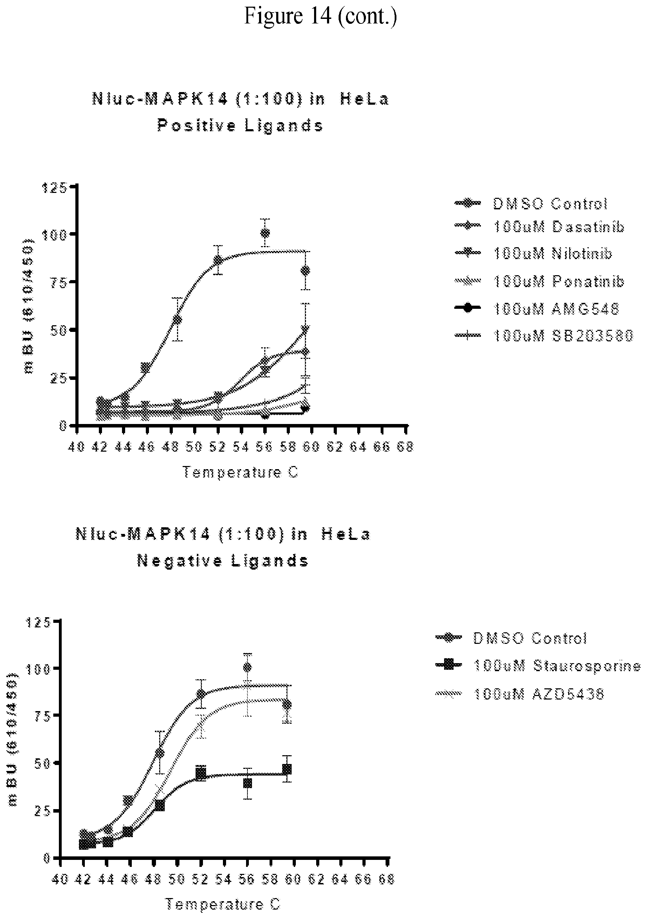

Figure 14 shows analysis of isothermal dose response curves for a panel of kinase inhibitors with CDK2-Nluc and LCK-Nluc as determined by BRET after incubation in the presence of different concentrations of the individual compounds while time of heating and temperature were kept constant. This highlights the concentration dependence of the thermal stabilization and allows for compound affinity signatures to be obtained.

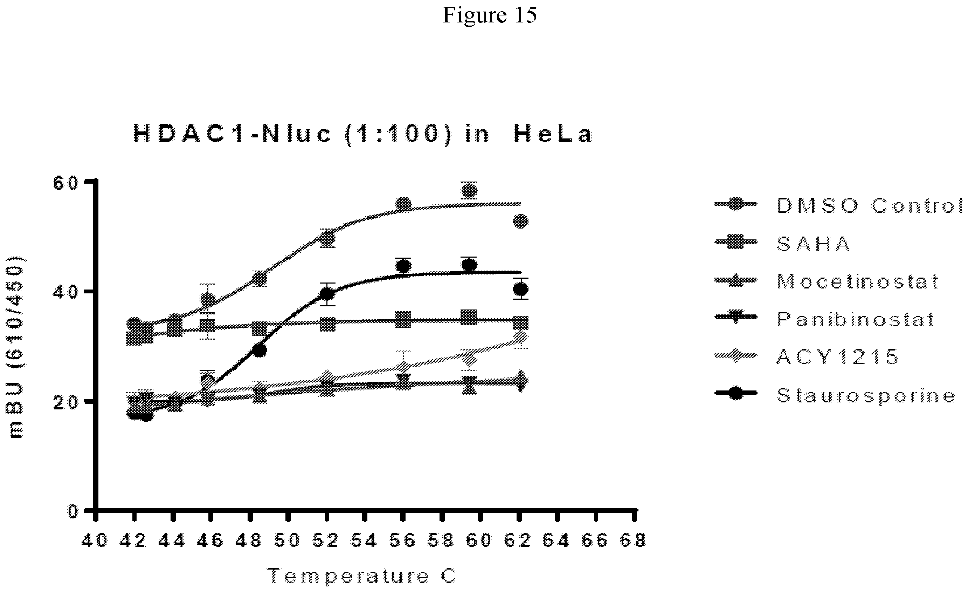

Figure 15 shows detection of an increase in melting temperatures (stabilizing ligand) or no change in melting temperatures (non-binding) for cytoplasmic target fusions CDK2-Nluc and Nluc-MAPK14 as determined by BRET after incubation with a panel of compounds thus displaying compound selectivity in mammalian cells subsequently exposed to digitonin and a temperature gradient.

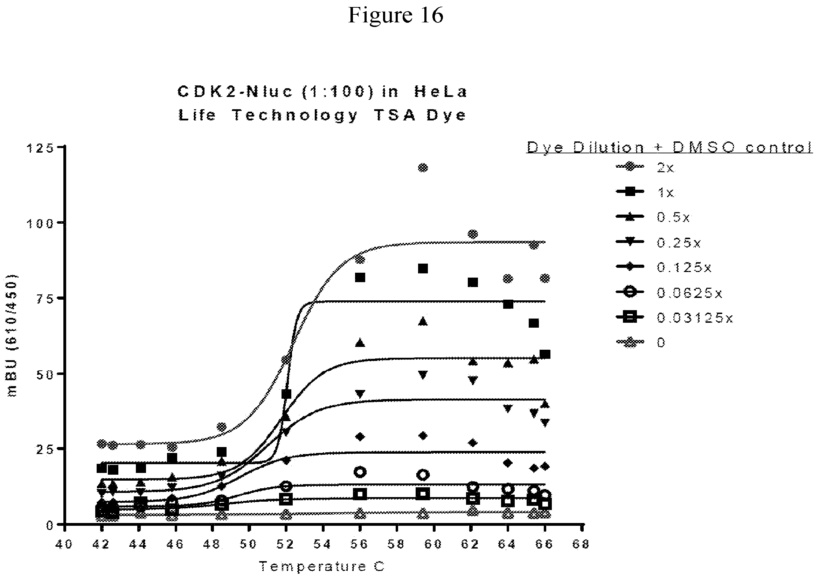

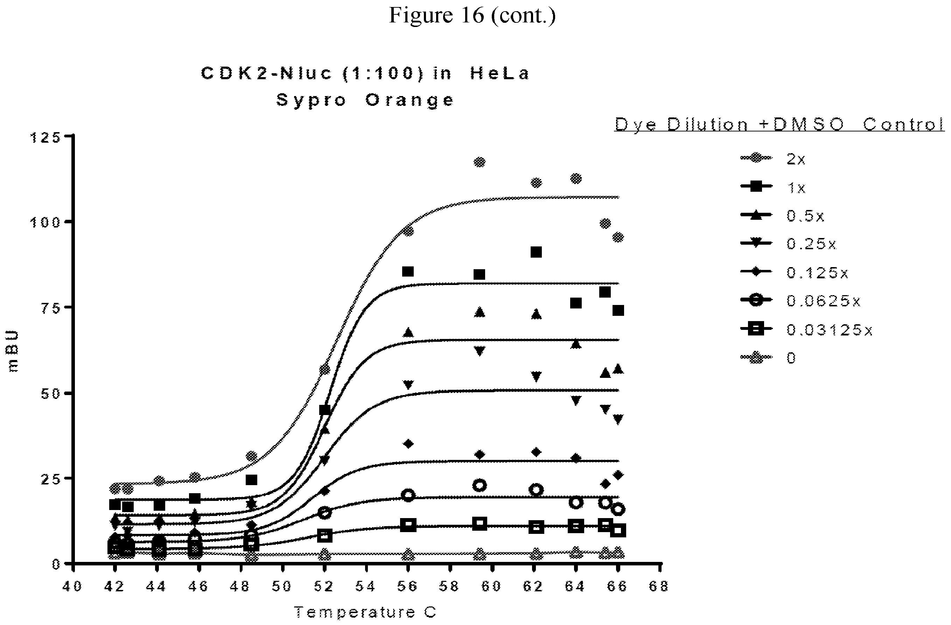

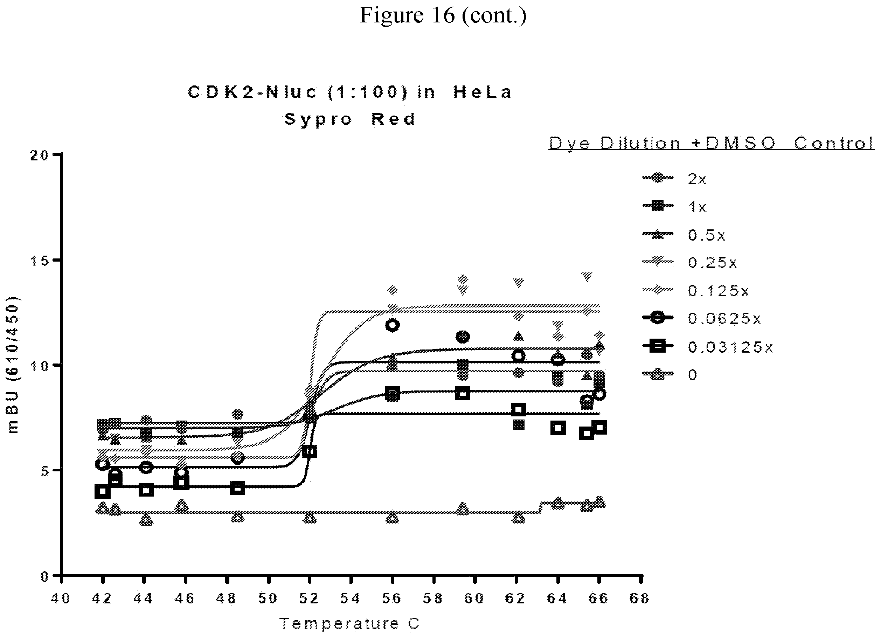

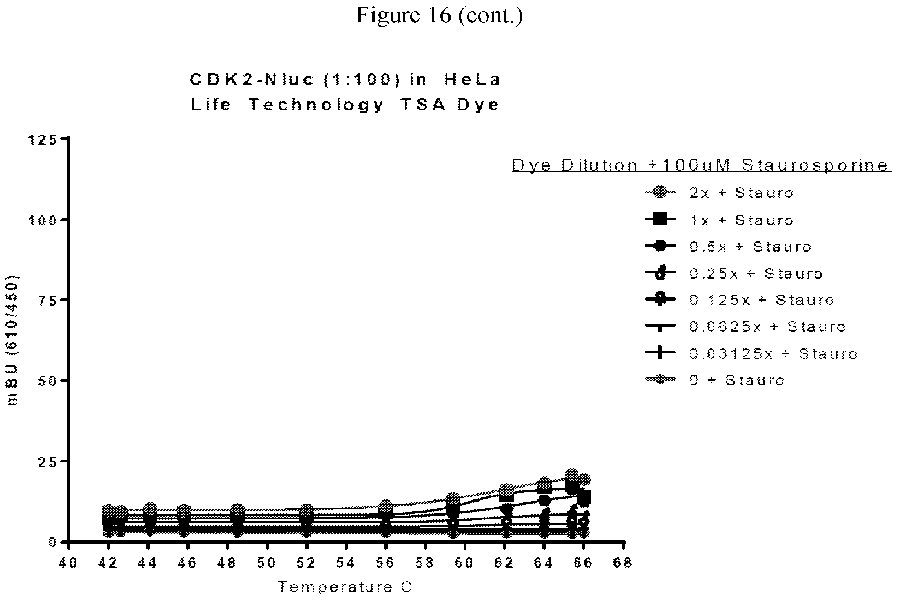

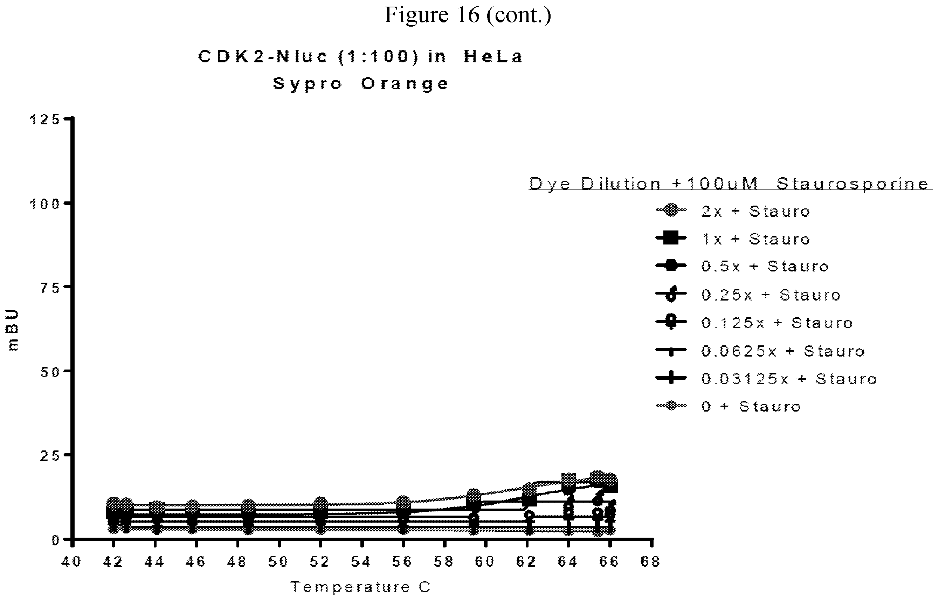

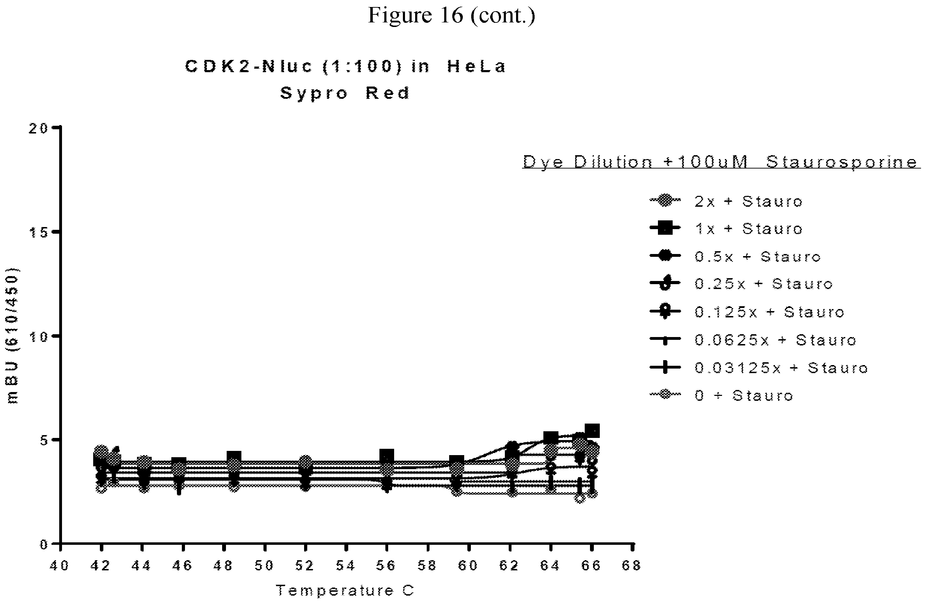

Figure 16 shows detection of an increase in melting temperatures (stabilizing ligand) or no change in melting temperatures (non-binding) for the nuclear target fusion HDAC1-Nluc as determined by BRET after incubation with a panel of compounds in mammalian cells subsequently exposed to digitonin and a temperature gradient.

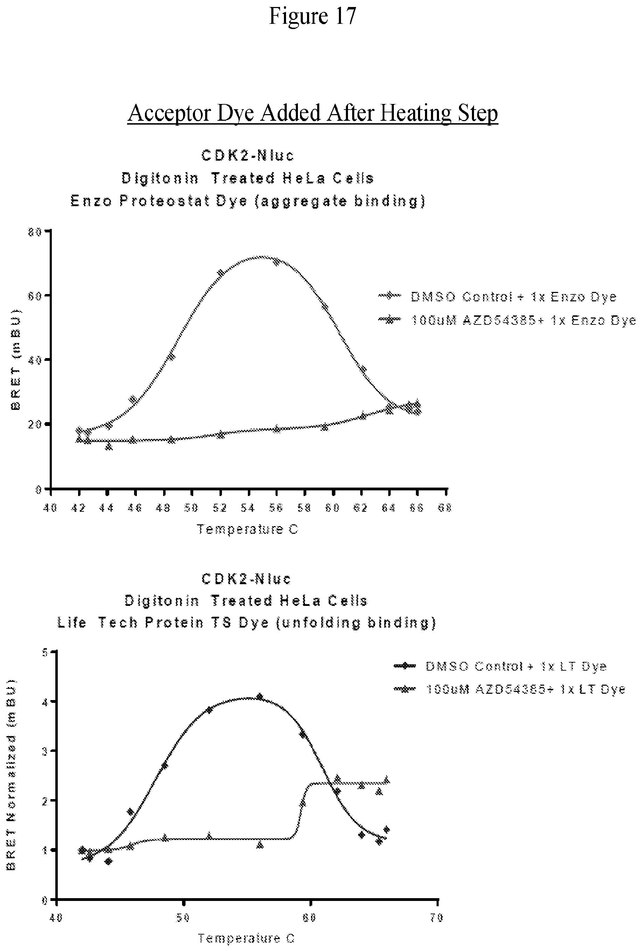

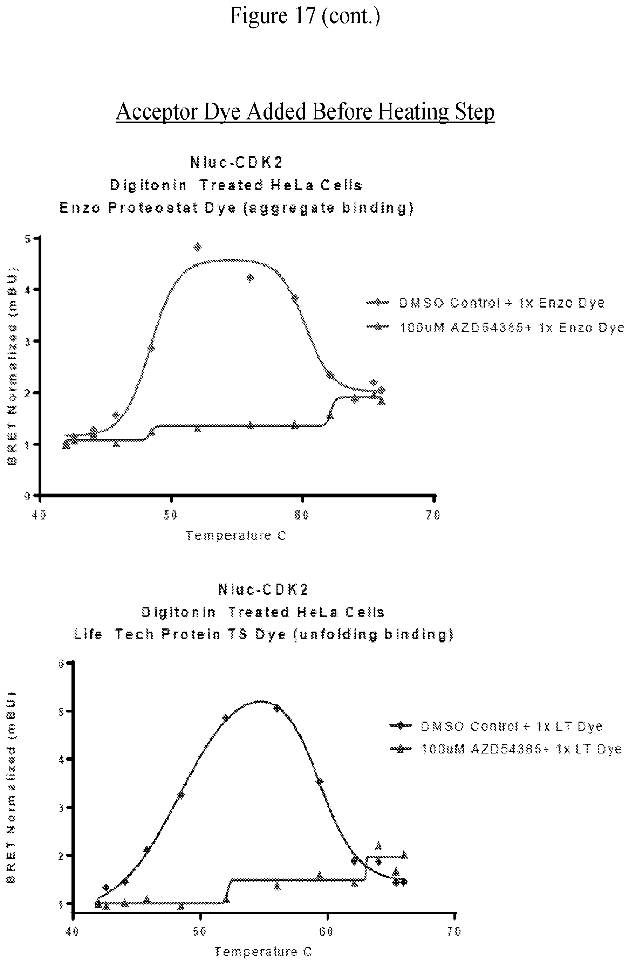

Figure 17 shows detection of an increase in melting temperature for CDK2-Nluc as determined by BRET after incubation with stabilizing ligand (staurosporine) in mammalian cells subsequently exposed to digitonin and a temperature gradient using three different environmentally-sensitive acceptor dyes reporting on protein folding status as BRET acceptor dyes. Dye examples included: Protein Thermal Shift^{™} Dye (Life Technologies), SYPRO Orange protein gel stain, and SYPRO Red protein gel stain. Bₘₐₓ is acceptor dye dose dependent, but that there is no change in the apparent melting temperature (T_{agg}).

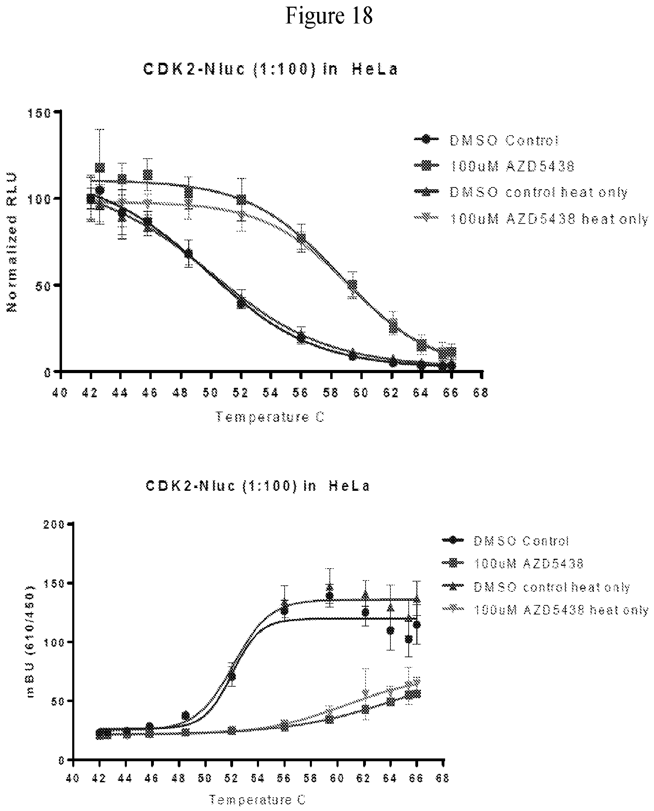

Figure 18 shows detection of an increase in melting temperature for CDK2-Nluc as determined by BRET after incubation with stabilizing ligand (AZD5438) in digitonin-treated mammalian cells subsequently exposed to a temperature gradient and using two different environmentally-sensitive dyes to report on the folded protein status and serve as BRET acceptor dyes which was either added before or after the heating step. Dye examples included: the dye included in the Life Technologies Thermal Shift Assay Dye Kit and the dye included in the Enzo Proteostat Thermal Shift Assay Kit.

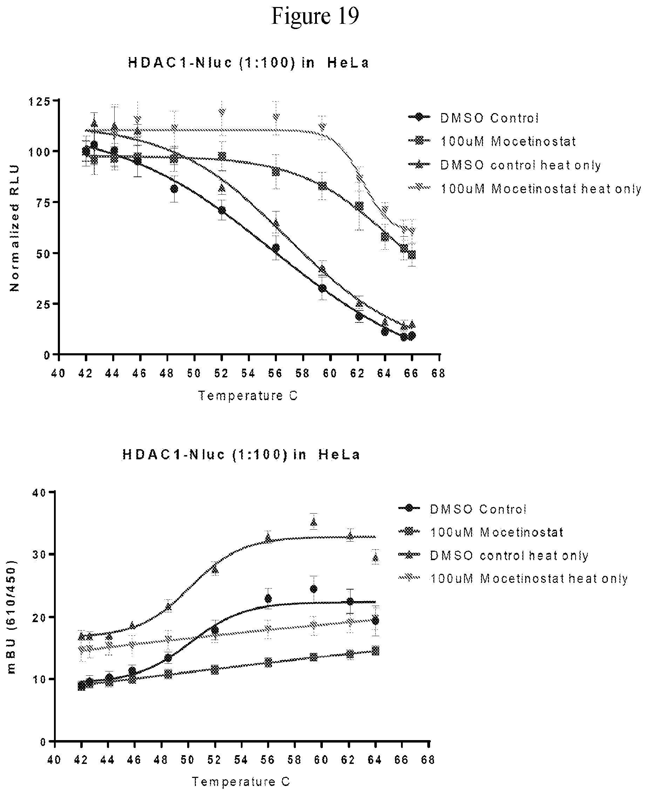

Figure 19 shows detection of an increase in melting temperature for CDK2-Nluc as determined by NanoLuc^{®} activity (RLU) and BRET (mBU) after incubation with stabilizing ligand in mammalian cells subsequently exposed to a temperature gradient and either analyzed immediately or incubated at room temperature for 3 minutes prior to analysis.

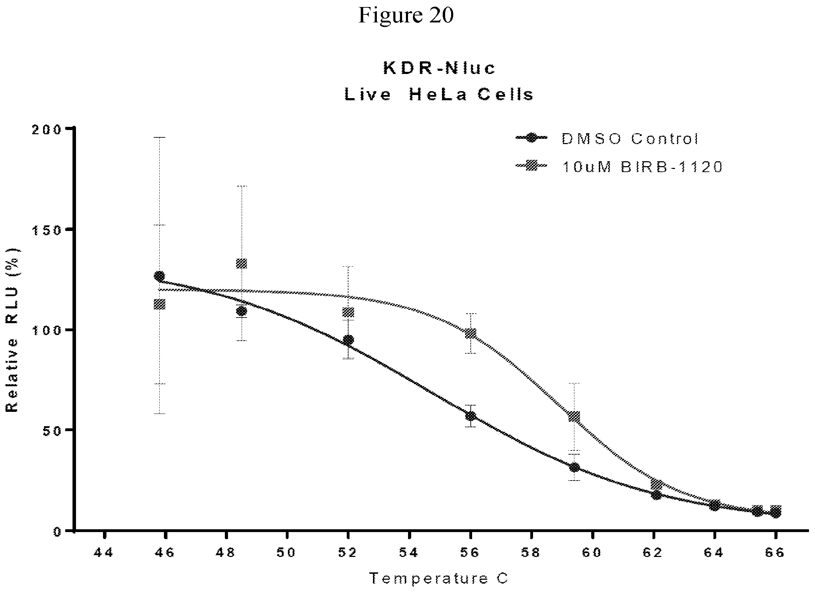

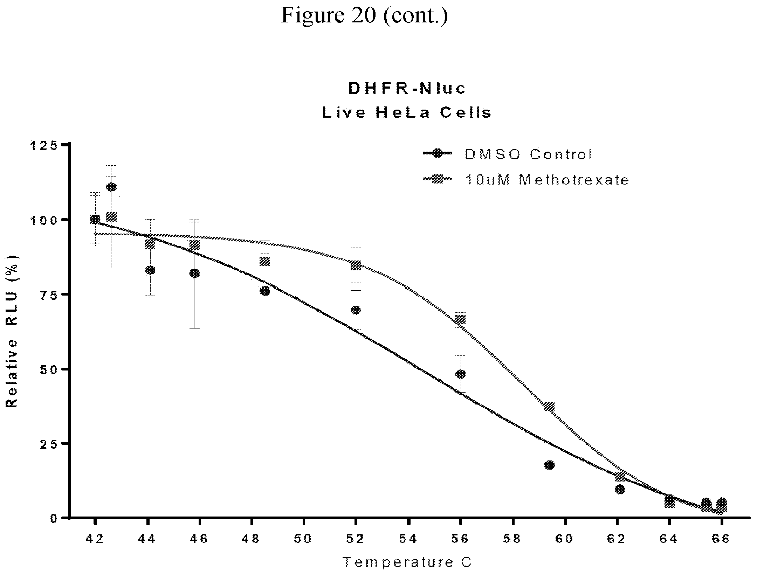

Figure 20 shows detection of an increase in melting temperature for the nuclear target fusion HDAC1-Nluc as determined by NanoLuc^{®} activity (RLU) and BRET (mBU) after incubation with stabilizing ligand in mammalian cells subsequently exposed to a temperature gradient and either analyzed immediately or incubated at room temperature for 3 minutes prior to analysis.

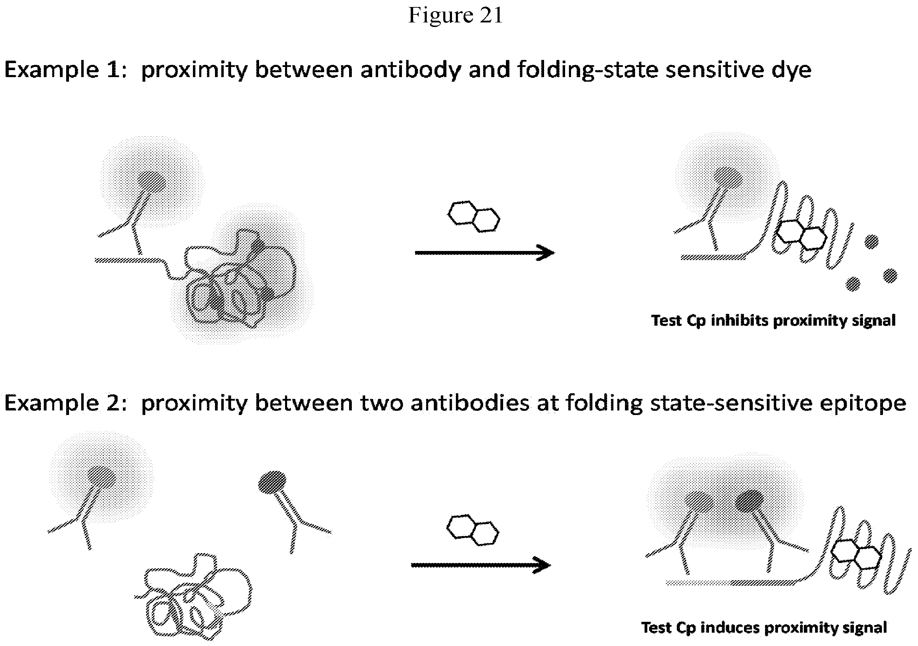

Figure 21 displays prophetic examples of alternative energy transfer strategies combined with environmentally sensitive dyes to use in a thermal shift assay.

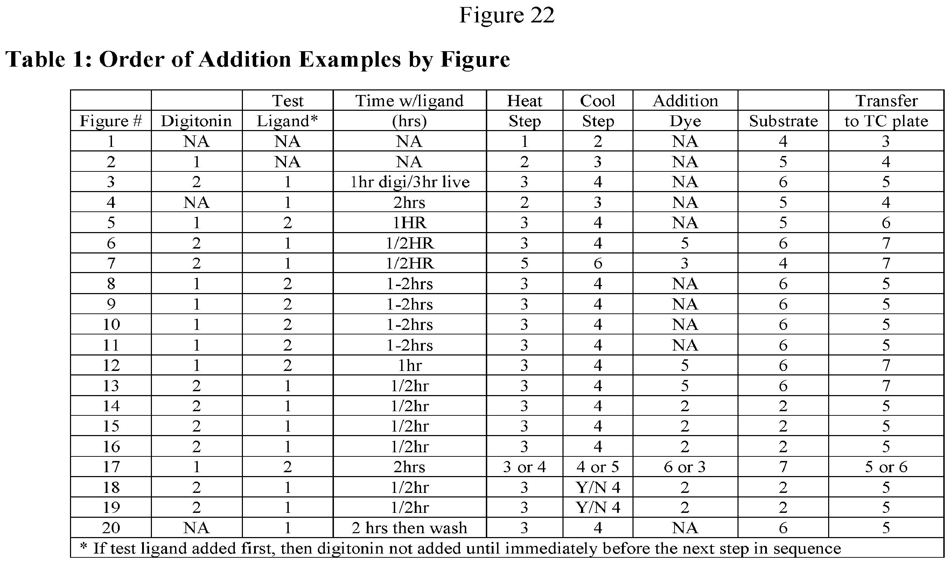

Figure 22 is Table 1, providing the order of addition of the components for the experiments depicted in Figures 1-20.

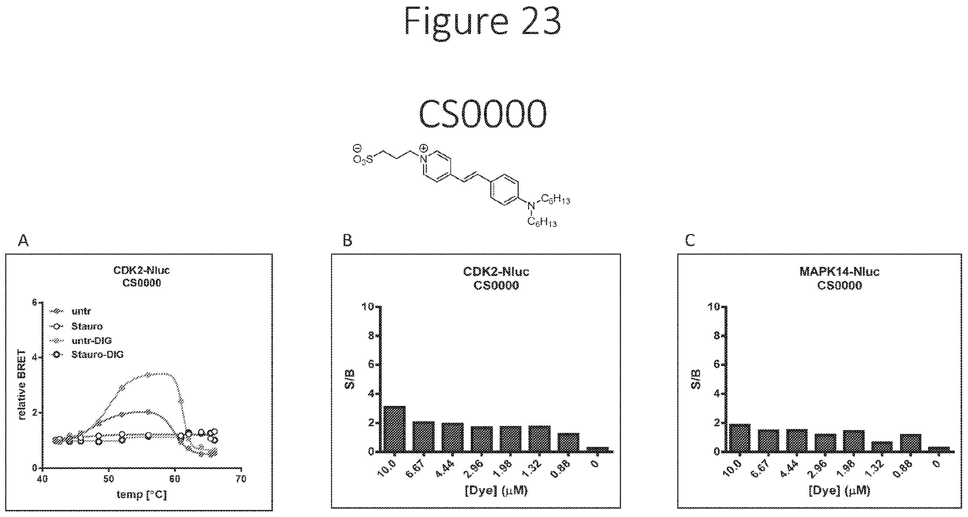

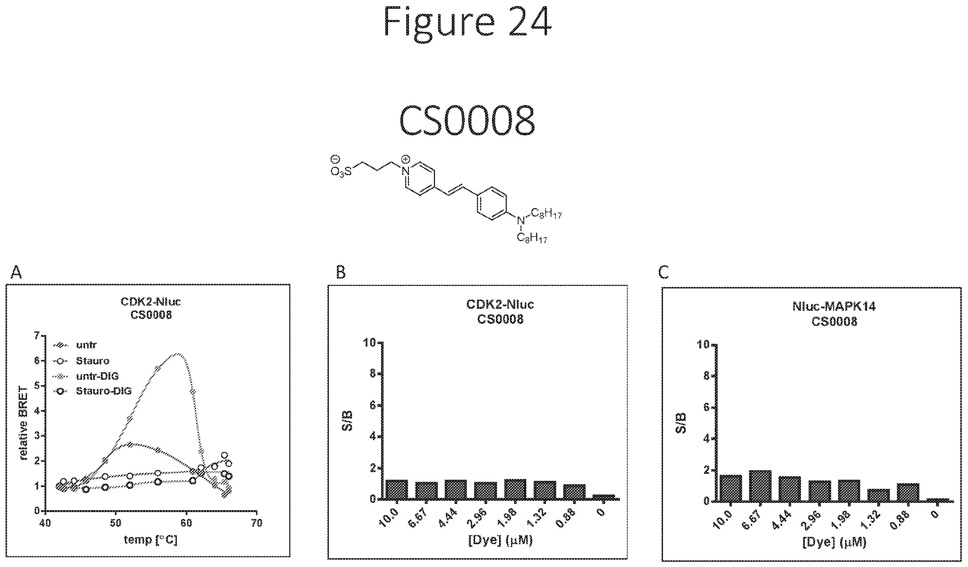

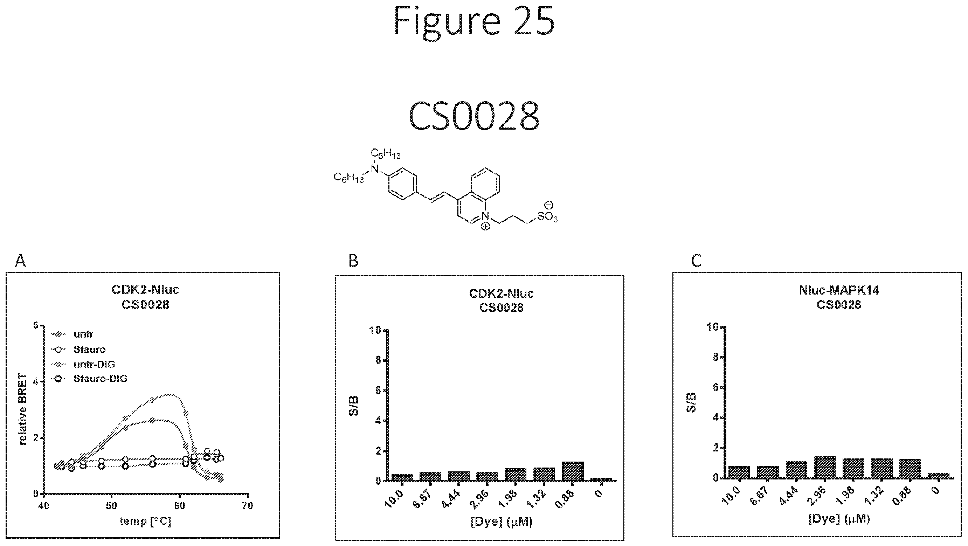

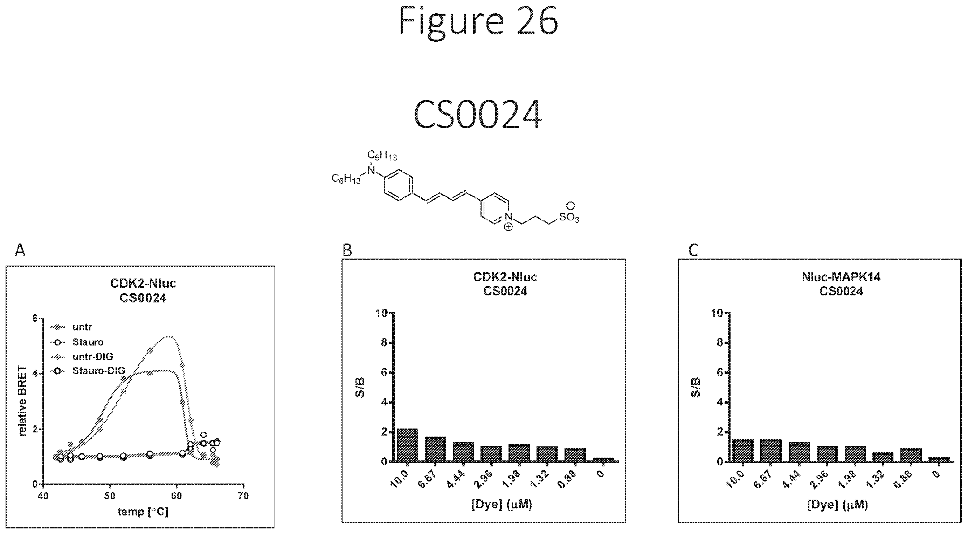

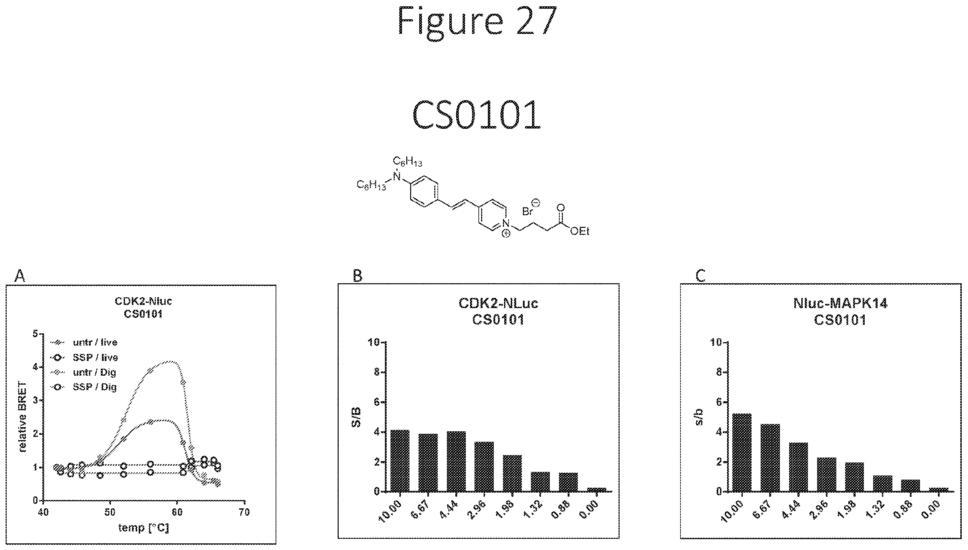

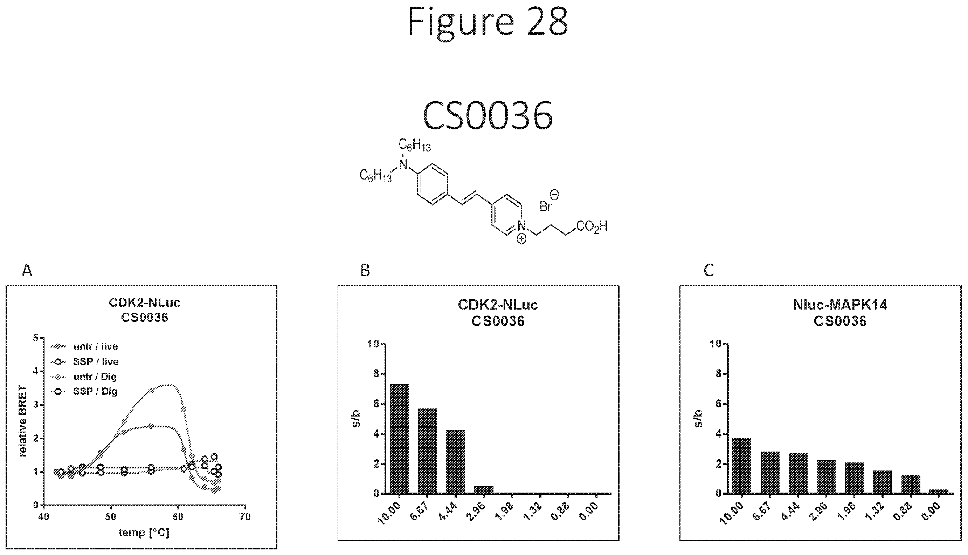

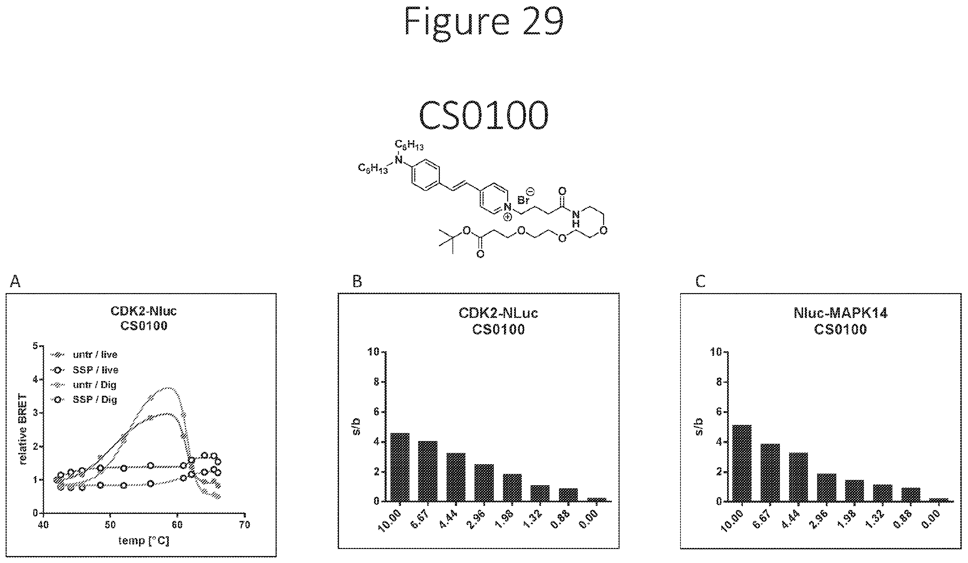

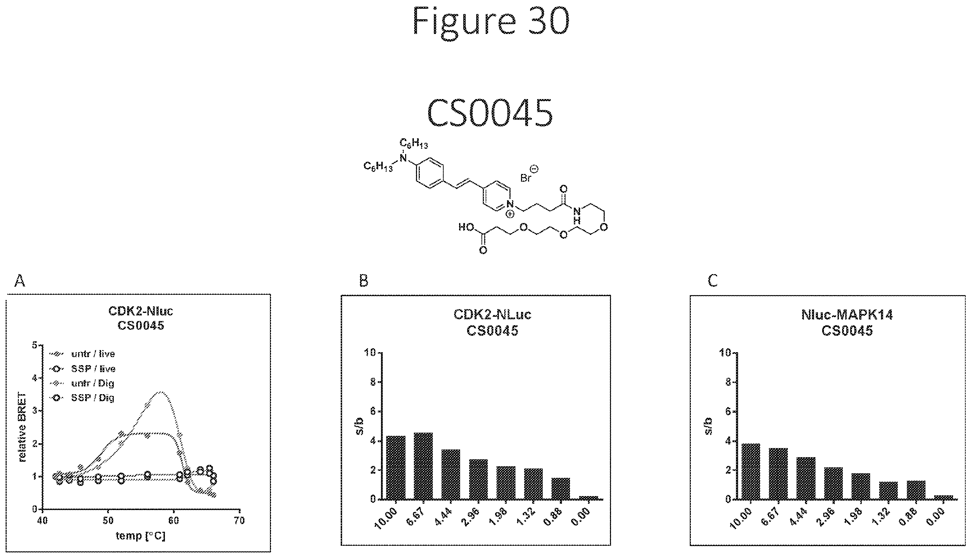

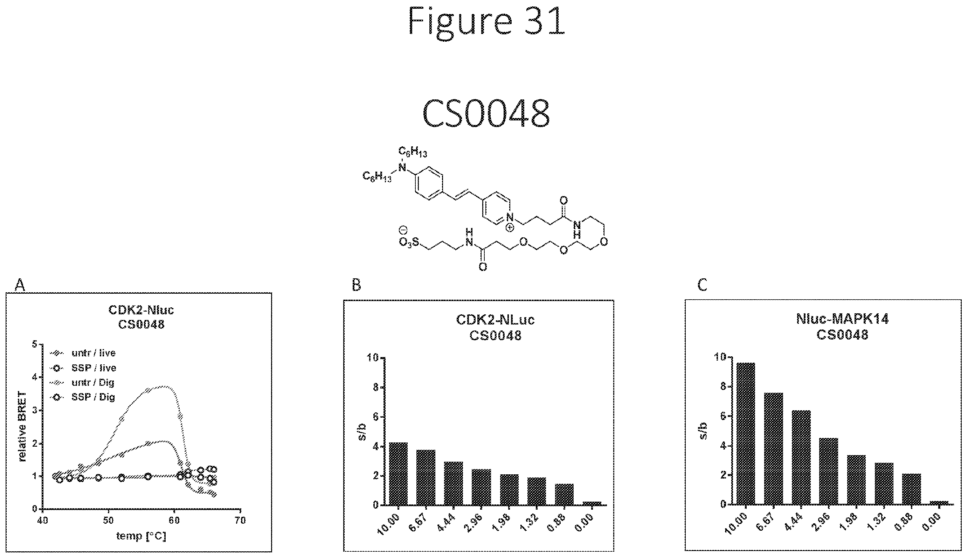

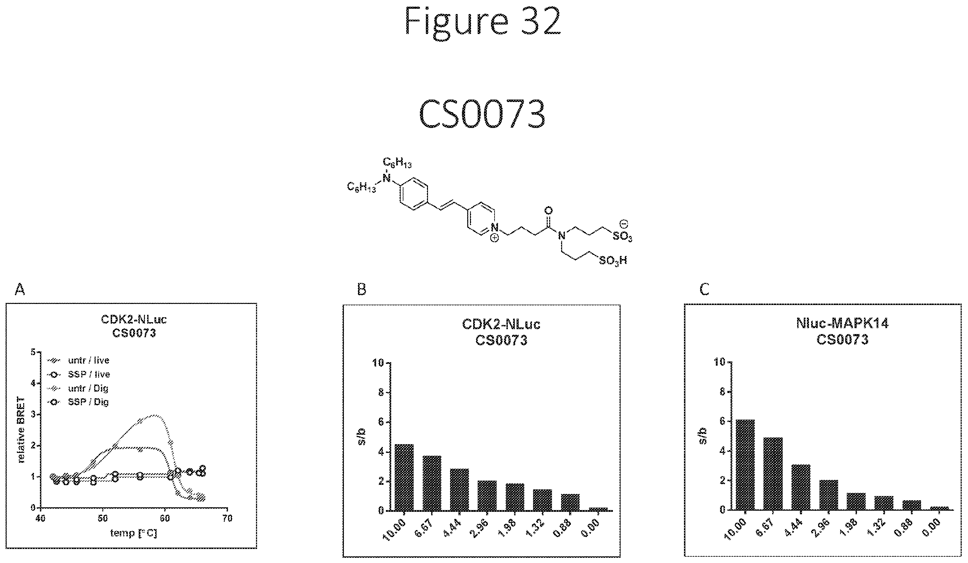

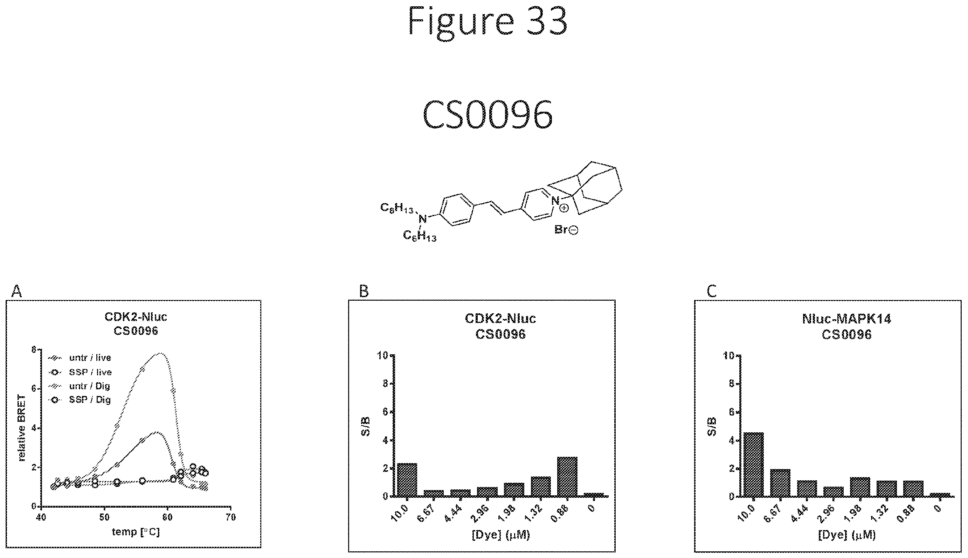

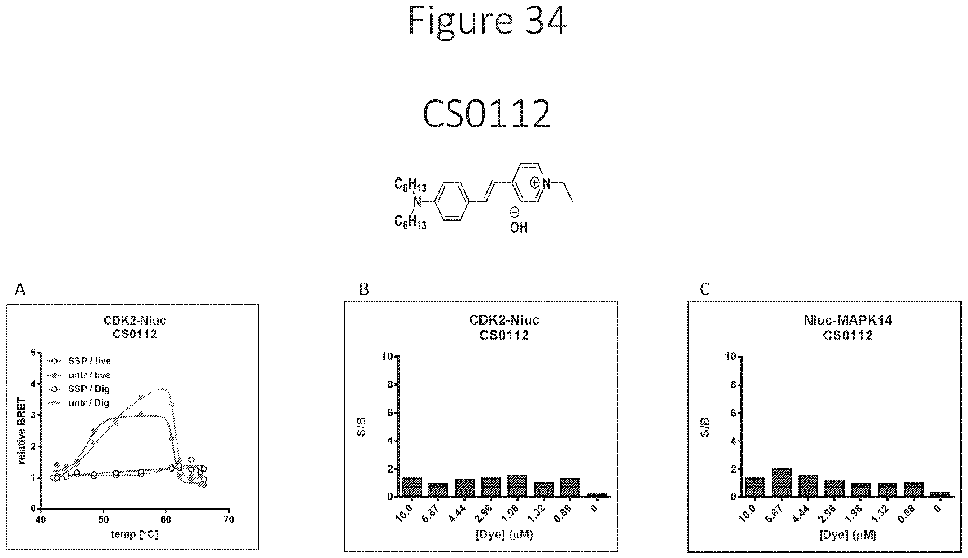

Figures 23-34 show graphs depiction characterization of dyes synthesized in Example 18: A) BRET be used to detect ligand binding through a change in relative BRET compared to DMSO controls (Unt) as exampled with CDK2-Nluc target fusions [+/- Staurosporine (SSP)] in live or lytic conditions [+/- digitonin (Dig)]. As expected, the shape of the BRET curves are bell-shaped due to the loss of Nluc signal with protein unfolding and increasing temperatures or dye dissociation upon protein aggregation or both; B) the fold change in BRET signal at 56°C over the background BRET signal at 42°C for cells transfected with CDK2-Nluc target that were treated with DMSO and varying dye concentrations. The larger the signal/background (S/B), the more signal window to allow for determining compound binding stability effects. This also allows optimal dye concentrations to be determined; C) the fold change in BRET signal at 52°C over the background BRET signal at 42°C for cells transfected with Nluc-MAPK14 target that were treated with DMSO and varying dye concentrations. The larger the signal/background (S/B), the more signal window to allow for determine compound binding stability effects, allowing determination of optimal dye concentrations.

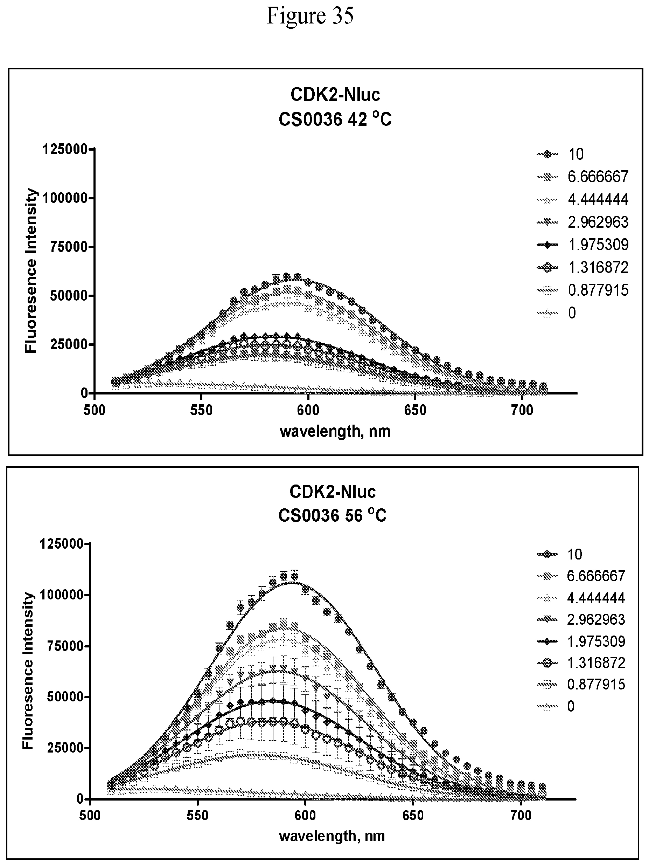

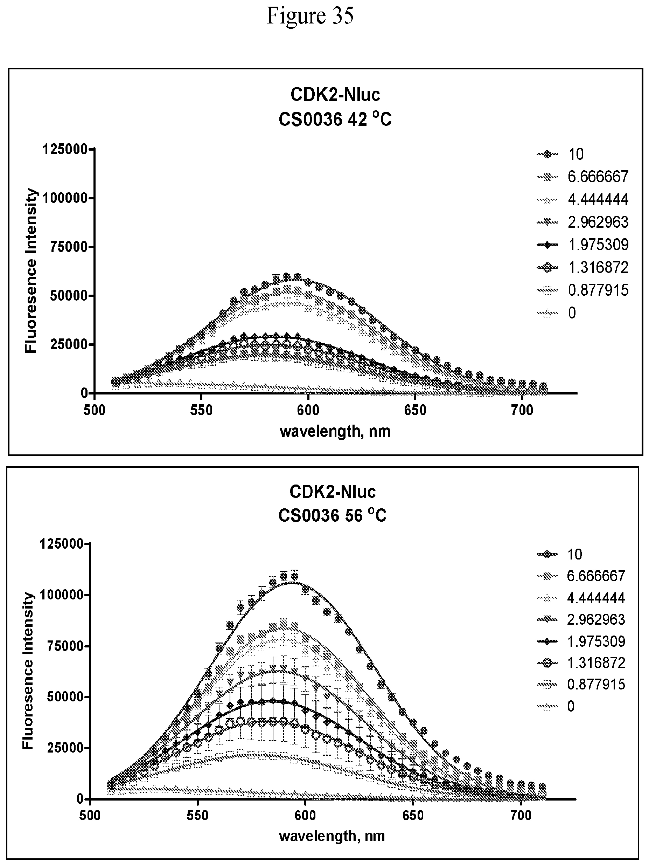

Figure 35 shows fluorescent mode analysis of CS0036.

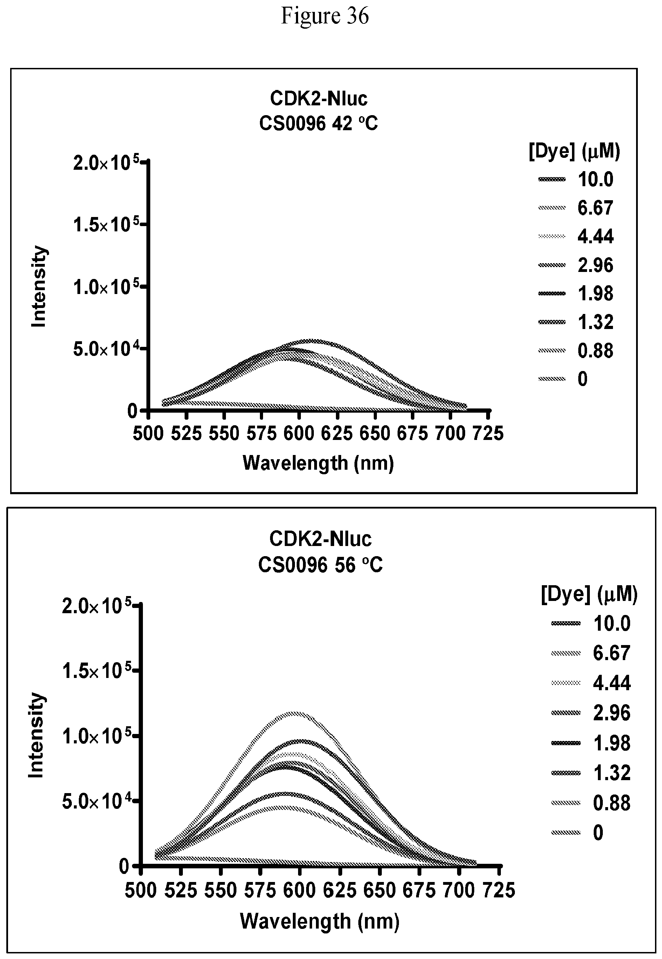

Figure 36 shows fluorescent mode analysis of CS0096.

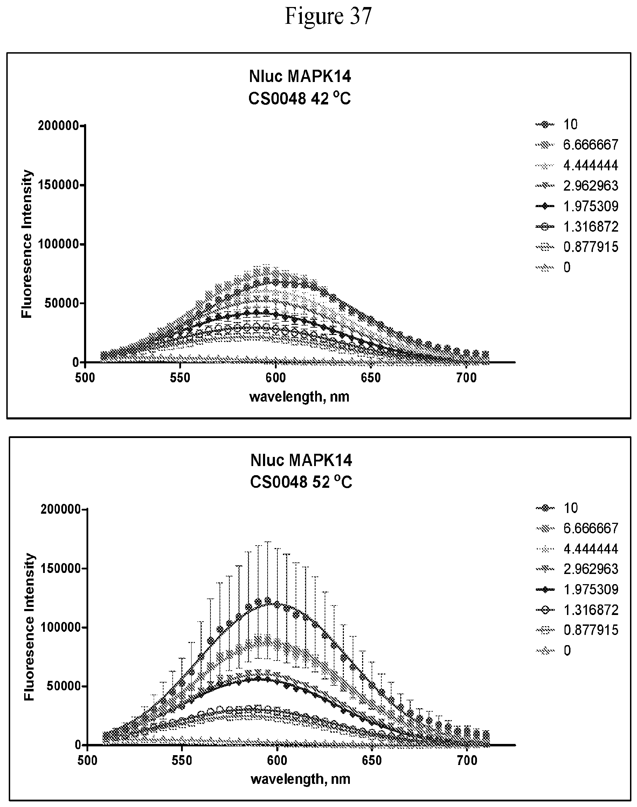

Figure 37 shows fluorescent mode analysis of CS0048.

### DEFINITIONS

As used herein, the terms "fusion," "fusion protein," and "fusion polypeptide" synonymously refer to a chimera of heterologous first and second protein or polypeptide segments, for example, a chimera of a protein of interest (e.g., target protein) joined to a reporter protein (e.g., luciferase). The second protein is typically fused to the N-terminus or C-terminus of the first protein, but may also be inserted internally within the sequence of the first protein.

As used herein, the term "luciferase" refers to any of a variety of monooxygenase enzymes that catalyze the conversion (e.g., oxidation) of a substrate (e.g., firefly luciferin, latia luciferin, bacterial luciferin, coelenterazine, dinoflagellate luciferin, vargulin, and derivatives thereof) into an excited-energy-state product that emits energy in the form of light upon decaying to its ground state.

As used herein, the term "natural polypeptide" as used herein refers to a polypeptide that exists in nature. For example, a "natural luciferase" is a luciferase polypeptide with a sequence and/or any other relevant features (e.g., post-translational modifications) that exists in nature. The term "synthetic polypeptide" refers to a polypeptide having an amino acid sequence that is distinct from those found in nature (e.g., not natural). When used in this context, the term "synthetic" does not relate to the method by which a polypeptide is produced (e.g., recombinant technology, chemical synthesis, etc.). A "wild-type polypeptide" or "wild-type luciferase" is the most common variant occurring in nature; whereas a "variant polypeptide" or "mutant polypeptide" refers to a polypeptide having an amino acid sequence that is distinct from the wild-type sequence. A variant or mutant polypeptide may be natural or synthetic. The aforementioned terms (e.g., "natural", "synthetic", "wild-type", "variant", and "mutant") have the same meanings when used in reference to nucleic acids, genes, peptide, proteins, etc.

As used herein, the term "sequence identity" refers to the degree to which two polymer sequences (e.g., peptide, polypeptide, nucleic acid, etc.) have the same sequential composition of monomer subunits. The term "sequence similarity" refers to the degree with which two polymer sequences (e.g., peptide, polypeptide, nucleic acid, etc.) have similar polymer sequences. For example, similar amino acids are those that share the same biophysical characteristics and can be grouped into the families, e.g., acidic (e.g., aspartate, glutamate), basic (e.g., lysine, arginine, histidine), non-polar (e.g., alanine, valine, leucine, isoleucine, proline, phenylalanine, methionine, tryptophan) and uncharged polar (e.g., glycine, asparagine, glutamine, cysteine, serine, threonine, tyrosine). The "percent sequence identity" (or "percent sequence similarity") is calculated by: (1) comparing two optimally aligned sequences over a window of comparison (e.g., the length of the longer sequence, the length of the shorter sequence, a specified window, etc.), (2) determining the number of positions containing identical (or similar) monomers (e.g., same amino acids occurs in both sequences, similar amino acid occurs in both sequences) to yield the number of matched positions, (3) dividing the number of matched positions by the total number of positions in the comparison window (e.g., the length of the longer sequence, the length of the shorter sequence, a specified window), and (4) multiplying the result by 100 to yield the percent sequence identity or percent sequence similarity. For example, if peptides A and B are both 20 amino acids in length and have identical amino acids at all but 1 position, then peptide A and peptide B have 95% sequence identity. If the amino acids at the non-identical position shared the same biophysical characteristics (e.g., both were acidic), then peptide A and peptide B would have 100% sequence similarity. As another example, if peptide C is 20 amino acids in length and peptide D is 15 amino acids in length, and 14 out of 15 amino acids in peptide D are identical to those of a portion of peptide C, then peptides C and D have 70% sequence identity, but peptide D has 93.3% sequence identity to an optimal comparison window of peptide C. For the purpose of calculating "percent sequence identity" (or "percent sequence similarity") herein, any gaps in aligned sequences are treated as mismatches at that position.

As used herein, the term "sample" is used broadly to refer to any of biological samples (e.g., fluids, tissues, etc.) and environmental samples as well as reaction mixtures or other multicomponent solutions and mixtures.

As used herein, the term "complex sample" refers to a sample comprising a large number and variety of different compounds, polymers, macromolecules, complexes, etc. A complex sample may comprise buffers, salts, peptides, polypeptides, proteins (including also enzymes), carbohydrates (complex and simple carbohydrates), lipids, fatty acids, fat, nucleic acids, organelles and other cellular components, etc. Examples of complex samples include cells, e.g., live intact cells, cell lysates, body fluids (e.g., blood (or blood products), saliva, urine, etc.), tissues (e.g., biopsy tissue), cells grown in vitro and subsequently injected into animal in vivo and recollected for ex vivo analysis, cells in 3D culture, cells in tissues, reaction mixtures, etc. In particular embodiments, a complex samples contain a target protein as well as additional non-target peptides, polypeptides, and/or proteins.

As used herein, the term "quenched" refers to a decrease in fluorescence emission from a fluorescent entity (e.g., dye) upon interaction of a particular substance (e.g., water) or condition, relative to the fluorescence emission from the fluorescent entity when not interacting with the particular substance or condition. The term "quenched" itself does not place any limitation on the extent of the decrease in fluorescence. The degree of quenching may be expressed at a percentage of fluorescence in the quench state compared to the unquenched state (e.g., 10 RLU in the quenched state compared to 100 RLU in the unquenched state is 90% quenching).

As used herein, the term "BRET" is used to describe the occurrence of bioluminescence resonance energy transfer between a bioluminescent donor (e.g., a luciferase protein) and an acceptor fluorophore. It is a distance-dependent interaction in which energy is transferred from the donor bioluminescent protein and substrate to an acceptor fluorophore without emission of a photon. The efficiency of BRET is dependent on the inverse sixth power of the intermolecular separation, making it useful over distances comparable with the dimensions of biological macromolecules (e.g., within 30-80 Å, depending on the degree of spectral overlap).

As used herein, the term "ligand for a target protein" refers to a molecular entity that binds to a target protein. The ligand may be a small molecule, peptide, antibodies, macromolecules (e.g., nucleic acid, viral proteins, bacterial proteins, polysaccharides, synthetic polymers), or other molecular entity that bind the target protein. The term "test ligand" refers to a molecular entity that is being assayed for the capacity to bind the target protein.

### DETAILED DESCRIPTION

Provided herein are systems and methods for characterizing target/ligand engagement. In particular, luciferase-labeled polypeptide targets are used to detect or quantify target/ligand engagement (e.g., within a cell or cell lysate).

In some embodiments, methods are provided for monitoring (e.g., detecting, quantitating, etc.) target/ligand engagement in complex (cellular) environments (e.g., based on the biophysical principle of ligand-induced thermal stabilization of target proteins). Also disclosed herein, but not forming part of the claimed invention are compositions (e.g., fusions of target polypeptides and luciferase reporter) and systems (e.g., kits or reaction mixtures comprising, for example, test ligands, luciferase substrates, assay reagents, cells, e.g., live intact cells, cell lysates, etc.) for monitoring (e.g., detecting, quantitating, etc.) target/ligand engagement in complex (cellular) environments (e.g., based on the biophysical principle of ligand-induced thermal stabilization of target proteins). In some embodiments, the luminescent signal generated by a fusion of a target protein and luciferase (e.g., NanoLuc (Promega Corp., Madison, WI); see, e.g., U.S. Pat. No. 8,557,970 and U.S. Pat. No. 8,669,103) or a luciferase peptide/polypeptide interacts with a complement polypeptide/peptide to produce an active enzyme (e.g., NLPep and NLPoly (Promega Corp., Madison, WI); see, e.g.., U.S. Pub. No. 2014/0348747) is monitored over temperature range sufficient to result in unfolding and aggregation of the unbound target protein, both in the presence and absence of a ligand or test ligand for the target protein. An altered or shifted luminescent signal over the range of temperatures indicates interaction between the target protein and ligand. In some embodiments, the luminescent signal (e.g., relative light units (RLU)) from the luciferase reporter portion of the fusion is directly monitored. In some embodiments, bioluminescence resonance energy transfer (BRET) from the luciferase reporter portion of the fusion to an environmentally sensitive dye (e.g., a dye that interacts with the hydrophobic portions of the target protein as they become exposed at higher temperatures, a protein-aggregation detection dye, etc.) is measured.

In some embodiments, the target/luciferase fusion is expressed in cells (e.g., a live intact cell or a relevant cell line), and the cells and/or a cell lysate thereof, is exposed to a ligand of interest (e.g., ligand for the target protein, test ligand (e.g., potential drug), etc.) and subsequently exposed to conditions (e.g., increased temperature) capable of causing the unfolding and/or aggregation of the unbound target protein. In such embodiments, a target protein that has been stabilized by interaction with ligand in the cell or cell lysate will exhibit an altered or shifted melting temperature as detected by alteration of the luminescent or BRET signal in the presence and absence of ligand.

In some embodiments in which luciferase signal is directly detected as a measure of thermal shift, luciferase substrate (and purified complementary peptide/polypeptide in the case complementation assays (e.g., using NLPep and NLPoly (Promega Corp., Madison, WI); see, e.g.., U.S. Pub. No. 2014/0348747) is added to the samples (e.g., cells, cell lysate, etc.) for analysis. In other embodiments in which BRET signal from the luciferase of the fusion to an environmentally sensitive dye (e.g., a dye that interacts with the hydrophobic portions of the target protein as they become exposed at higher temperatures, a protein-aggregation detection dye, etc.) is detected as a measure of thermal shift, luciferase substrate (and purified complementary peptide/polypeptide in the case complementation assays (e.g., using NLPep and NLPoly)) and the environmentally sensitive dye are added to the samples (e.g., cells, cell lysate, etc.) for analysis. In some embodiments, detergents (e.g. Digitonin), lysis buffer (e.g. NanoGlo^{®} Lytic reagent), and/or other reagents are added to the sample. In some embodiments, luminescence and/or BRET is analyzed using, for example, by a luminometer at one or more specific temperatures (e.g., encompassing a temperature in which the target protein unfolds). Shift of the signal (e.g., luciferase signal or BRET signal) in the presence vs. absence of ligand indicates interaction (e.g., binding) of the target and ligand.

It has long been recognized that the binding of low molecular weight ligands increases the thermal stability of a protein (Koshland (1958). Proc Natl Acad Sci U S A. 44 (2): 98-104.; Linderstrom-Lang & Schellman (1959). The Enzymes. 1(2) 443-510.). A TSA detects this stabilizing effect by measuring the thermal stability of a target protein in the presence and absence of a ligand, thereby detecting or quantifying the target/ligand interaction. A traditional TSA is a fluorescence-based method for monitoring target/ligand interactions (WO 1997/020952); the primary weakness of such an assay is that it detects the unfolding of any proteins present in the sample being assayed (e.g., target and non-target protein), and therefore can only be used to analyze purified protein. Because target/ligand interactions may be affected by a multitude of factors *in vivo*, a traditional TSA of purified protein may be of limited informational value. Other TSA-type assays have been developed (See, e.g., Moreau M J, et al. Quantitative determination of protein stability and ligand binding using a green fluorescent reporter system. Mol. Biosyst. (6); 1285-1292. 2010; and CETSA, U.S. Pub No. 2014/0057368); however, these assays rely on post-denaturation quantification of soluble vs. denatured protein as a measure of target protein stability and require multiple purification and/or detection steps. Provided herein are assays that can be used to identify ligands that bind to a target protein and/or to quantify the affinity of such interactions. The assays described herein are distinct from other methods (e.g., traditional thermal shift, GFP reporter systems, CESTA, etc.), for example, because: (1) the assays allow characterization of target/ligand interactions with non-purified protein; (2) the assays can be conducted in a cell, cell lysate, or other complex liquid containing many different biomolecules; (3) the assay technology does not rely upon antibodies recognizing target protein epitopes; (4) the assays are homogeneous; (5) the assays use common and simple reagents, materials and instruments (e.g., a luminometer); and/or (6) assays are not limited to a single read.

In some embodiments, the methods described herein utilize fusion constructs (e.g., fusion polypeptides and nucleic acids and vectors encoding them) comprising a target protein (e.g., the binding characteristics of which are being examined) and a reporter peptide or polypeptide. In some embodiments, any protein of interest may find use as the target protein. Assays described herein provide for analysis of the binding characteristics (e.g., potential ligands, binding affinity, etc.) of such proteins. A reporter of the fusion construct may be any peptide or polypeptide, the activity of which can be detected within a cell, e.g., a live intact cell, or cell lysate, in real-time. While the scope of embodiments herein is not limited by the identity of the reporter, the methods of the present invention utilize a luciferase bioluminescent reporter that has 70% or greater sequence identity with SEQ ID NO: 1. In such embodiments, a suitable luciferase is fused (e.g., directly or by a linker (e.g., peptide or other linker moiety)) to the N-terminus or C-terminus of a target protein (or inserted internally within the target protein) to generate a fusion polypeptide for use in the systems and methods herein. In some embodiments, nucleic acid constructs are provided that encode fusion polypeptides (e.g., N-target-reporter-C, N-reporter-target-C, N-target-linker-reporter-C, N-reporter-linker-target-C, etc.). In some embodiments, vectors (e.g., plasmids, bacmids, cosmids, viral vectors (e.g., lentivirus vectors, adeno-associated viral vectors (AAVs), etc.), etc.) comprising nucleic acid constructs that encode fusion polypeptides (e.g., along with appropriate incorporation and/or expression elements) are provided. In some embodiments, cells (e.g., bacterial, mammalian, human, etc.) transformed or transfected (e.g., transiently or stably) with nucleic acids and/or vectors encoding fusion polypeptides useful for assays described herein are provided.

The methods of the present invention utilize a luciferase bioluminescent reporter that has 70% or greater sequence identity with SEQ ID NO: 1. Also disclosed herein but not forming part of the claimed invention, the reporter of a fusion construct may be one that exhibits any suitably detectable activity. Suitable luciferase enzymes include those selected from the group consisting of: *Photinus pyralis* or North American firefly luciferase; Luciola cruciata or Japanese firefly or Genji-botaru luciferase; *Luciola italic* or Italian firefly luciferase; *Luciola lateralis* or Japanese firefly or Heike luciferase; *Luciola mingrelica* or East European firefly luciferase; *Photuris pennsylvanica* or Pennsylvania firefly luciferase; *Pyrophorus plagiophthalamus* or Click beetle luciferase; *Phrixothrix hirtus* or Railroad worm luciferase; *Renilla reniformis* or wild-type Renilla luciferase; *Renilla reniformis* Rluc8 mutant Renilla luciferase; Renilla reniformis Green Renilla luciferase; *Gaussia princeps* wild-type Gaussia luciferase; *Gaussia princeps* Gaussia-Dura luciferase; *Cypridina noctiluca* or Cypridina luciferase; *Cypridina hilgendorfii* or Cypridina or Vargula luciferase; *Metridia longa* or Metridia luciferase; and Oplophorus luciferase (e.g., *Oplophorus gracilirostris* (OgLuc luciferase), *Oplophorus grimaldii*, *Oplophorus spinicauda, Oplophorus foliaceus, Oplophorus noraezeelandiae, Oplophorus typus*, *Oplophorus noraezelandiae* or *Oplophorus spinous*). For any of the above luciferases, fusions may contain a wild-type or naturally-occurring variant of the luciferase or may comprise a synthetic version (e.g., optimized for one or more characteristics (e.g., emission, stability, etc.). In some embodiments, assays are carried out both in the presence and absence of a ligand for the target protein (or a test ligand) and in the presence of the appropriate substrate for the luciferase. A negative control assay may be performed in the absence of substrate. A positive control may be performed with the un-fused luciferase and appropriate substrate in the presence and absence of ligand and/or in the presence an absence of un-fused target protein.

In some embodiments, a substrate for the bioluminescent reporter is provided. In some embodiments, a bioluminescent reporter converts the substrate into a reaction product and releases light energy, e.g., luminescence, as a byproduct. In some embodiments, the substrate is a substrate for a luciferase enzyme. Appropriate substrates for known reporters are understood in the field.

As defined by the methods of the present invention, a fusion construct comprises a target protein fused to a *Oplophorus grachlorostris* luciferase (OgLuc) that has 70% or greater sequence identity with SEQ ID NO: 1. The fusion may comprise a natural (e.g., wild-type or variant) OgLuc sequence or may comprise a synthetic OgLuc (e.g., optimized for one or more characteristics (e.g., luminescence, signal stability, protein stability, etc.), etc.). The natural wild-type OgLuc sequence is given in SEQ ID NO: 1. Some suitable *Oplophorus* luciferases are described, for example, in U.S. Pat. No. 8,669,103 and U.S. 8,557,970. In some embodiments, a luciferase polypeptide comprises at least >70%, >75%. >80%, >85%, >90%, >95%, >98%, >99%, 100%, and any ranges therein) sequence identity with SEQ ID NO: 1. In some embodiments, comprises a amino acid substitutions at positions relative to one or more of positions: 1, 2, 4, 6, 10, 11, 14, 15, 16, 18, 19, 20, 21, 22, 23, 24, 25, 27, 28, 31, 32, 33, 34, 36, 38, 39, 40, 42, 43, 44, 45, 46, 47, 48, 49, 50, 51, 54, 55, 56, 58, 59, 60, 66, 67, 68, 69, 70, 71, 72, 74, 75, 76, 77, 86, 87, 89, 90, 92, 93, 94, 95, 96, 97, 98, 99, 100, 102, 104, 106, 109, 110, 111, 112, 113, 115, 117, 119, 124, 125, 126, 127, 128, 129, 130, 135, 136, 138, 139, 142, 143, 144, 145, 146, 147, 148, 149, 150, 152, 154, 155, 159, 158, 163, 166, 167, 168, or 169 of SEQ ID NO: 1. In some embodiments, a luciferase exhibits one or more of enhanced luminescence, enhanced signal stability, and enhanced protein stability relative to a wild-type *Oplophorus* luciferase. In some embodiments, comprises at least 60% (e.g., >65%, >70%, >75%. >80%, >85%, >90%, >95%, >98%, >99%, 100%, and any ranges therein) sequence identify with SEQ ID NO: 2, providing there is 70% or greater sequence identity with SEQ ID NO: 1. In some embodiments, comprises SEQ ID NO: 2.

In some embodiments, a target protein is fused to a first peptide or polypeptide (e.g., that does not independently exhibit substantial detectable activity) that forms an active reporter construct through structural complementation with a second polypeptide or peptide (See, e.g. U.S. Ser. No. 14/209,610 and U.S. Ser. No. 14/209,546). In such embodiments, only one of the two (or more) elements (peptide or polypeptide) that form the reporter construct is fused to the target. The structural complement of the fused element of the reporter construct is added separately to the system (e.g., cell (e.g., added exogenously, expressed by the cell), cell lysate, *in vitro* system, etc. In particular embodiments, a target protein is fused to a first peptide or polypeptide (e.g., that doesn't independently exhibit a detectable activity) that forms an active luciferase construct through structural complementation with a second polypeptide or peptide (See, e.g. U.S. Ser. No. 14/209,610 and U.S. Ser. No. 14/209,546). In such embodiments, the fusion polypeptide will not independently catalyze a significant amount of substrate a high-energy-state product that will produce light upon return to a stable state. Rather, only in the presence of the complement polypeptide or peptide is the active luciferase construct formed. Embodiments will typically be described as comprising a target fused to a complete reporter; however, unless indicated otherwise, it should be understood that the reporter may be formed by structural complementation of multiple (e.g., 2, 3, or more) elements, only one of which is fused to the target.

In some embodiments, a first peptide (e.g., fused to the target protein) comprises at least 60% (e.g., 65%, 70%, 75%. 80%, 85%, 90%, and any ranges therein) but less than 100% sequence identify with SEQ ID NO: 3, and a complement polypeptide comprises at least 60% (e.g., 65%, 70%, 75%. 80%, 85%, 90%, 95%, and any ranges therein) but less than 100% sequence identify with SEQ ID NO: 4, providing there is 70% or greater sequence identity with SEQ ID NO: 1. In some embodiments, the first peptide comprises an amino acid substitutions at positions relative to one or more of positions: G157del, T159S, C164F, E165K, N166K, L168S, A169del of SEQ ID NO: 3 (wherein SEQ ID NO: 3 numbering is 157-169). In some embodiments, the complement polypeptide comprises a amino acid substitutions at positions relative to one or more of positions: Q11E, G15A, F31L, G35A, L46R, G51A, G67A, G71A, M75E, I76V, H93P, I107L, D108N, N144T, L149M, 157S (addition of S at 157 position) of SEQ ID NO: 4 (wherein SEQ ID NO: 4 numbering is 1-156).

In some embodiments, a first polypeptide (e.g., fused to the target protein) comprises at least 60% (e.g., 65%, 70%, 75%. 80%, 85%, 90%, 95%, and any ranges therein) but less than 100% sequence identify with SEQ ID NO: 4, and a complement polypeptide comprises at least 60% (e.g., 65%, 70%, 75%. 80%, 85%, 90%, and any ranges therein) but less than 100% sequence identify with SEQ ID NO: 3, providing there is 70% or greater sequence identity with SEQ ID NO: 1. In some embodiments, the first polypeptide comprises a amino acid substitutions at positions relative to one or more of positions (e.g., Q11E, G15A, F31L, G35A, L46R, G51A, G67A, G71A, M75E, I76V, H93P, I107L, D108N, N144T, L149M, 157S (addition of S at 157 position) of SEQ ID NO: 4 (wherein SEQ ID NO: 4 numbering is 1-156)). In some embodiments, the complement peptide comprises a amino acid substitutions at positions relative to one or more of positions (e.g., G157del, T159S, C164F, E165K, N166K, L168S, A169del of SEQ ID NO: 3 (wherein SEQ ID NO: 3 numbering is 157-169)).

Depending upon the identity of the bioluminescent reporter used, an appropriate substrate will be selected, for example, from those including, but not limited to: firefly luciferin, latia luciferin, bacterial luciferin, coelenterazine, dinoflagellate luciferin, vargulin, and suitable derivatives thereof. In some embodiments, the substrate is a substrate for an *Oplophorus* luciferase, e.g., NANOLUC enzyme from Promega Corporation (e.g., SEQ ID NO: 2). In some embodiments, the substrate comprises coelenterazine, a coelenterazine derivative, a structural or functional equivalent of coelenterazine, a molecule substantially equivalent to coelenterazine (e.g., structurally and/or functionally), or molecule functionally or structurally similar to coelenterazine. In some embodiments, the bioluminescent reporter converts the coelenterazine, coelenterazine derivative, structural or functional equivalent of coelenterazine, or substantial equivalent to coelenterazine into coelenteramide, a coelenteramide derivative, a structural or functional equivalent of coelenteramide, or a substantial equivalent to coelenteramide and releases light energy as a byproduct.

As also disclosed herein but not forming part of the claimed invention, a reporter is an epitope tag (See Figure 21, top panel). In such examples not forming part of the claimed invention, a labeled (e.g., fluorescently labeled) antibody that recognizes and binds the epitope is included. In some examples not forming part of the claimed invention, BRET occurs between the label on the antibody and the fluorescent dye when both are bound to the epitope-tagged target protein. In some examples not forming part of the claimed invention, the epitope is accessible to the antibody when the protein is folded or unfolded. In other examples not forming part of the claimed invention, some degree of target unfolding is required for the antibody to access the epitope tag. In some examples not forming part of the claimed invention, following a thermal denaturation step, addition of a detection antibody labeled with a donor fluorophore (e.g. terbium, europium, etc.) is used in a FRET assay with denaturation/aggregation-sensitive (e.g., environmentally-sensitive dye, hydrophobic dye, etc.) dye as a FRET acceptor. Ligand-mediated thermal stabilization results in a loss of FRET/TR-FRET signal and/or the requirement of more denaturing conditions (e.g., higher temperature) to achieve the FRET/TR-FRET signal.

In some examples not forming part of the claimed invention, two epitope tags (e.g. FLAG-V5) are fused to a target protein (See Figure 21, bottom panel). In such examples not forming part of the claimed invention, the epitopes are accessible to separate, differently-labeled antibodies (e.g., donor and acceptor labeled) for the respective epitopes when the target protein is folded (FRET signal is produced). Upon partial or complete unfolding of the target, the epitopes become unavailable and the FRET signal is lost.

In some embodiments, the formation of a fusion protein does not substantially impact or alter the stability or function of either the target or reporter relative to the function of each individual protein. For example, for a fusion of target enzyme and a luciferase, the formation of the fusion does not substantially alter the stability of either portion, the luminescence of the luciferase, the signal stability of the luciferase, or the activity of the enzyme.

In some embodiments, the target polypeptide and reporter polypeptide luciferase are connected directly without intervening amino acid sequence. For example, the target polypeptide may be fused to the N-terminus or the C-terminus of the reporter. In other embodiments, the target polypeptide and reporter polypeptide luciferase are connected by a linker moiety or connector sequence. In some embodiments, a linker provides connection and allows a desired amount of space/distance between the elements (e.g., to reduce interactions between target and reporter). Although a linker is typically a peptide linker, in some embodiments, other chains or polymers may be utilized. In some embodiments, a connector sequence comprises or consists of one or more amino acids (e.g., a peptide or polypeptide chain of 2-50 amino acids (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, and any ranges therein). In some embodiments, the presence of a connector sequence in a fusion protein does not substantially impact or alter the stability or function of either the target or reporter relative to the function of each individual protein. For example, for a fusion of target enzyme and a luciferase, the presence of a connector sequence does not substantially alter the stability of either portion, the light output of the luciferase, or the activity of the enzyme. In some embodiments, a connector sequence is included in a fusion to prevent interactions between the target and reporter that could affect the stability of activity of either or both portions. For any particular combination of proteins in a fusion, a wide variety of connector sequences may be employed. In one embodiment, the connector sequence is a sequence recognized by an enzyme, e.g., a cleavable sequence. For instance, the connector sequence may be one recognized by a caspase or TEV protease, or may be a chemically-cleavable or photocleavable sequence.

In some embodiments, the activity (e.g., luminescence) of a reporter luciferase fused to a target protein is directly detected. In some embodiments, the activity (e.g., luminescence) of a reporter construct (e.g., luciferase dimer), a portion of which is fused to a target protein (e.g., the structural complement portion is not fused to the target protein), is directly detected. In some embodiments, reporter activity is measured at one or more temperatures as a measure of target protein stability. In some embodiments, at lower temperatures (e.g., <50°C, <48°C, <46°C, <44°C, <42°C, <40°C, <38°C, <36°C, 34°C, <32°C, <30°C, and ranges therein (e.g., 30-50°C, 35-45°C, etc.), depending upon the target and reporter stabilities) both the target and reporter are stably folded. As temperatures are increased, and one or both of the target and reporter begin unfolding or aggregating, signal from the reporter decreases (e.g., the activity of the reporter decreases as it becomes unfolded or aggregated). In some embodiments, because the reporter and target are fused, a more stable the target protein results in a more stable reporter and reporter signal. Similarly, a less stable target results in destabilization of the reporter and a loss, or reduction, of reporter signal at a lower temperature. In some embodiments, if a ligand binds to the target protein, the target protein is stabilized. Since, in some embodiments, stabilization of the target protein results in stabilization of the reporter and enhancement of signal stability, binding of the ligand to the target can be observed as an increase in the stability of the reporter signal. Embodiments in which the signal from the reporter (e.g., luciferase) at a given temperature (e.g., around the Tₘ of the protein) or temperature range is directly used as a measure of protein stability are referred to herein as "direct detection."

In some embodiments, ligand binding destabilized the target protein. In some embodiments, the reporter is thermally stable or labile compared to the protein of interest it is fused to.

In some embodiments, the activity (e.g., luminescence) from a target/reporter fusion is measured at one or more temperatures (e.g., over a range of temperatures (e.g., from below the Tₘ of the target protein to above the Tₘ of the target protein)), both in the presence and absence of a ligand for the target or a test ligand. If the ligand or test ligand binds to or interacts with the target protein under the conditions assayed (e.g., in a cell, in a cell lysate, etc.), a corresponding shift in the activity of the reporter is observed. For example, at a given temperature (e.g., near the Tₘ of the target protein) the activity (e.g., luminescence (e.g., RLUs)) of the reporter (e.g., luciferase) is increased. If the unfolding of the protein is analyzed at a range of temperatures, so as to determine a Tₘ, ligand binding and stabilization of the target protein is apparent as a shift (e.g., right shift) in the Tₘ to a higher temperature.

Traditional thermal shift assays utilize purified target protein and a dye that (1) binds nonspecifically to hydrophobic surfaces and (2) is quenched by water (e.g., Sypro Orange). In such an assay, at lower temperatures (e.g., <50°C, <48°C, <46°C, <44°C, <42°C, <40°C, <38°C, <36°C, 34°C, <32°C, <30°C, and ranges therein (e.g., 30-50°C, 35-45°C, etc.), depending upon the target stability) the target is stably folded, and the hydrophobic regions of the target are buried and inaccessible to the hydrophobic dye. Under these folded conditions, the dye cannot significantly bind to the target protein, and therefore fluorescence from the dye is quenched by water in the aqueous environment. As temperatures are increased, the target begins unfolding and/or aggregating, the hydrophobic regions of the target become reveled, and the dye binds to the target. Upon binding the target protein, the dye becomes unquenched and the fluorescent signal increases. The more stable the target, the higher the temperature required to observe the fluorescence increase. Similarly, a less stable target results in destabilization of the target and an increase in fluorescent signal at a lower temperature. In such an assay, if a ligand binds to the target protein, the target protein is stabilized, which can be observed as an increase in the temperature required to generate the fluorescent signal.

The traditional TSA relies on nonspecific binding of the dye (e.g., Sypro Orange) to the hydrophobic regions of the target protein. Therefore, if proteins other than the target protein are present in the assay, the dye will bind to those proteins as well (e.g., as they become unfolded), thereby confounding the results. For at least this reason, traditional TSAs are performed using purified target protein and cannot be performed in cells, cell lysate, or other complex environments.

The direct detection embodiments described above overcome the limitation of the traditional TSA by detecting activity of a reporter fused to the target protein. The unfolding of proteins not fused to the reporter (e.g., proteins present in the cellular milieu or cell lysate) do not directly affect the signal. In another embodiment, a BRET TSA is provided that makes use of the target-specificity of the direct-detection embodiments described herein (e.g., only the unfolding of proteins fused to a reporter are monitored (e.g., even in a complex environment)), as well as the sensitivity of the traditional TSA (e.g., the increase in fluorescence from the dye is directly correlated with the degree of unfolding of the target protein). In such embodiments, a fusion of a target protein and reporter luciferase is generated, and fluorescence measurements are taken in the presence of a hydrophobic dye that: (1) binds nonspecifically to aggregated, unfolded, or denatured proteins and/or hydrophobic peptide segments, (2) has an excitation spectra that significantly overlaps the emission spectra of the reporter, and optionally (3) the fluorescence of which is quenched by water. The activity of the dye may be quenched by water (e.g., as in a traditional TSA), or the dye may be active (e.g., capable of emitting light at a particular wavelength in response to an excitation) whether bound to a protein or free in solution. In some embodiments, because the dye will be excited via BRET from the reporter and not directly, the capacity for the dye to fluoresce when not bound to the target (e.g., when bound to other proteins within a cell or cell lysate.) does not affect the assay (e.g., because only target-bound dye is within the range (e.g., 1-10 nm) to receive energy transfer from the reporter). In some embodiments, at lower temperatures (e.g., <50°C, <48°C, <46°C, <44°C, <42°C, <40°C, <38°C, <36°C, 34°C, <32°C, <30°C, and ranges therein (e.g., 30-50°C, 35-45°C, etc.), depending upon the target stability) the target is stably folded, and the hydrophobic regions of the target are buried and inaccessible to the hydrophobic dye. Under these folded conditions, signal from the reporter (e.g., fused to the target) is detected, but because the dye cannot significantly bind to the folded target protein (e.g., because hydrophobic regions are no accessible on the folded target) fluorescence from the dye as a result of BRET is not detected (e.g., significant direct reporter signal is observed, but BRET to the dye is not observed, is decreased, and/or is minimal). The proximity limitation of BRET prevents significant energy transfer from occurring between the reporter and unbound dye. In embodiments in which the dye is quenched by water, unbound dye is also not detected by direct excitation (e.g., from a light source). In embodiments in which the dye maintains its activity in water, while not producing a signal from BRET, the unbound dye may be detected by direct excitation (e.g., from a light source). As temperatures are increased, the target begins unfolding and/or aggregating, the hydrophobic regions of the target become revealed, the dye binds to the target, and the dye becomes unquenched (e.g., the quantum yield of the dye increases). Under these partially-folded or unfolded conditions, BRET occurs from the reporter to the bound dye (e.g., dye bound to the revealed hydrophobic portions of the target protein), and fluorescence from the dye, as the result of BRET, is detectable as a direct measure of (e.g., is proportional to) target protein unfolding. The more stable the target, the higher the temperature required to observe the increase in BRET. Similarly, a less stable target results in destabilization of the target and an increase in the BRET signal at a lower temperature. In such an assay, if a ligand binds to the target protein, the target protein is stabilized, which can be observed as an increase in the temperature required to generate the BRET signal (e.g., to generate increased BRET signal (e.g., increased over background)). In BRET TSA, although the hydrophobic dye will bind to any proteins present in the complex sample (e.g., cell, cell lysate, etc.), only the target protein is fused to the reporter, therefore the BRET signal will be specific to the target protein. Due to the proximity constraints placed on BRET, significant energy transfer only occurs between the reporter and target-bound dye. The BRET signal is made possible by 1) presence of reporter (e.g., NLuc) activity and 2) binding of the dye to the fusion protein allowing the reporter to donate and excite the dye to emit at a certain wavelength. The fusion of the reporter to the target provides the target specificity; the dye provides a readout of the target protein state (e.g., folded, partially folded, unfolded, etc.).

In some embodiments, BRET TSAs described herein utilize fluorogenic dyes. In other embodiments, dyes are non-fluorogenic.

In some embodiments, BRET TSAs described herein utilize fluorescent dyes that: (1) are quenched by an aqueous environment (e.g., when not bound by a target); (2) interact with (e.g., bind) hydrophobic surfaces, such a hydrophobic peptide segments; and (3) exhibit enhanced or increased fluorescence upon binding a hydrophobic surface.

Certain fluorescent compounds exhibit only a weak fluorescence emission when free in aqueous solution (Semisotnov, et al., Biopolymers 31:119, 1991), but fluoresce much more strongly when bound to organized hydrophobic surfaces. Binding of these compounds to fully folded globular proteins is typically weak, since hydrophobic residues are predominantly buried in the interior of the protein. Furthermore, binding of these compounds to random coil conformations (as found in fully unfolded or denatured polypeptides) is also disfavored because in these conformations hydrophobic residues, though exposed, are not sufficiently well organized to support high affinity binding of the probes. The dyes, however, typically bind with higher affinity and stoichiometry to compact unfolded protein conformations, such as "molten globules", which are characterized by compactness relative to random coil unfolded states, the presence of substantial secondary structure, and the lack of a unique overall conformation. These probes may be referred to herein as "environmentally-sensitive hydrophobic dyes."

In some embodiments, BRET TSAs described herein utilize fluorescent dyes that bind to the hydrophobic regions of unfolding proteins, molten globules, and aggregates. Due to the proximity limitations of BRET, significant energy transfer from the bioluminescent report to the fluorescent dye only occurs when the dye is bound to the unfolding target protein. When the target protein is fully folded, the fluorescent dye does not bind the target protein, the dye is not within the proximity of the bioluminescent reporter, and BRET does not occur. In such embodiments, the bioluminescent reporter provides the system's specificity for the target protein while the fluorescent dye provides the measure of protein unfolding.

Examples of dyes that find use in embodiments described herein include SYPRO Orange, 1-anilino-8-naphthalene sulfonate (ANS) , bis-1-anilino-8-naphthalene sulfonate (bis-ANS), 6- propionyl-2- (N,N-dimethyl) -aminonaphthalene (Prodan) (Molecular Probes, Eugene, OR), SYPRO Red, SYPRO Ruby, SYPRO Tangerine, and Nile Red. Other suitable dyes are those listed in Table 1.

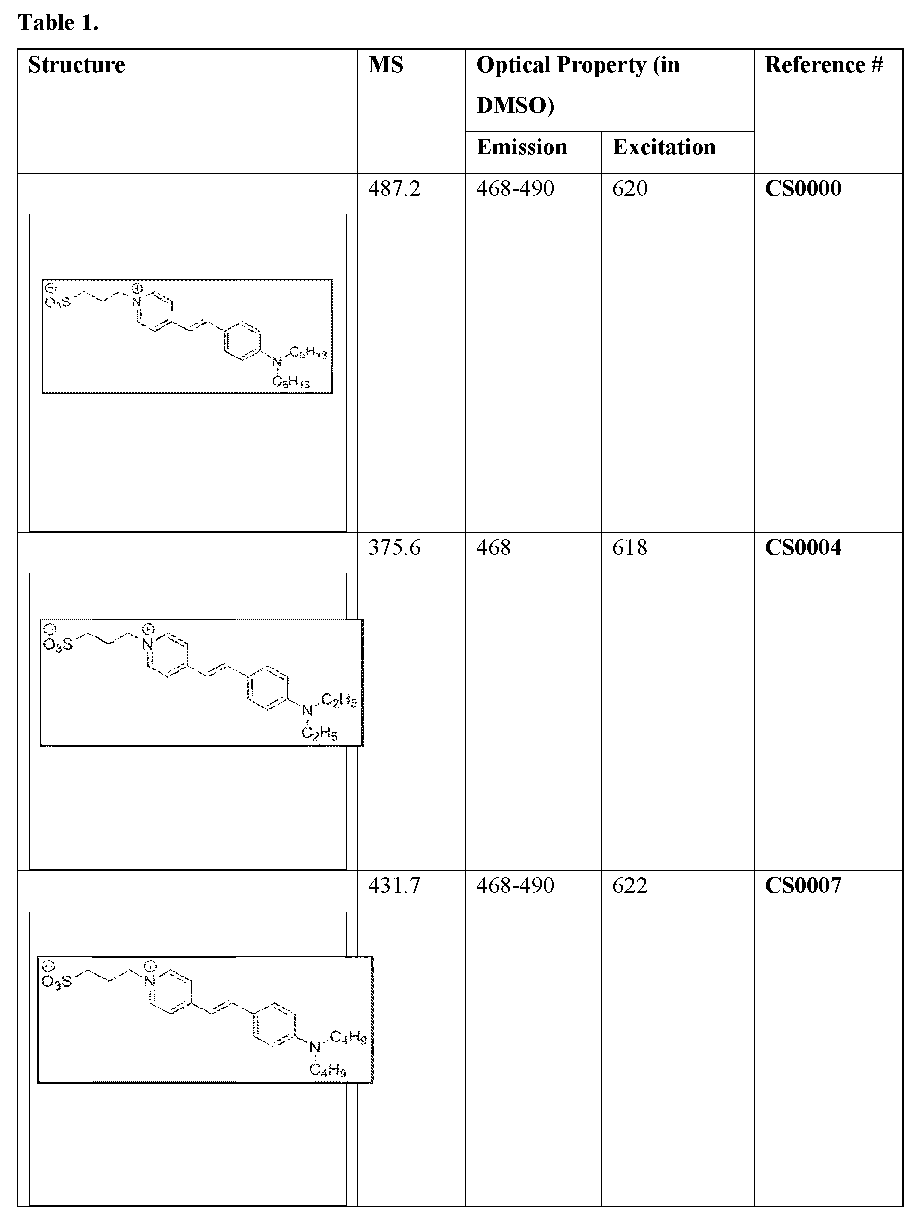

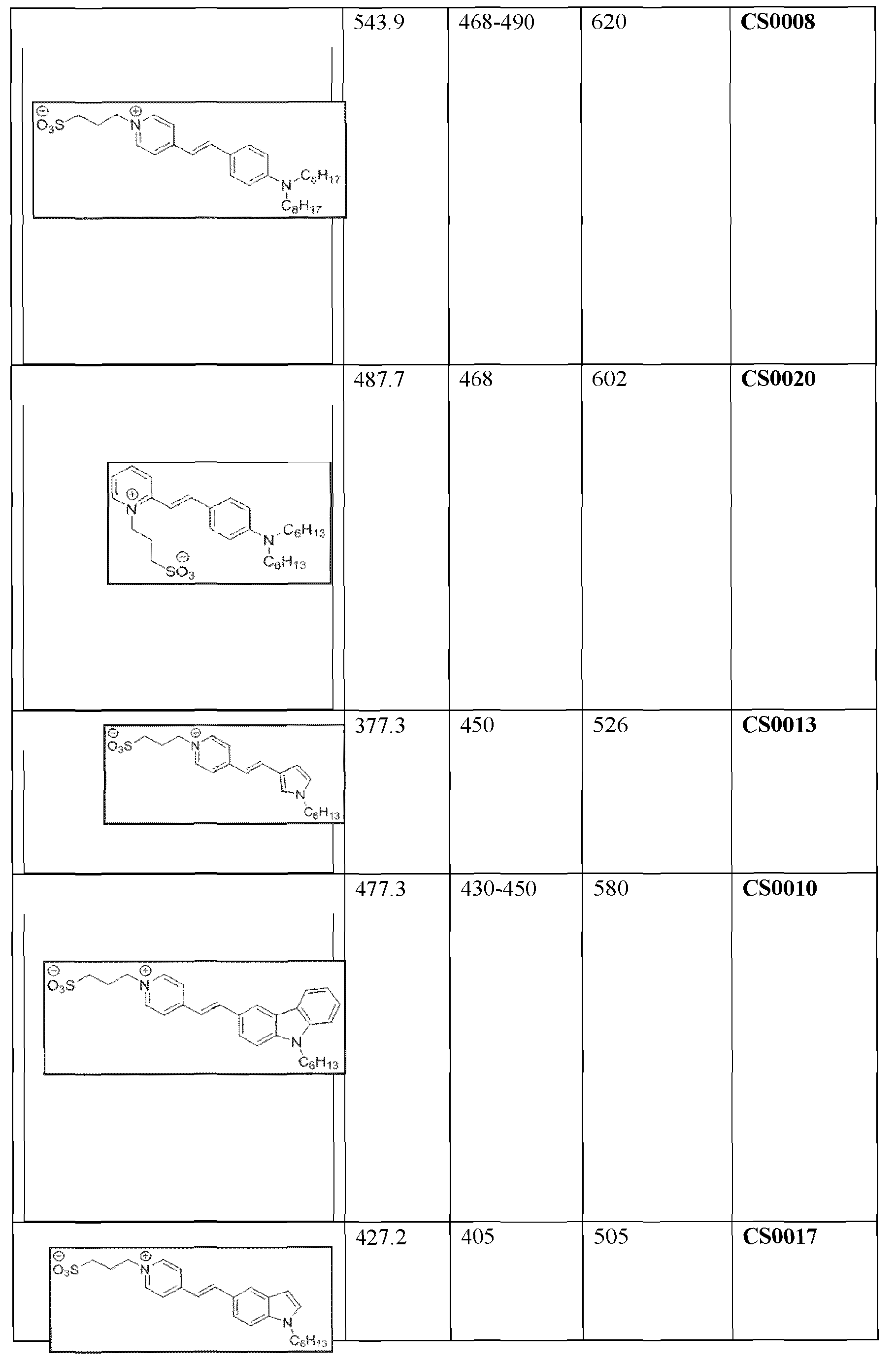

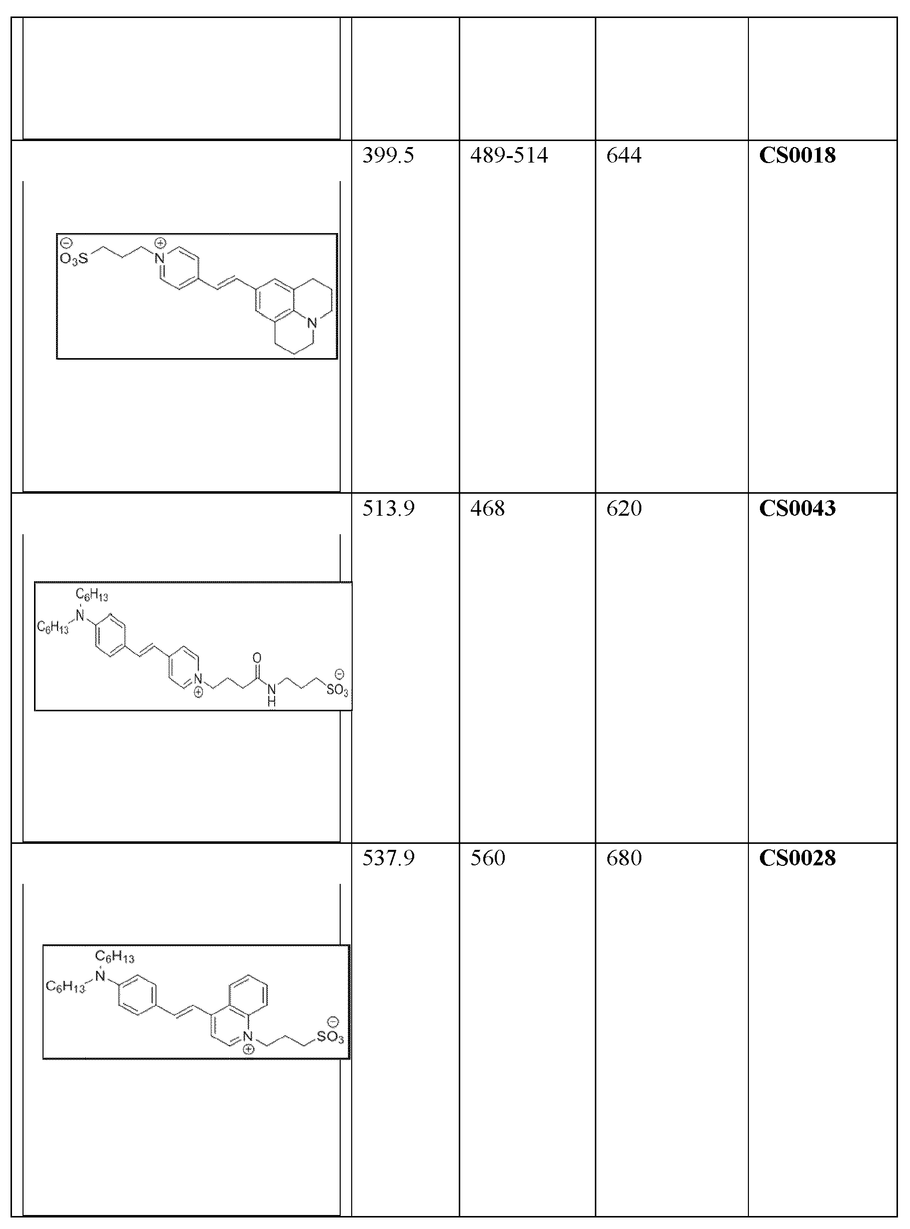

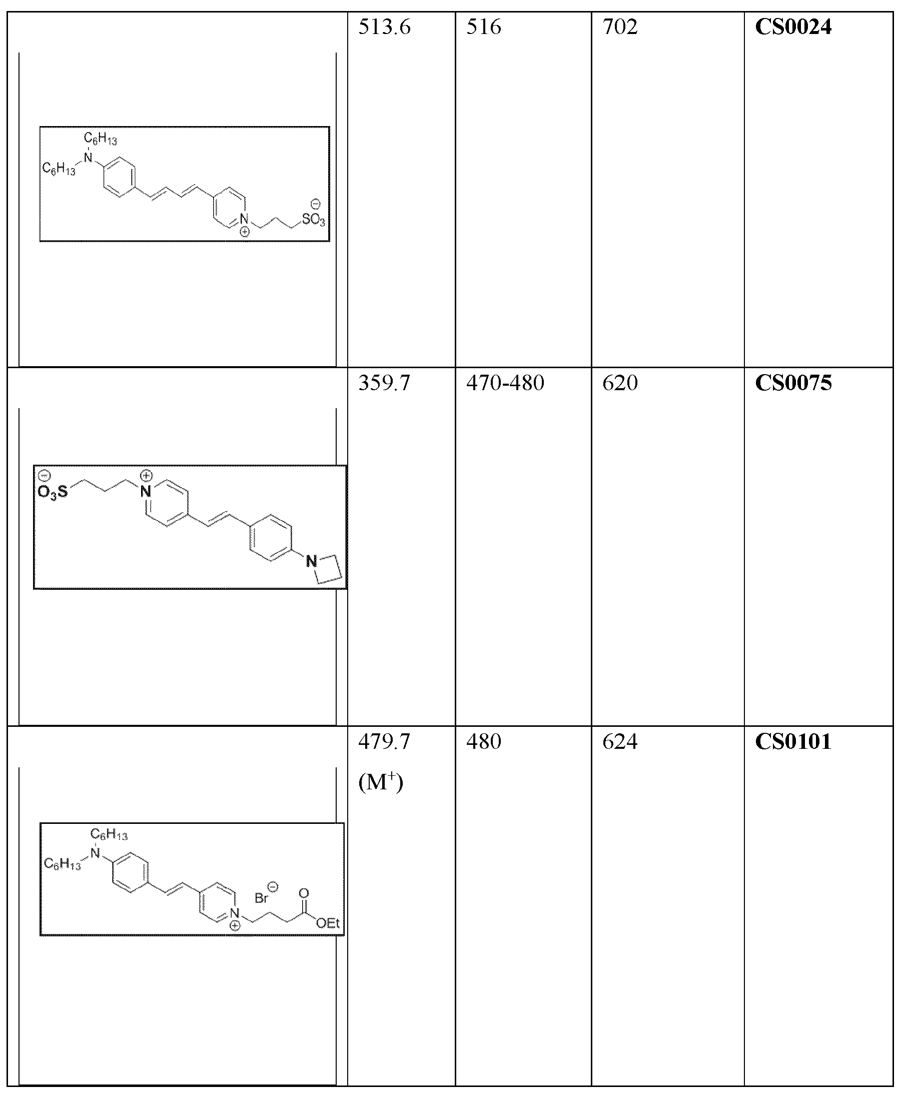

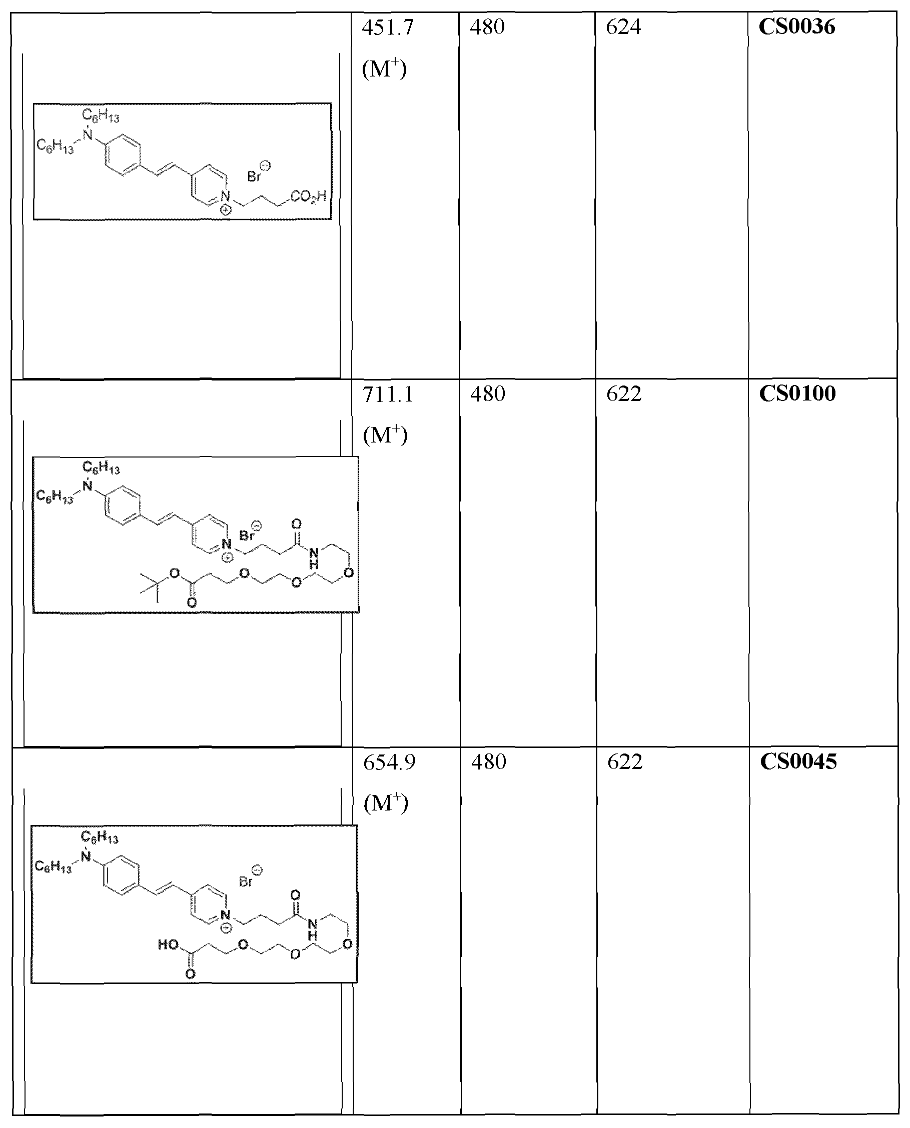

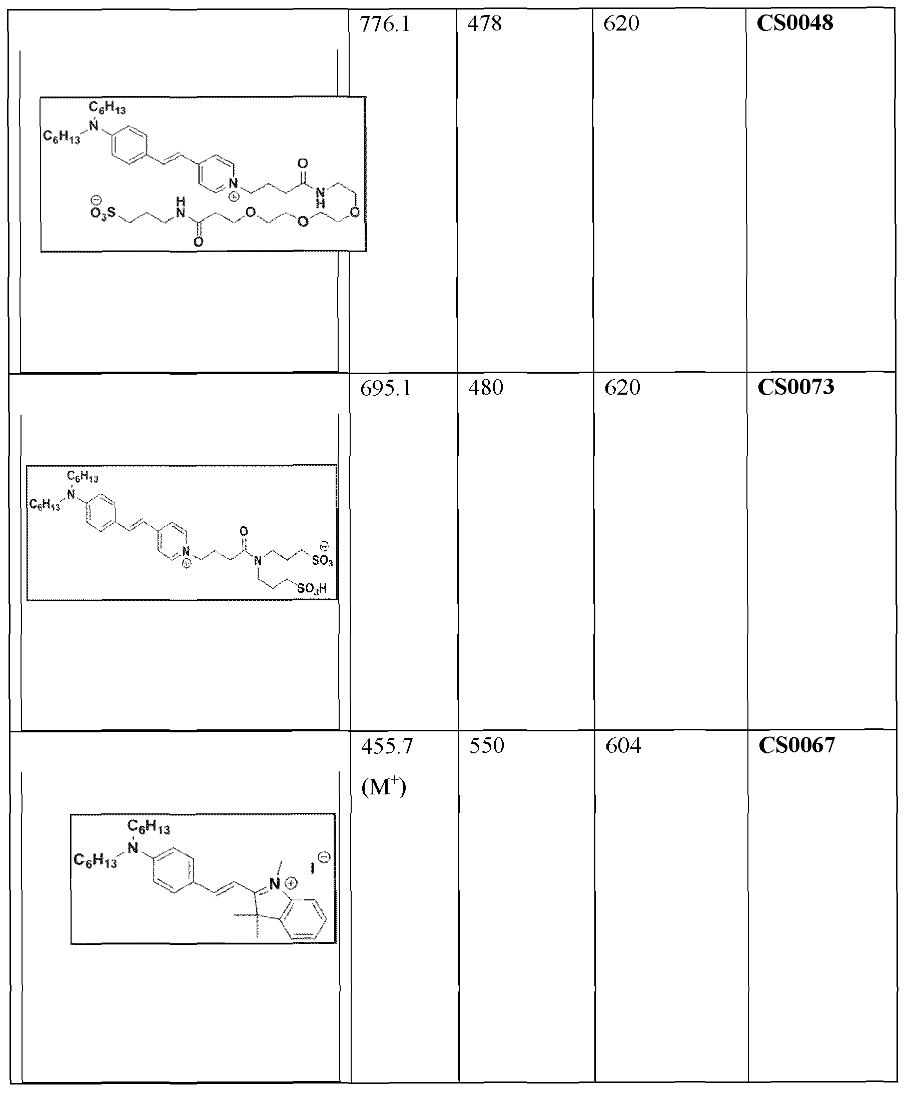

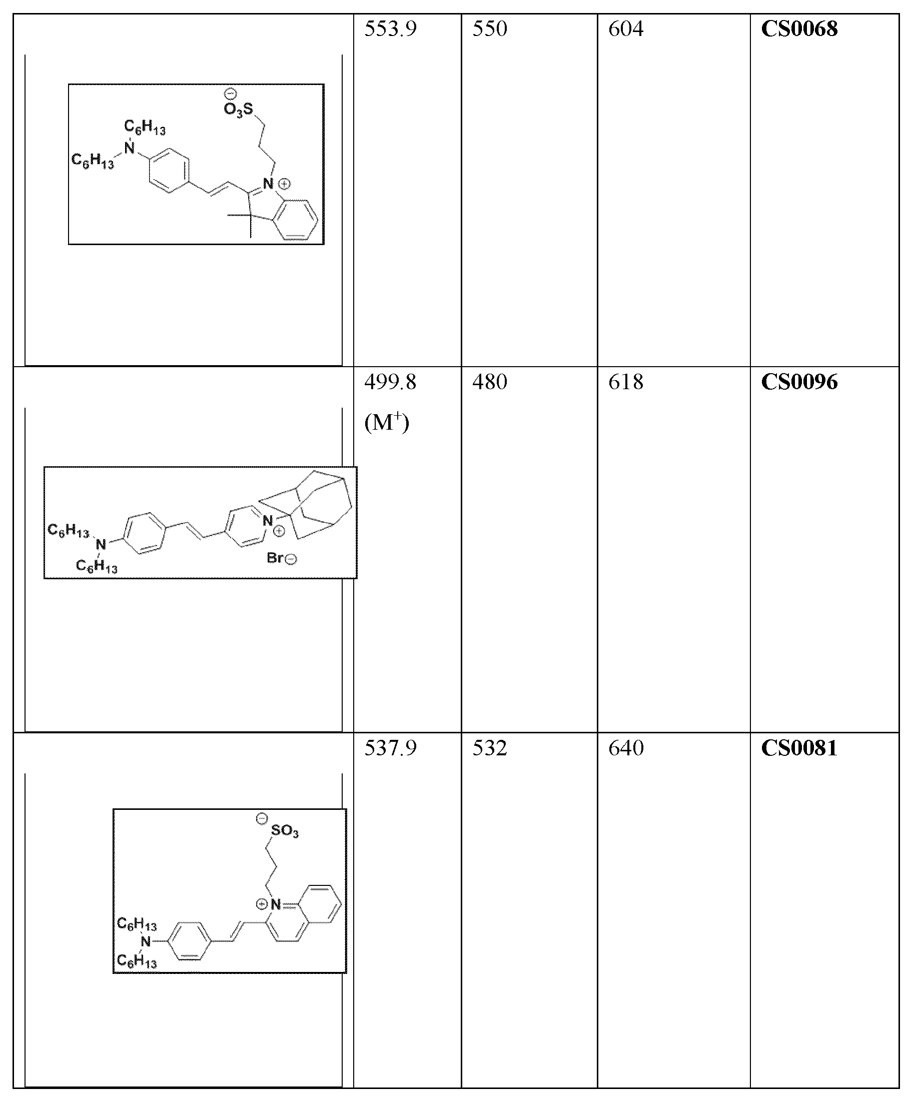

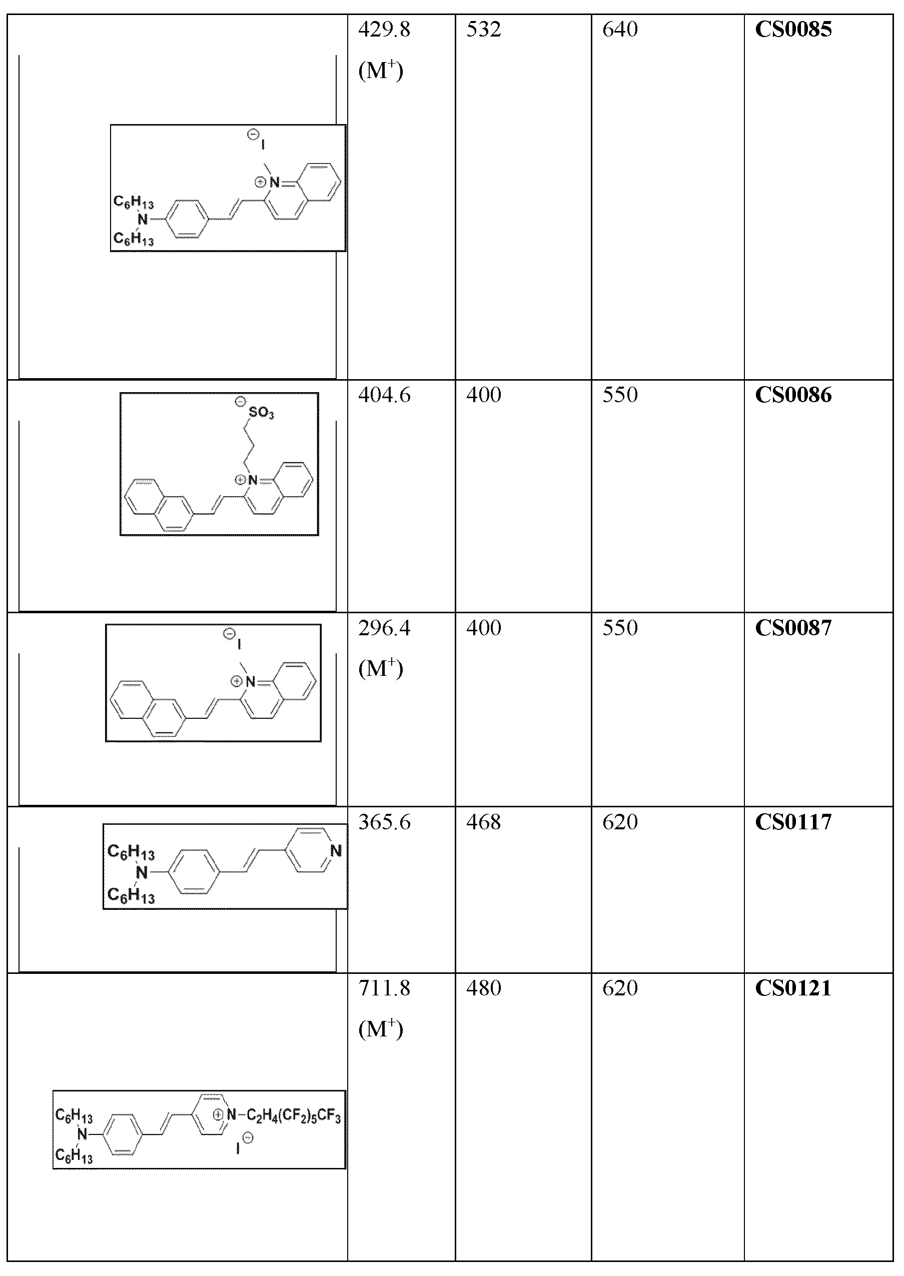

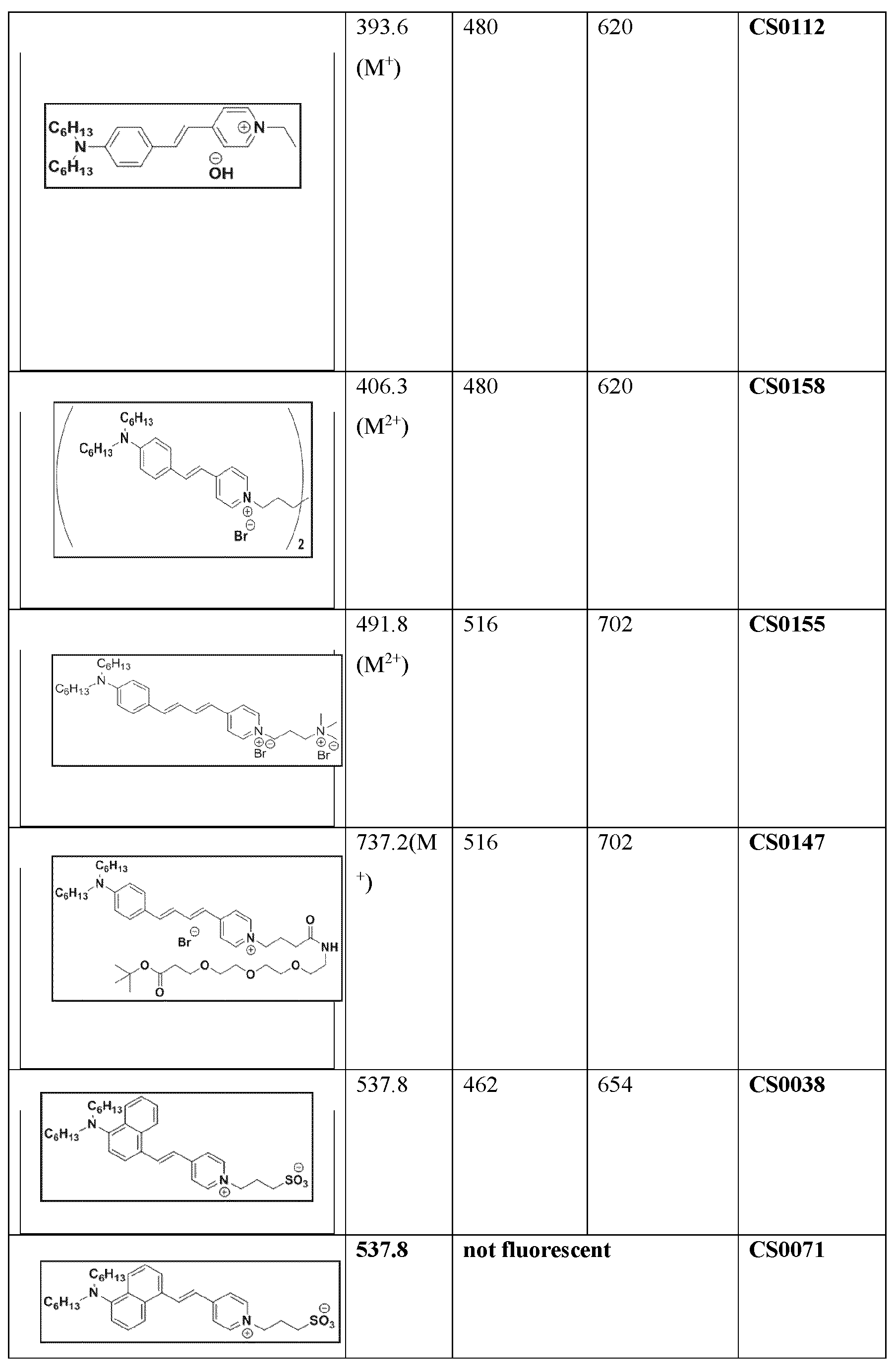

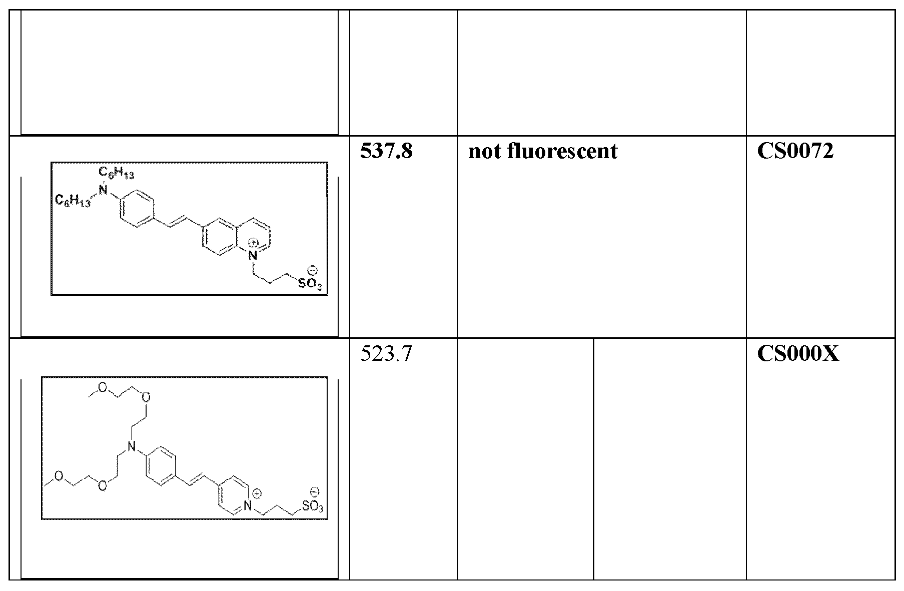

In some embodiments, combinations of dyes are used. In some embodiments, a combination of dyes having varying binding properties is used, such that the dye mix finds use with target proteins having different structural characteristics. Other dyes (e.g., dyes known in the field) may also find use in embodiments described herein.

One advantage of some embodiments described herein is the ability of the assays to be performed in complex samples (e.g., those containing non-target proteins). In some embodiments, samples that find use with the TSAs described herein include cells (e.g., cell culture, cell sample from a patient, etc.), tissue samples (e.g., tissue from biopsy), cell lysates (e.g., lysed of cells expressing a target/reporter fusion, cell lysate expressing target/reporter fusion *in vitro,* cell lysate with exogenous target/reporter fusion, etc.), complex reaction mixtures (e.g., *in vitro* reaction mixtures comprising the components necessary for the assay as well as non-essential components (e.g., non-target proteins, competitor proteins, competitor ligands, etc.), simple *in vitro* reaction mixtures comprising the components necessary for the assay, etc.), etc.

In some embodiments, reporter and/or BRET signal is detected under conditions that favor protein folding and/or conditions that favor some degree of protein unfolding. Two means of varying conditions are temperature and protein denaturants. For example, lower temperatures (e.g., <50°C, <48°C, <46°C, <44°C, <42°C, <40°C, <38°C, <36°C, 34°C, <32°C, <30°C, and ranges therein (e.g., 30-50°C, 35-45°C, etc.)) favor protein folding, while higher temperatures (e.g., >50°C, >55°C, >60°C, >65°C, >70°C, >75°C, >80°C, >85°C, >90°C, >95°C, and ranges therein (e.g., 60-90, °C 55-75°C, etc.)) favor protein unfolding, exposure of hydrophobic regions, and/or aggregation. Similarly, the presence of various denaturants and increasing concentrations thereof, favor protein unfolding. Numerous protein denaturants are known in the art and could be empirically selected for use herein. Without being limited to a particular protein denaturant, exemplary protein denaturants include guanidinium thiocyanate, guanidinium hydrochloride, arginine, sodium dodecyl sulfate (SDS), urea, or any combination thereof. In some embodiments, a combination of one or more protein denaturants and increased temperature is employed.

In some embodiments, the reporter signal and/or BRET signal (e.g., the result of energy transfer from bioluminescent protein to environmentally-sensitive hydrophobic dye) is detected under a single condition, and a comparison is made between the signals in the presence and absence of ligand. In such embodiments, the signal is typically detected under partially denaturing conditions, or conditions in which the unbound target protein is beginning to unfold, but it is expected that the ligand-bound target will not be unfolding or will be less unfolded (as will be apparent from the change in signal (e.g., direct reporter signal or BRET signal, depending upon the assay)). Appropriate conditions for such a single-point assay can be determined empirically based on the stability of the particular target protein. In some embodiments, conditions (e.g., temperature and/or denaturant concentration) are selected at or near (e.g., ±5%, ±10%, ±15%, ±20%, ±25%, ±30%, ±35%, ±40%, ±45%, ±50%) the melting temperature (Tₘ) for the target protein. The melting temperature (e.g., in the presence of the intended denaturant concentration) of the target protein can be determined using any of a variety of well-known techniques, including, but not limited to: UV/visible spectrometry, nuclear magnetic resonance spectroscopy (NMR), circular dichroism (CD), etc.

In other embodiments, the reporter signal and/or BRET signal (e.g., the result of energy transfer from bioluminescent protein to environmentally-sensitive hydrophobic dye) is detected under multiple conditions or over a range of conditions (e.g., varying temperature and/or denaturant concentration), and a comparison is made between the signals in the presence and absence of ligand. In such embodiments, signal may be detected at a first condition in which both the ligand-bound and inbound target are expected to be well folded. Under such conditions, similar signals are typically (although not always) expected in both the presence and absence of ligand. In some embodiments, signal is also detected under one or more additional conditions (e.g., increasing temperature, increasing pressure, and/or increasing denaturant concentration) approaching and/or exceeding the expected Tₘ (e.g., under the denaturant concentration) of the target protein in the absence of ligand. As increasingly unfavorable folding conditions are applied, a difference will be observed between the signal (e.g., from a direct reporter signal or BRET signal, depending upon the assay) from a ligand-bound sample and an unbound target sample.

The number of conditions to assay may be made by weighing factors such as: desired speed of the assay, desired precision/accuracy of the measurements, affinity of binding to be detected, etc. For example, in some embodiments, an assay detects signal under a first condition in which the target is well-folded, a second condition near the Tₘ of the unbound target, and a third condition in which the unbound target is expected to be unfolded and/or aggregated. These conditions may be determined empirically using the methods described herein or other techniques for monitoring protein folding that are known in the field. As another example, an assay makes stepwise (e.g., every 0.1°C, every 0.2°C, every 0.5°C, every 1.0°C, every 2.0°C, every 5.0°C, etc.) signal detections (e.g., from reporter and/or BRET) over a range of temperatures (e.g., from: 10°C, 15°C, 20°C, 25°C, 30°C, 35°C, 40°C, etc.; to: 50°C, 55°C, 60°C, 65°C, 70°C, 75°C, 80°C, 85°C, 90°C, 95°C, etc.), and or a range of denaturant concentrations.

In some embodiments, a melt-curve analysis is performed for each sample, according to the detected signals at the various conditions assayed. In some embodiments, a visual or automated analysis of the melt-curves in the presence and absence of ligand reveals target/ligand interaction or binding. In some embodiments, a melting temperature (Tₘ) is calculated for each sample, according to the detected signals at the various conditions assayed. In some embodiments, a comparison of Tₘ's in the presence and absence of ligand reveals target/ligand interaction or binding.

In some embodiments, assays described herein are performed in cells, e.g., live intact cells, or in a lysate of cells. In such embodiments, a nucleic acid construct encoding a target/reporter fusion may be transfected or transformed into cells in order to allow expression of the fusion within cells (e.g., live intact cells, cells in which the assay will be performed, cells which will be lysed before the assay is performed, etc.). In some embodiments, assays described herein provide the detection of molecular interactions between a target and ligand within a cell, e.g., a live intact cell, or in living organisms (e.g., bacteria, yeast, eukaryotes, mammals, primates, human, etc.). In some embodiments, a target/reporter fusion protein is expressed in the cell, e.g., a live intact cell, or whole organism, and signal is detected under various conditions in the presence and absence of ligand. In some embodiments, cells are transiently and/or stably transformed or transfected with vector(s) (e.g., encoding target/reporter fusion protein, structural complement (e.g., NLPep and NLPoly), etc.). In some embodiments, transgenic organisms are generated that code for the necessary assay components (e.g., target/reporter fusion protein, structural complement (e.g., NLPep and NLPoly), etc.) for carrying out the assays described herein. In other embodiments, vectors are injected into whole organisms. A suitable vector may be viral (e.g., lentivirus, AAV, etc.) or non-viral (e.g., plasmid, bacmid, etc.). In some embodiments, in addition to encoding a target/reporter fusion, a vector further comprises elements to allow/promote expression of as much.

In some embodiments, other assay components not expressed in cells or organisms (e.g., reporter substrate, ligand, environmentally-sensitive hydrophobic dye, etc.) are added to the cell or organism exogenously. In embodiments in which assays are not performed *in vivo*, the target/reporter fusion may also be added exogenously.

Embodiments described herein provide methods that are useful in a variety of fields including basic research, medical research, drug discovery, etc.

Typical applications that make use of embodiments described herein involve the monitoring/detection of protein-ligand interactions. Such assays are useful for monitoring molecular interactions under any suitable conditions (e.g., *in vitro*, *in vivo*, *in situ*, whole animal, etc.), and find use in, for example, drug discovery, elucidating molecular pathways, studying equilibrium or kinetic aspects of complex assembly, high throughput screening, etc.

In some embodiments, assays are used to elucidate the affinity of, or understand the interaction of, a protein of interest and a potentially associated entity of interest (protein, nucleic acid, small molecule, etc.) under complex conditions (e.g., intracellularly).

Embodiments described herein find use in drug screening and/or drug development. For example, the interaction of a small molecule drug or an entire library of small molecules with target protein of interest (e.g., therapeutic target) is monitored (e.g., within a physiologically-relevant cell line, etc.). In some embodiments, drug screening applications are carried out in a high through-put format to allow for the detection of the binding of tens of thousands of different molecules to a target, or to test the effect of those molecules on the binding of a ligand.

In some embodiments, provided herein is the detection of target/ligand interactions in living organisms (e.g., bacteria, yeast, eukaryotes, mammals, primates, human, etc.) and/or cells. In some embodiments, cells are transiently and/or stably transformed or transfected with vector(s) (e.g., encoding target/reporter fusions, complement polypeptide, etc.). In some embodiments, transgenic organisms are generated that code for the necessary components (e.g., encoding target/reporter fusions, complement polypeptide, etc.) to carry out the assays described herein. In other embodiments, vectors are injected into whole organisms.

### EXPERIMENTAL

### Example 1

### Materials and Methods

### Method for Expression Plasmid Construction for Full Length NanoLuc-target fusions (applies to examples using full length NanoLuc)

To produce NanoLuc^{®} fusions with protein targets of interest, pF31K Nluc [CMV/Neo] and pF32 [CMV/Neo] were used to place NanoLuc at the N-terminus or the C-terminus of the target protein (respectively) using the manufacturer's protocol (Promega). cDNAs encoding the following target proteins were made and were a 100% match to their respective NCBI reference sequence identifiers; CDK2, DHFR, KDR, SRC, EZH2, HDAC1, BRD4, MAPK14, LCK, ABL, DDR1, and BTK.

### Method for Expression Plasmid Construction for NLPep and NLPoly NLPep and NLPoly target fusions (pep86-CDK2, CDK2-pep86, CDK2-Nluc156, CDK2-pep11S)

For plasmid constructions, the source of CDK2 was pFN21-CDK2 (Promega Flexi^{®} vector backbone) received from Kazusa DNA Research Institute (Chiba, Japan).

**Pep86-CDK2:** This construct was built by transferring CDK2 to the Flexi^{®}vector, pF-n5K3-86-GSSG-barnase, according to Promega's recommended protocols in the Flexi^{®} vector technical manual.

**CDK2-Pep86:** This construct was built by transferring CDK2 to the Flexi^{®}vector, pF-c5K3-barnase-GSSG-86, according to Promega's recommended protocols in the Flexi^{®} vector technical manual.

**CDK2-Nluc156:** This construct was built by first transferring CDK2 to the Flexi^{®} vector, pF-nCA-bamase-GSSG-Nluc, according to Promega's recommended protocols in the Flexi^{®} vector technical manual. The Nluc portion of this vector was then modified by site-directed mutagenesis to contain a G51A substitution. It was then further modified by site-directed mutagenesis to contain a deletion of the sequence coding for the final 13 amino acids of Nluc.

**CDK2-11S:** This construct was built by transferring CDK2 to the Flexi^{®}vector, pF-c5K3-barnase-GSSG-115, according to Promega's recommended protocols in the Flexi^{®} vector technical manual.

Each of the constructs contained a GSSG linker between CDK2 and either NLPep86, Nluc156, or NLpoly11S.

### Method for Generation of Purified Detection NLPep86 and NLPolyllS

NLPep86 (SEQ ID NO: 5) (containing an N-terminal acetyl and C-terminal amide) was made synthetically by Peptide 2.0. NLpoly11S (SEQ ID NO: 6)(NLpoly11S with C-terminal 6His tag) was isolated by transforming E. coli KRX cells (Promega) with the Flexi^{®} vector, pF6HisCK, and then overexpressing and purifying according to Promega's recommended protocol for isolating His-tagged proteins (HisLink^{®} Protein Purification technical manual ).

### General Protocol Cell Transfection for All Experiments:

NanoLuc-target or NLPep and NLPoly-target fusion constructs were diluted into carrier DNA (pGEM3ZF-, Promega) at a mass ratio of 1:10 or 1:100 (mass/mas) prior to forming FuGENE HD complexes according to the manufacturer's protocol (Promega). DNA:FuGENE complexes were formed at a ratio of 1:3 (ug DNA/uL FuGENE), then 1 part of the transfection complexes was then mixed with 20 parts (volume/volume) of HeLa cells (ATCC) suspended at a density of 2 × 10⁵ in DMEM (Gibco) + 10% FBS (Hyclone), followed by incubation in a humidified, 37°C/5% CO₂ incubator for 18-24 hours.

### Example 2

### Direct-luciferase-detection thermal shift assay with full length NanoLuc

Experiments were conducted during development of embodiments of the present invention to demonstrate direct detection of the stabilization of a target protein by the interaction with a target ligand via temperature dependent alteration of luminescent signal from a luciferase fused to the target protein.

To ensure the NanoLuc (Nluc) luminescent signal was originating from intact cells after the cells had been exposed to a temperature gradient, the integrity of the cells and cell membranes after heating was investigated as well as measuring Nluc activity in the cells versus in the supernatant. HeLa cells were heated to different temperatures, cooled at room temperature, mixed with trypan blue, and the absolute cell numbers and dye exclusion were measured. Parallel samples were spun down to pellet, and the cells and supernatant collected.

HeLa cells were transfected with CDK2-Nluc DNA at a 1:10 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in a 100uL/well volume, and the plates were placed into a thermal cycler (MW Research) where the samples were heated individually at different temperatures spanning a gradient of 42-66°C across the plate for 3 minutes followed by cooling at 22°C for 3 minutes. To determine the absolute cell count and viability, 10uL cell aliquot from each temperature was mixed with an equal volume of 0.4% (w/v) trypan blue exclusion dye solution (Life technologies) and analyzed using the Countess Automated cell counter (Life Technologies). To determine where the Nluc luminescent signal was originating, the 96-well PCR plate was centrifuged to pellet the cells. The supernatant and cell pellet was collected separately and placed into a 96-well tissue culture plate (Corning). Furimazine Live Cell Substrate (Promega) was added to a final concentration of 1X, and luminescence was measured on a BMG Clariostar luminometer equipped with a 450nm BP filter. Samples were also collected from a 96-well PCR plate that had not undergone centrifugation and placed into a tissue culture plate followed by addition of furimazine substrate and luminescence analysis.

The data demonstrates that the cells were not lysed, even at higher temperatures, since the number of cells remained unchanged and membranes remained intact as determined by dye exclusion (Figure 1A-B). Furthermore, the Nluc activity was retained in the cells with the luciferase signal originating from the cell samples and not the supernatant post-heat treatment (Figure 1C).

### Example 3

### Direct-luciferase-detection thermal shift assay with full length NanoLuc

Experiments were conducted during development of embodiments of the present invention to demonstrate direct detection of the stabilization of a target protein by the interaction with a target ligand via temperature dependent alteration of luminescent signal from a luciferase fused to the target protein.

HeLa cells were transfected separately with the various Nluc-fusion DNA at a 1:10 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in a 90uL/well volume, and either treated with 10uL/well of a 10% DMSO solution (Sigma) or 10uL/well of a 0.5mg/ml solution of digitonin (Sigma). The plates were placed into a thermal cycler (MW Research) where the samples were heated individually at different temperatures spanning a gradient of 42-66°C across the plate for 3 minutes followed by cooling at 22°C for 3 minutes. 90uL/well samples were then transferred to a tissue culture plate (Corning), and Furimazine Live Cell Substrate (Promega) was added to a final concentration of 1X. Luminescence was measured on a BMG Clariostar luminometer equipped with a 450nm BP filter. Data was normalized by relating RLU signals to the RLU of the lowest temperature for the respective sample. Data was then fitted to obtain apparent melting temperature where the protein is precipitating (T_{agg} values) using the Boltzmann Sigmoidal equation with Graphpad Prism software.

Protein thermal melt curves can be generated for Nluc-target fusions spanning several different target classes and subcellular localization (nuclear, cytoplasmic, and membrane) in live, intact cells (Figure 2A), and cells that had been treated with the detergent digitonin (Figure 2B) with results indicating each protein displayed distinct melting curves as analyzed by luciferase activity and reported as apparent aggregation temperatures (T_{agg}).

### Example 4

### Direct-luciferase-detection thermal shift assay with full length NanoLuc

Experiments were conducted during development of embodiments of the present invention to demonstrate direct detection of the stabilization of a target protein by the interaction with a target ligand via temperature dependent alteration of luminescent signal from a luciferase fused to the target protein.

To determine if a shift in T_{agg} after exposure to stabilizing ligand can be detected, the thermal melt curve for CDK2, which showed a distinct melt curve in the presence of DMSO (control), was evaluated. When AZD5438 (a drug known to bind to CDK2) was added, clear shifts in the melting curves with increases in apparent T_{agg} were detected compared to DMSO controls and analyzed by luciferase activity indicating specific binding and protein stabilization of the CDK2 by the inhibitor.

HeLa cells were transfected with CDK2-Nluc or Nluc-CDK2 DNA at a 1:10 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in a 90uL/well volume and treated with 10uL of 10% DMSO (Sigma), 10uL/well of a 0.5mg/ml solution of digitonin (Sigma), 10uL of 1mM AZD5438 (Selleckchem) in 10% DMSO, and/or 10uL of 1mM Methotrexate (Sigma) in 10% DMSO. The plates were then incubated for 1-2 hours in a humidified, 37°C/5% CO₂ incubator. The plates were placed into a thermal cycler (MW Research) where the samples were heated individually at different temperatures spanning a gradient of 42-66°C across the plate for 3 minutes followed by cooling at 22°C for 3 minutes. 90uL/well samples were then transferred to a tissue culture plate (Corning), and Furimazine Live Cell Substrate (to a final concentration of 1X; Promega) or NanoGlo lytic reagent (Promega) was added. Luminescence was measured on a BMG Clariostar luminometer equipped with a 450nm BP filter. Data was normalized by relating RLU signals to the RLU signals of the lowest temperature for the respective sample. Data was then fitted to obtain apparent melting temperature where the protein is precipitating (T_{agg} values) using the Boltzmann Sigmoidal equation with Graphpad Prism software.

The thermal shift induced by AZD5438 was insensitive to Nluc positioning on the Nor C- terminal of CDK2 (Figure 3) and was demonstrated in live (Figure 3A-B) and digitonin-treated cells (Figure 3C-D) as well as in cells lysed with the addition of lytic substrate (Figure 4).

### Example 5

### Direct-luciferase-detection thermal shift assay with full length NanoLuc

Experiments were conducted during development of embodiments of the present invention to demonstrate direct detection of the stabilization of a target protein by the interaction with a target ligand via temperature dependent alteration of luminescent signal from a luciferase fused to the target protein.

To demonstrate that the assay can be applied to a larger class of kinase proteins, specific melt curves and thermal shifts across a panel of kinases were obtained after exposure to specific stabilizing compounds. HeLa cells, expressing different kinase Nluc-target fusions with varying functions and subcellular localization, were exposed to compounds known to target each one and then exposed to the temperature treatment.

HeLa cells were transfected separately with the various Nluc-target DNA at a 1:10 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in a 80uL/well volume and treated with 10uL/well of a 0.5mg/ml solution of digitonin (Sigma), 10uL of 1mM AZD5438 (Selleckchem) in 10% DMSO, 10uL of 0.25mM AMG548 (Tocris) in 10% DMSO, 10uL of 1mM Dasatinib (BioVision) in 10% DMSO, or 10uL of 0.5mM Staurosporine (LC Laboratories) in 10% DMSO. The plates were then incubated for 1 hour in a humidified, 37°C/5% CO₂ incubator. The plates were placed into a thermal cycler (MW Research) where the samples were heated individually at different temperatures spanning a gradient of 42-66°C across the plate for 3 minutes followed by cooling at 22°C for 3 minutes. Furimazine Live Cell Substrate (Promega) was added to a final concentration of 1X in 20uL/well volume. 100uL/well was then transferred to a tissue culture plate (Corning), and luminescence was measured on a BMG Clariostar luminometer equipped with a 450nm BP filter. Data was normalized by relating RLU signals to the RLU signals of the lowest temperature for the respective sample. Data was then fitted to obtain apparent melting temperature where the protein is precipitating (T_{agg} values) using the Boltzmann Sigmoidal equation with Graphpad Prism software.

Obvious shifts in melting temperature, with increases in T_{agg} ≥ 4°C in all cases, were seen when the cells were treated with the specific binding compounds as compared to DMSO controls for each kinase tested (Figure 5).

### Example 6

### Direct-luciferase-detection thermal shift assay with full length NanoLuc

Experiments were conducted during development of embodiments of the present invention to demonstrate direct detection of the stabilization of a target protein by the interaction with a target ligand via temperature dependent alteration of luminescent signal from a luciferase fused to the target protein.

To demonstrate the assay's ability to determine compound target selectivity and highlight the assay's potential screening capabilities for proposed inhibitors, an assay against two kinase target fusions, CDK2-Nluc and Nluc-ABL1, with the same panel of kinase inhibitors known to have different selectivity against these two kinase targets was performed. The assay accurately validated ligand selectivity, and rank order potency, with the compounds known to bind to CDK2 and/or ABL1 causing a shift in melting temperature (apparent T_{agg}), and those compounds known not to have affinity towards CDK2 and/or ABL1 leaving the melting temperature relatively unchanged, as compared to the DMSO control.

HeLa cells were transfected separately with CDK2-Nluc or Nluc-ABL1 DNA at a 1: 100 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in a 90uL/well volume and treated with 10uL of 10% DMSO (Sigma) or 10uL of a 1mM stock solution of test compound in 10% DMSO. Test compounds included: Staurosporine (LC Laboratories), Dasatinib (BioVision), Nilotinib (BioVision), Ponatinib (SYNKinase), AMG548 (Tocris), SB203580 (AdipoGen), and AZD5438 (Selleckchem). The plates were then incubated for 1/2 hour in a humidified, 37°C/5% CO₂ incubator prior to addition of 10uL/well of a 0.5mg/ml solution of digitonin (Sigma) and 10% DMSO (Sigma). The plates were placed into a thermal cycler (MW Research) where the samples were heated individually at different temperatures spanning a gradient of 42-66°C across the plate for 3 minutes followed by cooling at 22°C for 3 minutes. Furimazine Live Cell Substrate (Promega) was added to a final concentration of 1X in 20uL/well volume. 100uL/well was then transferred to a tissue culture plate (Corning), and luminescence was measured on a BMG Clariostar luminometer equipped with a 450nm BP filter. Data was normalized by relating RLU signals to the RLU of the lowest temperature for the respective sample. Data was then fitted to obtain apparent melting temperature where the protein was precipitating (T_{agg} values) using the Boltzmann Sigmoidal equation with Graphpad Prism software.

For the CDK2 target fusion, the known target inhibitors, staurosporine, Ponatinib, and AZD5438, all caused a significant increase in apparent T_{agg}. Dasatinib, Nilotinib, AMG548, and SB20358, which are all compounds not known to interact with CDK2, did not result in a significant shift in apparent T_{agg} compared to DMSO controls when incubated with cells expressing the CDK2-Nluc fusion (Figure 6A-B). Similarly, Staurosporine, Dasatinib, Nilotinib, and Ponatinib, all of which target ABL1 kinase, caused a significant shift in apparent T_{agg} compared to DMSO controls, whereas the negative ligands AMG548, SB203580, and AZD5438 did not as expected (Figure 6C-D).

### Example 7

### Direct-luciferase-detection thermal shift assay with full length NanoLuc

Experiments were conducted during development of embodiments of the present invention to demonstrate direct detection of the stabilization of a target protein by the interaction with a target ligand via temperature dependent alteration of luminescent signal from a luciferase fused to the target protein.

To demonstrate the TSA approach described herein can be applied broadly, the experiment performed in Figure 6 was repeated using a target protein that maintains a nuclear subcellular localization and originated from a drug target class very different from kinases. Cells were transfected with HDAC1-Nluc fusions and tested against a panel of known HDAC inhibitors with Staurosporine, a kinase inhibitor, serving as an additional negative control with DMSO.

HeLa cells were transfected with HDAC1-Nluc DNA at a 1:100 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in a 90uL/well volume and treated with 10uL of 10% DMSO (Sigma) or 10uL of a 1mM stock solution of test compound in 10% DMSO. Test compounds included: SAHA (TOCRIS), Mocetinostat (Selleckchem), Panibinostat (LC Laboratories), ACY1215 (Selleckchem), and Staurosporine (LC Laboratories). The plates were then incubated for 1/2 hour in a humidified, 37°C/5% CO₂ incubator prior to addition of 10uL/well of a 0.5mg/ml solution of digitonin (Sigma) and Furimazine Live Cell Substrate (final concentration 1X (Promega)). The plates were placed into a thermal cycler (MW Research) where the samples were heated individually at different temperatures spanning a gradient of 42-66°C across the plate for 3 minutes followed by cooling at 22°C for 3 minutes. 100uL/well was then transferred to a tissue culture plate (Corning), and luminescence was measured on a BMG Clariostar luminometer equipped with a 450nm BP filter. Data was normalized by relating RLU signals to the RLU of the lowest temperature for the respective sample. Data was then fitted to obtain apparent melting temperature where the protein is precipitating (T_{agg} values) using the Boltzmann Sigmoidal equation with Graphpad Prism software.

All four HDAC known inhibitors (SAHA, Mocetinostat, Panibinostat, and ACY1215) produced a significant shift in melting temperature (apparent T_{agg}) indicating ligand binding compared to the controls as analyzed by luciferase activity (Figure 7).

### Example 8

### Direct-luciferase-detection thermal shift assay with full length NanoLuc

Experiments were conducted during development of embodiments of the present invention to demonstrate direct detection of the stabilization of a target protein by the interaction with a target ligand via temperature dependent alteration of luminescent signal from a luciferase fused to the target protein.

To determine if the assay can be performed when the ligands added are not maintained under equilibrium conditions, the assay was performed with a compound washout step. This would also confirm that the target engagement was happening in live cells as well as have the ability to analyze binding kinetics.

HeLa cells were transfected with KDR-Nluc or DHFR-Nluc at a 1:10 DNA ratio and incubated overnight in t75 tissue culture flasks (Corning). Media was aspirated and replaced with media containing a final concentration of 100uM BIBF-1120 (Selleckchem) for cells expressing KDR-Nluc or 100uM Methotrexate (Sigma) for cells expressing DHFR-Nluc. DMSO at a final concentration of 1% was used as control and added to each cell population. The cells were allowed to incubate with compounds for 2 hours in a humidified, 37°C/5% CO₂ incubator. The cells were trypsinized using Trypsin-EDTA (0.25%) (Gibco) and re-suspended in Opti-MEM without phenol red (Gibco) + 1x Protease Inhibitor Cocktail (Promega) which had been reconstituted in DMSO (Sigma). Cells were seeded into 96-well tall-chimney PCR plate (Fisherbrand) at a density of 2 × 10⁴ cells/well in a 100uL/well and placed into a thermal cycler (MW Research) where the samples were heated individually at different temperatures spanning a gradient of 42-66°C across the plate for 3 minutes followed by cooling at 22°C for 3 minutes. 90uL/well was then transferred to a tissue culture plate (Corning), and Furimazine Live Cell Substrate (final concentration 1X (Promega)) was added. Luminescence was measured on a BMG Clariostar luminometer equipped with a 450nm BP filter. Data was normalized by relating RLU signals to the RLU of the lowest temperature for the respective sample. Data was then fitted to obtain apparent melting temperature where the protein is precipitating (T_{agg} values) using the Boltzmann Sigmoidal equation with Graphpad Prism software.

Figure 8 shows detection of an increase in melting temperature for the targets KDR-Nluc (Fig. 8A) and DHFR-Nluc (Fig. 8B) as determined by NanoLuc^{®} activity (RLU) after incubation with known stabilizing ligand in mammalian cells subsequently harvested with the compound being washed out, and then subjected to a temperature. KDR is a membrane protein highlighting another subcellular location that the assay is able to monitor.

### Example 9

### Direct-luciferase-detection thermal shift assay with NLPep and NLPoly through spontaneous binary complementation

Experiments were conducted during development of embodiments of the present invention to demonstrate direct detection of the stabilization of a target protein by the interaction with a target ligand via temperature dependent alteration of luminescent signal in which the luminescent signal results through spontaneous binary complementation from a peptide fused to the target protein with the complementary subunit present in the detection reagent.

To determine if the luciferase-based thermal shift assay described herein could be performed using a spontaneous binary complementation, the NLPep and NLPoly technology (Promega; See, e.g.., U.S. Pub. No. 2014/0348747) was used.

HeLa cells were transfected with CDK2-NLpep86 or NLpep86-CDK2 DNA at a 1:10 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega), which had been reconstituted in DMSO (Sigma). The cells were seeded into 96-well tall-chimney PCR plate (Fisherbrand) at a density of 2 × 10⁴ cells/well in a 90uL/well volume and treated with 1uL of 100% DMSO (Sigma) or 1uL of a 10mM stock solution of AZD5438 (Selleckchem) in 10% DMSO, and 10uL/well of a 0.5mg/ml solution of digitonin (Sigma). The plates were then incubated for 1-2 hour in a humidified, 37°C/5% CO₂ incubator. The plates were placed into a thermal cycler (MW Research) where the samples were heated individually at different temperatures spanning a gradient of 42-66°C across the plate for 3 minutes followed by cooling at 22°C for 3 minutes. 90uL/well was then transferred to a tissue culture plate (Corning), and the complementary subunit, NLpoly11S, was added to a final concentration of 1uM with Furimazine Live Cell Substrate (final concentration 1X (Promega)). Luminescence was measured on a BMG Clariostar luminometer equipped with a 450nm BP filter. Data was normalized by relating RLU signals to the RLU of the lowest temperature for the respective sample and subtracting the background luminescence signal generated by NLpoly11S. Data was then fitted to obtain apparent melting temperature where the protein is precipitating (T_{agg} values) using the Boltzmann Sigmoidal equation with Graphpad Prism software.

Figure 9 shows detection of an increase in melting temperature for CDK2-NLpep86 and NLpep86-CDK2 as determined by luciferase activity through spontaneous binary complementation after incubation in the presence of the stabilizing ligand AZD5438 in digitonin- treated mammalian cells subsequently exposed to a temperature gradient and addition of substrate and complementary subunit NLpoly11S as compared to the DMSO control.

### Example 10

### Direct-luciferase-detection thermal shift assay with NLPep and NLPoly through spontaneous binary complementation

Experiments were conducted during development of embodiments of the present invention to demonstrate direct detection of the stabilization of a target protein by the interaction with a target ligand via temperature dependent alteration of luminescent signal in which the luminescent signal results through spontaneous binary complementation from a peptide fused to the target protein with the complementary subunit present in the detection reagent.

The following experiment is similar to Example 9, but used another pair of complementary subunits, NLpoly156 (SEQ ID NO: 7) and NLpep86. NLpoly156 was fused to the target protein, and NLpep86 was placed in the detection reagent.

HeLa cells were transfected with CDK2-Nluc156 DNA at a 1:10 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in a 90uL/well volume and treated with 1uL of 100% DMSO (Sigma) or 1uL of a 10mM stock solution of AZD5438 (Selleckchem) in 10% DMSO, and 10uL/well of a 0.5mg/ml solution of digitonin (Sigma). The plates were then incubated for 1-2 hour in a humidified, 37°C/5% CO₂ incubator. The plates were placed into a thermal cycler (MW Research) where the samples were heated individually at different temperatures spanning a gradient of 42-66°C across the plate for 3 minutes followed by cooling at 22°C for 3 minutes. 90uL/well was then transferred to a tissue culture plate (Corning), and the complementary subunit, NLpep86, was added to a final concentration of 1uM with Furimazine Live Cell Substrate (final concentration 1X (Promega)). Luminescence was measured on a BMG Clariostar luminometer equipped with a 450nm BP filter. Data was normalized by relating RLU signals to the RLU of the lowest temperature for the respective. Data was then fitted to obtain apparent melting temperature where the protein is precipitating (T_{agg} values) using the Boltzmann Sigmoidal equation with Graphpad Prism software.

Figure 10 demonstrates the detection of an increase in melting temperature for CDK2-NLpoly156 as determined by luciferase activity through spontaneous binary complementation after incubation in the presence of the stabilizing ligand AZD5438 in digitonin-treated mammalian cells subsequently exposed to a temperature gradient and addition of substrate and complementary subunit NLpep86 as compared to the DMSO control.

### Example 11

### Direct-luciferase-detection thermal shift assay with NLPep and NLPoly through spontaneous binary complementation

Experiments were conducted during development of embodiments of the present invention to demonstrate direct detection of the stabilization of a target protein by the interaction with a target ligand via temperature dependent alteration of luminescent signal in which the luminescent signal results through spontaneous binary complementation from a peptide fused to the target protein with the complementary subunit present in the detection reagent.

HeLa cells were transfected with CDK2-NLpep86 or CDK2-NLpoly11S DNA at a 1:10 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in a 90uL/well volume and treated with 1uL of a 100x stock solution in a dilution series of AZD5438 (Selleckchem) in 10% DMSO, and 10uL/well of a 0.5mg/ml solution of digitonin (Sigma). The plates were then incubated for 1-2 hour in a humidified, 37°C/5% CO₂ incubator. The plates were placed into a thermal cycler (MW Research) where the samples were heated individually at different temperatures spanning a gradient of 42-66°C across the plate for 3 minutes followed by cooling at 22°C for 3 minutes. 90uL/well was then transferred to a tissue culture plate (Corning) and complementary subunit NLpoly11S or NLpep86 was added to a final concentration of 1uM with Furimazine Live Cell Substrate (final concentration 1X (Promega)). Luminescence was measured on a BMG Clariostar luminometer equipped with a 450nm BP filter. For EC₅₀ analysis, compound concentration was plotted against RLU, and the data was transformed prior to being fitted using the sigmoidal dose response (variable slope) equation with Graphpad Prism software. For apparent T_{agg} analysis, data was normalized by relating RLU signals to the RLU of the lowest temperature for the respective. Data was then fitted to obtain apparent melting temperature where the protein is precipitating (T_{agg} values) using the Boltzmann Sigmoidal equation with Graphpad Prism software.

Figures 11 and 12 demonstrates the temperature and stabilizing ligand concentration dependency of the luciferase-based thermal shift assay which is consistent with other reported methods of thermal shift assay. Here, the EC₅₀ of the ligand AZD5438 shifts as a function of temperature and the apparent T_{agg} shifts as a function of stabilizing ligand concentration. In this case, the targets consisted of CDK2-NLpep86 fusions (Fig. 11) and CDK2-NLpoly11S fusions (Fig. 12).

### Example 12

### BRET-detection thermal shift assay

Experiments were conducted during development of embodiments of the present invention to demonstrate detection of the stabilization of a target protein by the interaction with a target ligand via temperature dependent alteration of BRET signal generated by the energy transfer from a luciferase fused to the target protein to an environmentally sensitive dye that binds to all proteins unfolding in the sample including the target protein.

Protein thermal melting curves can be generated for purified proteins in which the extent of unfolding is measured by the gain in fluorescent signal from environmentally sensitive dyes that bind to the exposed hydrophobic surfaces of the protein as they unfold. Taking advantage of this property and adding these dyes to the luciferase-based thermal shift assay described herein, the environmentally-sensitive dye binds to the target protein-Nluc fusion, and BRET is produced upon addition of substrate when there is active Nluc available to excite the dye. The dye acts as a fluorescent BRET acceptor, and the transfer of energy is moderated by the proximity of the two partners. While the dye will bind to all proteins unfolding in the sample, the luciferase fusion gives the target protein specificity; hence any BRET signal is a direct readout on the state of the protein of interest.

HeLa cells were transfected with CDK2-Nluc or Nluc-LCK DNA at a 1:100 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in an 80uL/well volume and treated with 10uL/well of a 0.5mg/ml solution of digitonin (Sigma) and 10uL of 10% DMSO (Sigma) or 10uL of a 1mM stock solution of test compound in 10% DMSO. Test compounds included: Dasatinib (BioVision), AZD5438 (Selleckchem), Staurosporine (LC Laboratories), and Nilotinib (BioVision). The plates were then incubated for 1 hour in a humidified, 37°C/5% CO₂ incubator. The plates were placed into a thermal cycler (MW Research) where the samples were heated either individually at different temperatures spanning a gradient of 42-66°C across the plate for 3 minutes to obtain apparent T_{agg}, or the samples are all heated for 3 minutes at one constant temperature to obtain ITDRC, followed by cooling at 22°C for 3 minutes. The Protein Thermal Shift^{™} dye (Life Technologies) was added at a final concentration of 0.5x concentration (supplied as 1000x) in 1uL/well volume. Furimazine Live Cell Substrate (Promega) was added to a final concentration of 1X in 20uL/well volume, and a total volume of 100uL/well was then transferred to a tissue culture plate (Corning). To measure BRET, filtered luminescence was measured on a BMG Clariostar luminometer equipped with 450nm BP filter (donor) and 610nm LP filter (acceptor), using 0.5s integration time. MilliBRET units (mBU) were calculated by using the equation: (acceptor/donor)* 1000. To obtain apparent T_{agg} values, data was fitted using the bell shaped curve equation. To analyze ITDFC data, data was transformed and fitted to obtain apparent EC50s using the sigmoidal dose-response (variable slope) equation. All analyses were performed with Graphpad Prism software.

Figure 13 demonstrates that BRET can be used to detect ligand binding through a change in melting temperature curves compared to DMSO controls as exampled with CDK2-Nluc (+/-AZD5438) and LCK-Nluc (+/-Dasatinib) target fusions. As expected, the shape of the BRET curves are bell shaped due to loss of Nluc signal with protein unfolding and increasing temperatures or dye dissociation upon protein aggregation or both.

In Figure 14, to obtain isothermal dose response curves (ITDRC) in order to obtain compound rank order affinity and apparent EC50s, the curves generated when the samples were subjected to the temperature gradient to ensure compliance with the model were inspected first, apparent T_{agg} calculated, and an appropriate temperature chosen for follow-up ITDRC or screening experiments. This was a temperature at which a majority of the protein is unfolded or precipitated in the absence of stabilizing compound, but at which a majority of the protein remains soluble in the presence of a saturating concentration of known stabilizing compound. To demonstrate this, ITDRC for a panel of kinase inhibitors with known different selectivity was established and tested for target engagement with CDK2-Nluc and LCK-Nluc fusions as determined by BRET after incubation in the presence of different concentrations of the individual compounds while time of heating and temperature were kept constant. This demonstrates the concentration dependence of the thermal stabilization and allows for compound affinity signatures to be obtained.

### Example 13

### BRET-detection thermal shift assay

Experiments were conducted during development of embodiments of the present invention to demonstrate detection of the stabilization of a target protein by the interaction with a target ligand via temperature dependent alteration of BRET signal generated by the energy transfer from a luciferase fused to the target protein to an environmentally sensitive dye that binds to all proteins unfolding in the sample including the target protein.

To demonstrate analysis of compound selectivity using the BRET method of thermal shift, a panel of compounds with known selectivity against CDK2, MAPK14, and HDAC1 was tested. It was found that the assay accurately detected the positive and negative ligands against these targets.

HeLa cells were transfected with CDK2-Nluc, Nluc-MAPK14, or HDAC1-Nluc DNA at a 1: 100 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in an 90uL/well volume and treated with 10uL of 10% DMSO (Sigma) or 10uL of a 1mM stock solution of test compound in 10% DMSO. Test compounds included: Dasatinib (BioVision), AZD5438 (Selleckchem), Staurosporine (LC Laboratories), Nilotinib (BioVision), Ponatinib (SYNKinase), AMG548 (TOCRIS), SB203580(Adipogen), SAHA (TOCRIS), Mocetinostat (Selleckchem), Panibinostat (LC Laboratories), and ACY1215 (Selleckchem). The plates were then incubated for 1/2 hour in a humidified, 37°C/5% CO₂ incubator. Immediately prior to heating, 10uL/well of a 0.5mg/ml solution of digitonin (Sigma), Protein Thermal Shift^{™} dye (Life Technologies) was added at a final concentration of 0.5x concentration (supplied as 1000x) in 1uL/well volume, and Furimazine Live Cell Substrate (Promega) was added to a final concentration of 1X in 20uL/well volume. Plates were placed into a thermal cycler (MW Research) where the samples were heated individually at different temperatures spanning a gradient of 42-66°C across the plate for 3 minutes followed by cooling at 22°C for 3 minutes, and a total volume of 100uL/well was then transferred to a tissue culture plate (Corning). To measure BRET, filtered luminescence was measured on a BMG Clariostar luminometer equipped with 450nm BP filter (donor) and 610nm LP filter (acceptor), using 0.5s integration time. MilliBRET units (mBU) were calculated by using the equation: (acceptor/donor)*1000. Data was analyzed up to 62°C, and the data fitted to obtain apparent melting temperature where the protein is precipitating (T_{agg} values) using the Boltzmann Sigmoidal equation with Graphpad Prism software.

Figure 15 shows detection of an increase in melting temperatures (stabilizing ligand) or no change in melting temperatures (non-binding) for cytoplasmic target fusions CDK2-Nluc and Nluc-MAPK14 as determined by BRET after incubation with a panel of compounds, thus displaying compound selectivity in mammalian cells subsequently exposed to digitonin and a temperature gradient. Figure 16 shows detection of an increase in melting temperatures (stabilizing ligand) or no change in melting temperatures (non-binding) for the nuclear target fusion HDAC1-Nluc as determined by BRET after incubation with a panel of compounds in mammalian cells subsequently exposed to digitonin and a temperature gradient.

### Example 14

### BRET-detection thermal shift assay

Experiments were conducted during development of embodiments of the present invention to demonstrate detection of the stabilization of a target protein by the interaction with a target ligand via temperature dependent alteration of BRET signal generated by the energy transfer from a luciferase fused to the target protein to an environmentally sensitive dye that binds to all proteins unfolding in the sample including the target protein.

To demonstrate that many dyes are suitable for the luciferase-based thermal shift assay analyzed by BRET described herein, several different dyes were tested. Dye examples included: Protein Thermal Shift^{™} Dye, SYPRO Orange protein gel stain, and SYPRO Red protein gel stain.

HeLa cells were transfected with CDK2-Nluc at a 1: 100 DNA ratio and incubated overnight. The cells were trypsinized using Trypsin-EDTA(0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in an 90uL/well volume and treated with 10uL of 10% DMSO (Sigma) or 10uL of a 1mM stock solution of Staurosporine (LC Laboratories) in 10% DMSO. The plates were then incubated for 1/2 hour in a humidified, 37°C/5% CO₂ incubator. Immediately prior to heating, 10uL/well of a 0.5mg/ml solution of digitonin (Sigma), Furimazine Live Cell Substrate (Promega) to a final concentration of 1X in 20uL/well volume, and 1ul of each dye dilution was added. Dyes included the SYPRO Orange, SYPRO Red, and Protein Thermal Shift ^{™} Dye (all from Life Tech). Plates were placed into a thermal cycler (MW Research) where the samples were heated individually at different temperatures spanning a gradient of 42-66°C across the plate for 3 minutes followed by cooling at 22°C for 3 minutes, and a total volume of 100uL/well was then transferred to a tissue culture plate (Corning). To measure BRET, filtered luminescence was measured on a BMG Clariostar luminometer equipped with 450nm BP filter (donor) and 610nm LP filter (acceptor), using 0.5s integration time. MilliBRET units (mBU) were calculated by using the equation: (acceptor/donor)* 1000. Data was then fitted to obtain apparent melting temperature where the protein is precipitating (T_{agg} values) using the Boltzmann Sigmoidal equation with Graphpad Prism software.

Figure 17 shows detection of an increase in melting temperature for CDK2-Nluc as determined by BRET after incubation with stabilizing ligand (staurosporine) in mammalian cells subsequently exposed to digitonin and a temperature gradient using three different environmentally sensitive acceptor dyes reporting on protein folding status as BRET acceptor dyes. Bₘₐₓ is acceptor dye dose dependent, but that there is no change in the apparent melting temperature (T_{agg}).

### Example 15

### BRET-detection thermal shift assay

Experiments were conducted during development of embodiments of the present invention to demonstrate detection of the stabilization of a target protein by the interaction with a target ligand via temperature dependent alteration of BRET signal generated by the energy transfer from a luciferase fused to the target protein to an environmentally sensitive dye that binds to all proteins unfolding in the sample including the target protein.

To demonstrate that the acceptor dye can be added either before or after the heating step in the luciferase-based thermal shift assay using BRET analysis, two different environmentally sensitive dyes were used to determine folded protein status and serve as BRET acceptor dyes. The dyes included in the Protein Thermal Shift^{™} Dye (Life Technologies) and the Proteostat^{™} dye (Enzo), which have differing properties in regards to protein status binding. The Proteostat^{™} dye binds to protein aggregates rather than unfolding proteins, whereas the Protein Thermal Shift^{™} dye binds to proteins as they unfold, but dissociates as the proteins aggregate. (Figure 18).

HeLa cells were transfected with CDK2-Nluc DNA at a 1:10 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in a 90uL/well volume and treated with 10uL/well of a 0.5mg/ml solution of digitonin (Sigma) in 10% DMSO (Sigma) and 1uL of 100% DMSO (Sigma) or 1uL of a 10mM stock solution of Staurosporine (LC Laboratories) in 100% DMSO. The plates were then incubated for 2 hour in a humidified, 37°C/5% CO₂ incubator. The plates were placed into a thermal cycler (MW Research) where the samples were heated either individually at different temperatures spanning a gradient of 42-66°C across the plate for 3 minutes followed by cooling at 22°C for 3 minutes. The Protein Thermal Shift^{™} Dye (Life Technologies) and the Proteostat^{™} dye (Enzo) were added at a final concentration of 1x (supplied as 1000x) in 1uL/well volume either before or after the heating step. 90ul/well was transferred to a tissue culture plate (Corning), and Furimazine Live Cell Substrate (Promega) was added to a final concentration of 1X in 20uL/well volume. To measure BRET, filtered luminescence was measured on a BMG Clariostar luminometer equipped with 450nm BP filter (donor) and 610nm LP filter (acceptor), using 0.5s integration time. MilliBRET units (mBU) were calculated by using the equation: (acceptor/donor)*1000. Data was fitted using the bell shaped curve equation with Graphpad Prism software.(Figure 18)

### Example 16

### BRET-detection thermal shift assay

Experiments were conducted during development of embodiments of the present invention to demonstrate detection of the stabilization of a target protein by the interaction with a target ligand via temperature dependent alteration of BRET signal generated by the energy transfer from a luciferase fused to the target protein to an environmentally sensitive dye that binds to all proteins unfolding in the sample including the target protein.

HeLa cells were transfected with CDK2-Nluc or HDAC1-Nluc at a 1:100 DNA ratio and incubated overnight. The cells were trypsinized using Trypsin-EDTA(0.25%) (Gibco) and re-suspended in Opti-MEM without phenol red (Gibco) + 1x Protease Inhibitor Cocktail (Promega) which had been reconstituted in DMSO (Sigma). The cells were seeded into 96-well tall-chimney PCR plate (Fisherbrand) at a density of 2 × 10⁴ cells/well in an 80uL/well volume and treated with 10uL of 10% DMSO or 10uL of a 1mM stock solution of AZD5438 (for CDK2-Nluc) or Mocetinostat (for HDAC1-Nluc) in 10% DMSO. The plates were then incubated for 1/2 hour in a humidified, 37°C/5% CO₂ incubator. Immediately prior to heating, 10uL/well of a 0.5mg/ml solution of digitonin (Sigma), Furimazine Live Cell Substrate (Promega) to a final concentration of 1X in 20uL/well volume, and 1ul of the Protein Thermal Shift^{™} Dye (Life Technologies) for a final concentration of 0.5x was added. Plates were placed into a thermal cycler (MW Research) where the samples were heated individually at different temperatures spanning a gradient of 42-66°C across the plate for 3 minutes. Some plates were also then further cooled at 22°C for 3 minutes. A total volume of 100uL/well was then transferred to a tissue culture plate (Corning). To measure BRET, filtered luminescence was measured on a BMG Clariostar luminometer equipped with 450nm BP filter (donor) and 610nm LP filter (acceptor), using 0.5s integration time. MilliBRET units (mBU) were calculated by using the equation: (acceptor/donor)* 1000. Data was then fitted to obtain apparent melting temperature where the protein is precipitating (T_{agg} values) using the Boltzmann Sigmoidal equation with Graphpad Prism software.

Figure 19 and 20 demonstrates data obtained when the assay was run with and without the cooling step that follows the 3 minutes heating gradient step using two different targets, CDK2-Nluc (Fig 19) and HDAC1-Nluc (Fig. 20). A clear shift in apparent T_{agg} was found whether a cooling step was included in the protocol or not as analyzed by both, direct luciferase readout and BRET.

### Example 17

### Other proposed proximity based reporter methods for detection of ligand binding in a thermal shift assay

Based on the successful use of a direct luciferase fusion or BRET to detect ligand-mediated thermal stabilization, other proximity-based reporter chemistries are understood to be useful. Figure 21 gives schematic examples of other proximity based reporter methods that may be suitable for analysis of target engagement using thermal shift. For example, an epitope tag is attached to the protein of interest and following a thermal denaturation step, addition of a detection antibody labeled with a donor fluorophore (e.g., terbium, europium, etc.) is used in a FRET assay with an denaturation/aggregation-sensitive dye as a FRET acceptor. Ligand-mediated thermal stabilization results in a loss of FRET/TR-FRET signal. Another potential iteration is using a combination of labeled antibodies (e.g. donor-labeled anti-FLAG and acceptor labeled anti-V5) and a tandem epitope (e.g. FLAG-V5) tethered to the target protein are used as a detection system. When the target is stabilized by binding of a ligand, the epitope is presented and both antibodies bind, generating a proximity-based signal (e.g. FRET, TR-FRET). Upon thermal denaturation, the epitopes are unavailable to the antibody pair, resulting in a loss of proximity-based signal. Various detection chemistries can be applied for these prophetic examples (e.g. TR-FRET, proximity ligation, singlet oxygen transfer/alphascreen, etc.).

### Example 18

### Chemical Synthesis Description

A series of styryl dye derivatives were synthesized and screened in BRET detection thermal shift assay. The dye derivatives **CS000, CS0004, CS0007, CS0008, CS0013, CS0010, CS0017, CS0018, CS0020, CS0024, CS0028, CS0038, CS0067, CS0068, CS0075, CS0081, CS0085, CS0086, CS0087, CS0096, and CS0155** and are generally synthesized analogously to **CS0101** via a condensation reaction between various aryl aldehydes and pyridinium betaines or *N*-alkyl pyridinium, which has been described in literature (1^{st} reaction in **Scheme 1**) (Hassner, A.; Birnbaum, D.; Loew, L.M. J. Org. Chem. 1984, 49, 2546-2551.). As depicted in **Scheme 1, CS0101** was hydrolyzed under acidic condition to afford **CS0036.** The subsequent amide coupling and the acidic deprotection afforded **CS0100** and **CS0045** respectively. **CS0048** was obtained via an amide coupling between **CS0045** and 3-amino-1-propanesulfonic acid. **CS0043** and **CS0147** was synthesized analogously to **CS0045. CS0073** was obtained via a *N*-alkylation reaction between **CS0043** and neat 1,3 propane sultone (**Scheme 2**). **CS0117** was synthesized via a Heck reaction between the *para-*bromo aniline **5** and *para*-vinyl pyridine **6** (**Scheme 3**).¹ *N*-alkylation of **CS0117** afforded **CS0121** (**Scheme 3**).¹ **CS0112, CS0158, CS0071** and **CS0072** were synthesized analogously to **CS0121.** 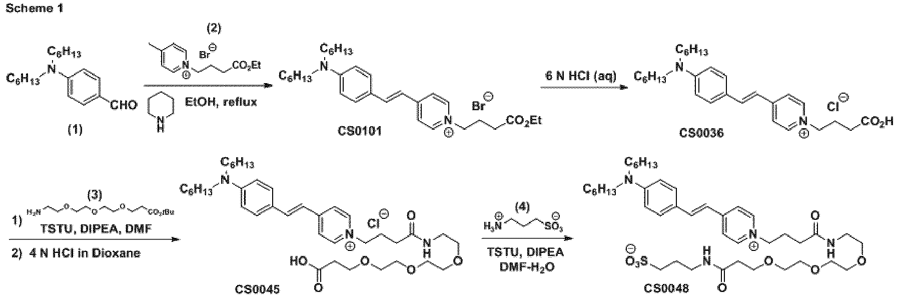 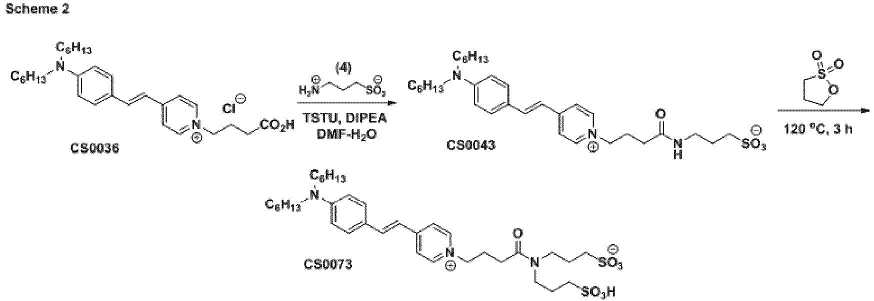 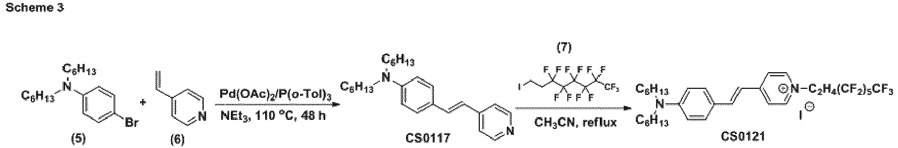
***(E)-4-(4-(Dihexylamino)styryl)-1-(4-ethoxy-4-oxobutyl)pyridin-1-ium bromide (CS0101)***: To a solution of 4-(dihexylamino)benzaldehyde (**1**, 144 mg, 0.5 mmol, 1.0 equiv) in EtOH (4 mL) at RT, piperidine (4.3 mg, 0.05 mmol. 0.1 equiv) and 1-(4-ethoxy-4-oxobutyl)-4-methylpyridin-1-ium bromide (**2**, 144 mg, 0.5 mmol, 1.0 equiv) was added in one portion. The mixture was stirred at 90°C for 15 h and cooled down at RT for 30 min. The desired product precipitated from the solution and was purified by silica gel chromatography (CH₂Cl₂/MeOH, 1/9). LRMS: observed for [M - Br] ⁺, 479.7.
***(E)-1-(3-Carboxypropyl)-4-(4-(dihexylamino)styryl)pyridin-1-ium chloride (CS0036)***: To a solution of **CS0101** (324 mg, 0.58 mmol, 1.0 equiv) in 1,4-dioxane (4 mL) at RT, 6 N HCl (aq, 4 mL, 24 mmol, 41 equiv) was added. The mixture was stirred at 100°C for 15 h and concentrated in vacuo to afford the crude. The desired product was purified by silica gel chromatography (CH₂Cl₂/MeOH, 1/9). LRMS: observed for [M - Cl] ⁺, 451.7.
***(E)-4-(4-(Dihexylamino)styryl)-1-(2,2-dimethyl-4,17-dioxo0-3,7,10,13-tetraoxa-16-azaicosan-20-yl)pyridin-1-ium chloride (CS0100):*** To a solution of **CS0036** (70 mg, 0.13 mmol, 1.0 equiv) in DMF (1.5 mL) at RT, TSTU (40 mg, 0.13 mmol, 1.0 equiv) and NEt₃ (60 µL, 0.43 mmol, 2.5 equiv) was added. The solution was stirred at RT for 1 h. To the reaction mixture, *tert*-butyl 3-(2-(2-(2-aminoethoxy)ethoxy)ethoxy)propanoate (**3**, 43.8 mg, 0.16 mmol, 1.2 equiv) in DMF (1.5 mL) was added. The reaction was stirred at RT for 15 h. LC-MS analysis indicated full conversion of the starting material. The solvent was removed in vacuo to afford the crude and the desired product was purified by silica gel chromatography (CH₂Cl₂/MeOH, 1/9). LRMS: observed for [M - Cl] ⁺, 711.1.
***(E)-1-(1-Carboxy-13-oxo-3,6,9-trioxa-12-azahexadecan-16-yl)-4-(4-(dihexylamino)styryl)pyridin-1-ium chloride (CS0045):*** The starting material **CS0100** (40 mg, 0.5 mmol, 1.0 equiv) was dissolved in 4N HCl in dioxane (2 mL, 8 mmol, 16 equiv). The mixture was stirred at 100°C for 15 h and concentrated in vacuo to afford the crude. The desired product was purified by silica gel chromatography (CH₂Cl₂/MeOH, 1/9). LRMS: observed for [M - Cl] ⁺, 654.9.
***(E)-21-(4-(4-(Dihexylamino)styryl)pyridin-1-ium-1-yl)-5,18-dioxo-8,11,14-trioxa-4,17-diazahenicos-ane-1-sulfonate (CS0048):*** To a solution of **CS0045** (60 mg, 0.09 mmol, 1.0 equiv) in DMF (1.5 mL) at RT, TSTU (33 mg, 0.11 mmol, 1.2 equiv) and NEt₃ (51 µL, 0.37 mmol, 4 equiv) was added. The solution was stirred at RT for 1 h. To the reaction mixture, 3-amino-1-propanesulfonic acid (**4**, 19.1 mg, 0.14 mmol, 1.5 equiv) in H₂O (1 mL) was added. The reaction was stirred at RT for 15 h. LC-MS analysis indicated full conversion of the starting material. The solvent was removed in vacuo to afford the crude and the desired product was purified by silica gel chromatography (CH₂Cl₂/MeOH, 1/9). LRMS: observed for [M + H] ⁺, 776.1.
***(E)-3-(4-(4-(4-(Dihexylamino)styryl)pyridin-1-ium-1-yl)-N-(3-sulfopropyl)butanamido)propane-1-sulfonate (CS0073):* CS0043** was mixed with 1,3 propane sultone (1 mL) and heated at 120⁰C for 3 h. The mixture was cooled down to RT and resuspended in hot MeOH (20 mL), filtered over Celite. Concentration in vacuo afforded the crude, which was purified by silica gel chromatography (CH₂Cl₂/MeOH, 1/9). LRMS: observed for [M + H] ⁺, 695.1.
***(E)-N,N-Dihexyl-4-(2-(pyridin-4-yl)vinyl)aniline (CS0117):*** To a flask charged with Pd(OAc)₂ (11.2 mg, 50 µL, 0.05 equiv) and tri-(o-tolyl) phosphine (45.6 mg, 0.14 mmol, 0.15 equiv), NEt₃ (5 mL) was added. The mixture was stirred under N₂ for 20 min. To the mixture, para-bromo aniline (**5**, 340 mg, 1mmol, 1.0 equiv) and *para*-vinyl pyridine (**6**, 115.7 mg, 1.1 mmol, 1.1 equiv) was added. The mixture was heated up to 120⁰C and stirred for 48 h under N₂. The reaction was cooled down and concentrated to afford the crude. The desired product was purified by silica gel chromatography (Heptane/EtOAc, 6/4). LRMS: observed for [M + H] ⁺, 365.6.
***(E)-4-(4-(dihexylamino)styryl)-1-(3,3,4,4,5,5,6,6,7,7,8,8,8-tridecafluorooctyl)pyridin-1-iumiodide (CS0121):*** To a solution of **CS0117** (70 mg, 0.19 mmol, 1.0 equiv)in CH₃CN (3 mL), 1,1,1,2,2,3,3,4,4,5,5,6,6-tridecafluoro-8-iodooctane (**7**, 108 mg, 0.23 mmol, 1.2 equiv) was added. The reaction was heated to reflux for 15 h. LC-MS indicated full conversion to the desired product. The mixture was concentrated in vacuo and purified by silica gel chromatography (CH₂Cl₂/MeOH, 1/9). LRMS: observed for [M - I] ⁺, 711.8.

### Example 19

### BRET-detection thermal shift assay

Experiments were conducted during development of embodiments of the present invention to demonstrate detection of the stabilization of a target protein by the interaction with a target ligand via temperature dependent alteration of BRET signal generated by the energy transfer from a luciferase fused to the target protein to an environmentally sensitive dye that binds to all proteins unfolding in the sample including the target protein.

To demonstrate analysis of compound selectivity using the BRET method of thermal shift, a panel of compounds with known selectivity against CDK2 and MAPKI4 was tested. It was found that the assay accurately detected the positive and negative ligands against these targets.

HeLa cells were transfected with CDK2-Nluc or Nluc-MAPKI4 DNA at a 1:10 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega), which had been reconstituted in DMSO (Sigma). The cells were seeded into wells of a 96-well, tall-chimney PCR plate (Fisherbrand) at a density of 2 × 10⁴ cells/well in an 90uL/well volume and treated with 10uL of 10% DMSO (Sigma) or 10uL of a 1mM stock solution of the test compound Staurosporine (LC Laboratories) in 10% DMSO. The plates were then incubated for 1/2 hour in a humidified, 37°C/5% CO₂ incubator. Immediately prior to heating, 10uL/well of a 0.5mg/ml solution of digitonin (Sigma) and dye derivative was added at a final concentration of 10uM or in a dose response as indicated in B and C of each figure in 1 uL/well volume. Plates were placed into a thermal cycler (MW Research) where the samples were heated individually at different temperatures spanning a gradient of 42-66°C across the plate for 3 minutes followed by cooling at 22°C for 3 minutes, and a total volume of 100uL/well was then transferred to a tissue culture plate (Coming). Furimazine Live Cell Substrate (Promega) was added to a final concentration of 1X in 20uL/well volume. To measure BRET, filtered luminescence was measured on a BMG Clariostar luminometer equipped with 450nm BP filter (donor) and 610nm LP filter (acceptor), using 0.5s integration 5 time. MilliBRET units (mBU) were calculated by using the equation: (acceptor/donor) * 1000.

Data was analyzed up to 62°C, and the data fitted to obtain apparent melting temperature where the protein is precipitating (Tagg values) using the Boltzmann Sigmoidal equation with Graphpad Prism software.

Figures 23-34 demonstrate: (A) that BRET using dyes synthesized in Example 18 detects ligand binding through a change in relative BRET compared to DMSO controls; (B) the fold change in BRET signal at 56°C over the background BRET signal at 42°C for cells transfected with CDK2-Nluc target that were treated with DMSO and varying dye concentrations; and (C) the fold change in BRET signal at 52°C over the background BRET signal at 42°C for cells transfected with Nluc-MAPK14 target that were treated with DMSO and varying dye concentrations

### Example 20

The properties of environmentally sensitive dyes for thermal shift assays include having a gain in fluorescence upon binding to proteins that are unfolding due to thermal insult. Figures 35-37 demonstrate the fluorescent spectra for some of the synthesized dyes (described in Example 18) when added to cells lysates that had been transfected with Nluc-target protein, and then either heated at 42°C (baseline), 56°C (for CDK2-Nluc target), or 52°C (for Nluc-MAPK14 target). The higher temperature results in a gain in fluorescence thus showing the change in dye binding and fluorescent properties indicating that it is a useful dye for this application.

HeLa cells were transfected with CDK2-Nluc or Nluc-MAPKI4 DNA at a 1: 10 DNA ratio and incubated overnight. The cells were trypsinized using Trypsin-EDTA (0.25%) (Gibco) and re-suspended in Opti-MEM without phenol red (Gibco) + 1x Protease Inhibitor Cocktail (Promega), which had been reconstituted in DMSO (Sigma). The cells were seeded into wells of a 96-well, tall-chimney PCR plate (Fisherbrand) at a density of2 × 104 cells/well in an 90uL/well volume and treated with 10uL of 10× dye solution and heated at 42°C, 52°C, or 56°C for 3 minutes followed by cooling at 25°C for 2 minutes. Samples were transferred to a tissue culture plate and analyzed in fluorescent spectral mode on a Clariostar.

### Example 21

### Target genome editing

Targeted genome editing technologies to introduce reporter tags to a target protein of interest are used to evaluate ligand binding induced thermal stabilization at endogenous levels of protein expression in the relevant cellular context. Targeted genome editing technologies include, but are not limited to, clustered regularly-interspaced short palindromic repeats (CRISPR/Cas system), zinc finger nucleases (ZFN), transcription-activator-like effector-based nucleases (TALENs), and engineered meganucleases re-engineered homing endonucleases. Targeted protein fusions are made, in some embodiments, with a high affinity NLpoly, NLpoly, or NanoLuc^{®} luciferase to a protein of interest. These tags are fused to the N- or C- terminal of the protein of interest or internally within the target protein of interest. Assays are performed in both selected clonal cell lines as well as in unselected pools of cells. These assays are performed under live cell or lytic conditions. Assays in which the peptide reporters are used (high affinity NLpoly and NLpoly) require that the complementary peptide (i.e., NLpep) be present for reconstitution of active reporter luciferase and signal. In some embodiments, this is done lytically using a reagent that contains the complementary NLpep or through using mammalian cells that expresses the NLpep complementary to the NLpep used for the genomic editing of the target of interest.

### SEQUENCES

SEQ ID NO: 1 (wild-type OgLuc) - 
mftladfvgdwqqtagynqdqvleqgglsslfqalgvsvtpiqkvvlsgenglkadihviipyeglsgfqmgliemifkvvypv
ddhhfkiilhygtlvidgvtpnmidyfgrpypgiavfdgkqitvtgtlwngnkiyderlinpdgsllfrvtingvtgwrlcenila
SEQ ID NO: 2 (NANOLUC) - 
mvftledfvgdwrqtagynldqvleqggvsslfqnlgvsvtpiqrivlsgenglkidihviipyeglsgdqmgqiekifkvvypv
ddhhfkvilhygtlvidgvtpnmidyfgrpyegiavfdgkkitvtgtlwngnkiiderlinpdgsllfrvtingvtgwrlcerila
SEQ ID NO: 3 - gvtgwrlcerila
SEQ ID NO: 4 - 
mvftladfvgdwqqtagynqdqvleqgglsslfqalgvsvtpiqkvvlsgenglkadihviipyeglsgfqmgliemifkvvyp
vddhhfkiilhygtlvidgvtpnmidyfgrpypgiavfdgkqitvtgtlwngnkiyderlinpdgsllfrvtin
SEQ ID NO: 5 (NLpep86) - vsgwrlfkkis
SEQ ID NO: 6 (NLpoly11S) - 
mvftledfvgdweqtaaynldqvleqggvssllqnlavsvtpiqrivrsgenalkidihviipyeglsadqmaqieevfkvvypv
ddhhfkvilpygtlvidgvtpnmlnyfgrpyegiavfdgkkitvtgtlwngnkiiderlitpdgsmlfrvtins
SEQ ID NO: 7 (NLpoly156) -

mvftledfvgdwrqtagynldqvleqggvsslfqnlgvsvtpiqrivlsgenalkidihviipyeglsgdqmgqiekifkvvypvd
dhhfkvilhygtlvidgvtpnmidyfgrpyegiavfdgkkitvtgtlwngnkiiderlinpdgsllfrvtin

## Claims

1. A method to detect a target/ligand interaction, comprising the steps of:
(a) incubating a fusion of a target protein and a bioluminescent reporter polypeptide:
(i) in the presence of a ligand to produce a test sample, and
(ii) in the absence of a ligand, to produce a control sample;
(b) treating said test and control samples under conditions that cause the target protein to unfold to an appropriate extent;
(c) measuring signal from the reporter polypeptide in said test and control samples; and
(d) comparing the measurement made in step (c) between the test and control samples, wherein increase of the signal from said reporter polypeptide in the test sample compared to the control sample indicates the presence of a target/ligand interaction;
and wherein the bioluminescent reporter has 70% or greater sequence identity with SEQ ID NO: 1.

2. The method of claim 1, wherein a plurality of test samples are produced using a plurality of test ligands.

3. A method to detect a target/ligand interaction, comprising the steps of:
(a) incubating a fusion of a target protein and a bioluminescent reporter:
(i) in the presence of a ligand to produce a test sample, and
(ii) in the absence of a ligand, to produce a control sample;
(b) contacting the test and control samples with a hydrophobic dye that binds nonspecifically to aggregated, unfolded, or denatured proteins and/or hydrophobic peptide segments, wherein the emission spectrum of the bioluminescent reporter upon interaction with a substrate and the excitation spectrum of the hydrophobic dye that binds nonspecifically to aggregated, unfolded, or denatured proteins and/or hydrophobic peptide segments overlap;
(c) treating said test and control samples under conditions that cause the target protein to unfold to an appropriate extent;
(d) exposing the test and control samples to the substrate of the bioluminescent reporter;
(e) measuring signal from the hydrophobic dye that binds nonspecifically to aggregated, unfolded, or denatured proteins and/or hydrophobic peptide segments in said test and control samples; and
(f) comparing the measurement made in step (e) between the test and control samples, wherein decrease of the signal from said hydrophobic dye that binds nonspecifically to aggregated, unfolded, or denatured proteins and/or hydrophobic peptide segments in the test sample compared to the control sample indicates the presence of a target/ligand interaction,
and wherein the bioluminescent reporter has 70% or greater sequence identity with SEQ ID NO: 1.

4. The method of claim 1 or 3, wherein the fusion is within a cell, cell lysate, or reaction mixture; optionally wherein the fusion is expressed within the cell, cell lysate, or reaction mixture, and further optionally wherein the ligand is added exogenously to the cell, cell lysate, or reaction mixture

5. The method of claim 3 wherein the hydrophobic dye that binds nonspecifically to aggregated, unfolded, or denatured proteins and/or hydrophobic peptide segments is added exogenously to the cell, cell lysate, or reaction mixture.

6. The method of claim 1 or 3, wherein the bioluminescent reporter is a peptide or polypeptide tag that forms a bioluminescent complex upon interaction with a complement polypeptide or peptide.

7. The method of claim 2 wherein the complement polypeptide or peptide is added exogenously to the test and control samples prior to step (d) or the complement polypeptide or peptide is expressed within the cell, cell lysate, or reaction mixture.

8. The method of claim 1 or 3, wherein the conditions that cause the target protein to unfold to an appropriate extent comprise elevated temperature and/or a denaturant; optionally wherein, an elevated temperature comprises one or more temperatures either above physiologic temperature or near the approximate melting temperature of the target protein.

9. The method of claim 3, wherein the hydrophobic dye that binds nonspecifically to aggregated, unfolded, or denatured proteins and/or hydrophobic peptide segments binds preferentially to the folded or molten globule states of the protein; optionally wherein the fluorescence of the hydrophobic dye that binds nonspecifically to aggregated, unfolded, or denatured proteins and/or hydrophobic peptide segments is quenched by water, and further optionally wherein the environmentally-sensitive hydrophobic dye is SYPRO Orange, SYPRO Red, Nile Red, ANS, Bis-ANS, SYPRO Ruby, SYPRO Tangerine, and/or Dapoxyl Sulfonic Acid Sodium Salt, Protein Thermal Shift^{™} Dye (Life Technologies), Proteostat^{™} Dye (Enzo), or a combination thereof.

10. A method of screening a group of test ligands for binding to a target protein comprising:
(a) creating a plurality of test samples each comprising at least one test ligand and a fusion of a target protein and a bioluminescent reporter;
(b) creating at least one control sample comprising a fusion of a target protein and a bioluminescent reporter in the absence of a test ligand;
(c) contacting the test and control samples with a hydrophobic dye that binds nonspecifically to aggregated, unfolded, or denatured proteins and/or hydrophobic peptide segments, wherein the emission spectrum of the bioluminescent reporter upon interaction with a substrate and the excitation spectrum of the hydrophobic dye that binds nonspecifically to aggregated, unfolded, or denatured proteins and/or hydrophobic peptide segments overlap;
(d) treating the test and control samples under conditions that cause the target protein to unfold to an appropriate extent;
(e) exposing the test and control samples to the substrate of the bioluminescent reporter;
(f) measuring signal from the hydrophobic dye that binds nonspecifically to aggregated, unfolded, or denatured proteins and/or hydrophobic peptide segments in said test and control samples; and
(g) comparing the measurement made in step (f) between the test and control samples, wherein decrease of the signal from said hydrophobic dye that binds nonspecifically to aggregated, unfolded, or denatured proteins and/or hydrophobic peptide segments in one or more test samples compared to the control sample indicates the presence of a target/ligand interaction,
and wherein the bioluminescent reporter has 70% or greater sequence identity with SEQ ID NO: 1.

11. A method to detect a target/ligand interaction, comprising the steps of:
(a) incubating a fusion of a target protein and a bioluminescent reporter polypeptide in the presence of a ligand to produce a test sample;
(b) treating said test sample under conditions that cause the target protein to unfold to an appropriate extent;
(c) measuring signal from the reporter polypeptide in said test sample; and
(d) detecting target/ligand interaction based on the signal from said reporter polypeptide in the test sample, and wherein the difference or ratio of the signal is compared to a difference or ratio of signal in a control sample without the ligand, and wherein the bioluminescent reporter has 70% or greater sequence identity with SEQ ID NO: 1.

12. A method to detect a target/ligand interaction, comprising the steps of:
(a) incubating a fusion of a target protein and a bioluminescent reporter in the presence of a ligand to produce a test sample;
(b) contacting the test sample with a hydrophobic dye that binds nonspecifically to aggregated, unfolded, or denatured proteins and/or hydrophobic peptide segments, wherein the emission spectrum of the bioluminescent reporter upon interaction with a substrate and the excitation spectrum of the hydrophobic dye that binds nonspecifically to aggregated, unfolded, or denatured proteins and/or hydrophobic peptide segments overlap;
(c) treating test sample under conditions that cause the target protein to unfold to an appropriate extent;
(d) exposing the test sample to the substrate of the bioluminescent reporter;
(e) measuring signal from the hydrophobic dye that binds nonspecifically to aggregated, unfolded, or denatured proteins and/or hydrophobic peptide segments and bioluminescent reporter in said test sample; and
(f) detecting target/ligand interaction based on the signals from said hydrophobic dye that binds nonspecifically to aggregated, unfolded, or denatured proteins and/or hydrophobic peptide segments and bioluminescent reporter in the test sample, and wherein the difference or ratio of the signal is compared to a difference or ratio of signal in a control sample without the ligand,
and wherein the bioluminescent reporter has 70% or greater sequence identity with SEQ ID NO: 1.

13. A method to detect a target/ligand interaction within a live, intact cell, comprising the steps of:
(a) providing a live, intact cell expressing a fusion of a target protein and a bioluminescent reporter;
(b) allowing time for the target protein to interact with the ligand within the live, intact cell;
(c)(i)
(A) contacting the live, intact cell with a hydrophobic dye that binds nonspecifically to aggregated, unfolded, or denatured proteins and/or hydrophobic peptide segments, wherein the emission spectrum of the bioluminescent reporter upon interaction with a substrate and the excitation spectrum of the hydrophobic dye that binds nonspecifically to aggregated, unfolded, or denatured proteins and/or hydrophobic peptide segments overlap;
or
(c)(ii)
(A) lysing the cell to produce a cell lysate;
(B) contacting the cell lysate with a hydrophobic dye that binds nonspecifically to aggregated, unfolded, or denatured proteins and/or hydrophobic peptide segments, wherein the emission spectrum of the bioluminescent reporter upon interaction with a substrate and the excitation spectrum of the hydrophobic dye that binds nonspecifically to aggregated, unfolded, or denatured proteins and/or hydrophobic peptide segments overlap;
or
(c)(iii)
(A) contacting the live, intact cell with a hydrophobic dye that binds nonspecifically to aggregated, unfolded, or denatured proteins and/or hydrophobic peptide segments, wherein the emission spectrum of the bioluminescent reporter upon interaction with a substrate and the excitation spectrum of the hydrophobic dye that binds nonspecifically to aggregated, unfolded, or denatured proteins and/or hydrophobic peptide segments overlap;
(B) lysing the cell to produce a cell lysate;
(d) treating the cell lysate under conditions that cause the target protein to unfold to an appropriate extent;
(e) exposing the cell lysate to the substrate of the bioluminescent reporter;
(f) measuring signal from the hydrophobic dye that binds nonspecifically to aggregated, unfolded, or denatured proteins and/or hydrophobic peptide segments and bioluminescent reporter in the cell lysate; and
(g) detecting target/ligand interaction based on the signals from said hydrophobic dye that binds nonspecifically to aggregated, unfolded, or denatured proteins and/or hydrophobic peptide segments and bioluminescent reporter in the cell lysate, wherein the signal from said hydrophobic dye that binds nonspecifically to aggregated, unfolded, or denatured proteins and/or hydrophobic peptide segments in the cell lysate is compared to a control sample without the ligand; optionally further comprising a step of adding the ligand to the live, intact cell, and wherein the bioluminescent reporter has 70% or greater sequence identity with SEQ ID NO: 1.

14. A method to detect a target/ligand interaction within a live, intact cell comprising the steps of:
(a) providing a live, intact cell expressing a fusion of a target protein and a bioluminescent reporter;
(b)(i)
(A) allowing time for the target protein to interact with the ligand within the live, intact cell;
(B) treating the live, intact cell under conditions that cause the target protein to unfold to an appropriate extent;
or
(b)(ii)
(A) allowing time for the target protein to interact with the ligand within the live, intact cell;
(B) treating the live, intact cell under conditions that cause the target protein to unfold to an appropriate extent;
(C) lysing the cell to produce a cell lysate
or
(b)(iii)
(A) lysing the cell to produce a cell lysate
(B) allowing time for the target protein to interact with the ligand within the cell lysate;
(C) treating the cell lysate under conditions that cause the target protein to unfold to an appropriate extent;
(c) exposing the live, intact cell or cell lysate to the substrate of the bioluminescent reporter;
(d) measuring signal from the bioluminescent reporter in the live, intact cell or cell lysate; and
(e) detecting target/ligand interaction based on the signal from said bioluminescent reporter in the live, intact cell or cell lysate, and wherein the difference or ratio of the signal is compared to a difference or ratio of signal in a control sample without the ligand,
and wherein the bioluminescent reporter has 70% or greater sequence identity with SEQ ID NO: 1.

15. A method to detect a target/ligand interaction within a live, intact cell comprising the steps of:
(a) providing a live, intact cell expressing a fusion of a target protein and a bioluminescent reporter;
(b) optionally lysing the cell to produce a cell lysate
(c) allowing time for the target protein to interact with the ligand within the live, intact cell or cell lysate;
(d) exposing the live, intact cell or cell lysate to the substrate of the bioluminescent reporter;
(e) measuring signal from the bioluminescent reporter in the live, intact cell or cell lysate under pre-denaturing conditions;
(f) treating the live, intact cell or cell lysate under conditions that cause the target protein to unfold to an appropriate extent;
(g) measuring signal from the bioluminescent reporter in the live, intact cell or cell lysate under post-denaturing conditions; and
(h) detecting target/ligand interaction based on the difference or ratio in signal from the bioluminescent reporter under the pre-denaturing and post-denaturing conditions in the live, intact cell or cell lysate, and wherein the difference or ratio of the signal is compared to a difference or ratio of signal in a control sample without the ligand,
and wherein the bioluminescent reporter has 70% or greater sequence identity with SEQ ID NO: 1.

16. A method to detect a target/ligand interaction within a live, intact cell, comprising the steps of:
(a) providing a live, intact cell expressing a fusion of a target protein and a bioluminescent reporter;
(b)(i)
(A) allowing time for the target protein to interact with the ligand within the live, intact cell;
(B) treating the live, intact cell under conditions that cause the target protein to unfold to an appropriate extent;
(C) contacting the live, intact cell with a hydrophobic dye that binds nonspecifically to aggregated, unfolded, or denatured proteins and/or hydrophobic peptide segments, wherein the emission spectrum of the bioluminescent reporter upon interaction with a substrate and the excitation spectrum of the hydrophobic dye that binds nonspecifically to aggregated, unfolded, or denatured proteins and/or hydrophobic peptide segments overlap;
(D) exposing the live, intact cell or cell lysate to the substrate of the bioluminescent reporter;
or
(b)(ii)
(A) allowing time for the target protein to interact with the ligand within the live, intact cell;
(B) treating the live, intact cell under conditions that cause the target protein to unfold to an appropriate extent;
(C) lysing the cell to produce a cell lysate
(D) contacting the cell lysate with a hydrophobic dye that binds nonspecifically to aggregated, unfolded, or denatured proteins and/or hydrophobic peptide segments, wherein the emission spectrum of the bioluminescent reporter upon interaction with a substrate and the excitation spectrum of the environmentally-sensitive hydrophobic dye overlap;
(E) exposing the live, intact cell or cell lysate to the substrate of the bioluminescent reporter;
or
(b)(iii)
(A) lysing the cell to produce a cell lysate
(B) contacting the cell lysate with a hydrophobic dye that binds nonspecifically to aggregated, unfolded, or denatured proteins and/or hydrophobic peptide segments, wherein the emission spectrum of the bioluminescent reporter upon interaction with a substrate and the excitation spectrum of the hydrophobic dye that binds nonspecifically to aggregated, unfolded, or denatured proteins and/or hydrophobic peptide segments overlap;
(C) allowing time for the target protein to interact with the ligand within the cell lysate;
(D) treating the cell lysate under conditions that cause the target protein to unfold to an appropriate extent;
(E) exposing the live, intact cell or cell lysate to the substrate of the bioluminescent reporter;
or
(b)(iv)
(A) allowing time for the target protein to interact with the ligand within the live, intact cell;
(B) contacting the live, intact cell with a hydrophobic dye that binds nonspecifically to aggregated, unfolded, or denatured proteins and/or hydrophobic peptide segments, wherein the emission spectrum of the bioluminescent reporter upon interaction with a substrate and the excitation spectrum of the hydrophobic dye that binds nonspecifically to aggregated, unfolded, or denatured proteins and/or hydrophobic peptide segments overlap;
(C) exposing the live, intact cell to the substrate of the bioluminescent reporter;
(D) measuring signal from the hydrophobic dye that binds nonspecifically to aggregated, unfolded, or denatured proteins and/or hydrophobic peptide segments in the live, intact cell under pre-denaturing conditions;
(E) treating the live, intact cell under conditions that cause the target protein to unfold to an appropriate extent;
optionally further comprising a step of adding the ligand to the live, intact cell prior to step (D);
(c) measuring signal from the hydrophobic dye that binds nonspecifically to aggregated, unfolded, or denatured proteins and/or hydrophobic peptide segments and bioluminescent reporter in the live, intact cell or cell lysate; and
(d)(i) when steps (b)(i),(b)(ii) or (b)(iii) are performed, detecting target/ligand interaction based on the ratiometric signal from said hydrophobic dye and bioluminescent reporter in the live, intact cell or cell lysate, wherein the hydrophobic dye binds nonspecifically to aggregated, unfolded, or denatured proteins and/or hydrophobic peptide segments;
or
(d)(ii) when step (b)(iv) is performed, detecting target/ligand interaction based on the difference or ratio in signals from said hydrophobic dye and bioluminescent reporter under the pre-denaturing and post-denaturing conditions in the live, intact cell, wherein the hydrophobic dye binds nonspecifically to aggregated, unfolded, or denatured proteins and/or hydrophobic peptide segments, and wherein the difference or ratio of the signal is compared to a difference or ratio of signal in a control sample without the ligand,
and wherein the bioluminescent reporter has 70% or greater sequence identity with SEQ ID NO: 1.

17. The method of claim 13 or 16 wherein the ligand is endogenous to the live, intact cell.

## Patentansprüche

1. Verfahren zum Nachweisen einer Target-Ligand-Interaktion, das die folgenden Schritte umfasst:
(a) Inkubieren einer Fusion eines Zielproteins und eines biolumineszenten Reporterpolypeptids:
(i) in Anwesenheit eines Liganden, um eine Testprobe zu produzieren, und
(ii) in Abwesenheit eines Liganden, um eine Kontrollprobe zu produzieren;
(b) Behandeln der Test- und Kontrollproben unter Bedingungen, die das Zielprotein veranlassen, sich in einem entsprechenden Ausmaß zu entfalten;
(c) Messen des Signals des Reporterpolypeptids in den Test- und Kontrollproben; und
(d) Vergleichen der in Schritt (c) durchgeführten Messung zwischen den Test- und Kontrollproben, wobei eine Zunahme des Signals von dem Reporterpolypeptid in der Testprobe verglichen mit der Kontrollprobe die Anwesenheit einer Target-Ligand-Interaktion anzeigt;
und wobei der biolumineszente Reporter 70 % oder mehr Sequenzidentität mit SEQ **ID** NO: 1 aufweist.

2. Verfahren nach Anspruch 1, wobei eine Vielzahl von Testproben unter Verwendung einer Vielzahl von Testliganden produziert wird.

3. Verfahren zum Nachweisen einer Target-Ligand-Interaktion, das die folgenden Schritte umfasst:
(a) Inkubieren einer Fusion eines Zielproteins und eines biolumineszenten Reporters:
(i) in Anwesenheit eines Liganden, um eine Testprobe zu produzieren, und
(ii) in Abwesenheit eines Liganden, um eine Kontrollprobe zu produzieren;
(b) Inkontaktbringen der Test- und Kontrollproben mit einem hydrophoben Farbstoff, der unspezifisch an aggregierte, entfaltete oder denaturierte Proteine und/oder hydrophobe Peptidsegmente bindet, wobei sich das Emissionsspektrum des biolumineszenten Reporters bei Interaktion mit einem Substrat und das Anregungsspektrum des hydrophoben Farbstoffs, der unspezifisch an aggregierte, entfaltete oder denaturierte Proteine und/oder hydrophobe Peptidsegmente bindet, überlappen;
(c) Behandeln der Test- und Kontrollproben unter Bedingungen, die das Zielprotein veranlassen, sich in einem entsprechenden Ausmaß zu entfalten;
(d) Aussetzen der Test- und Kontrollproben gegenüber dem Substrat des biolumineszenten Reporters;
(e) Messen des Signals von dem hydrophoben Farbstoff, der unspezifisch an aggregierte, entfaltete oder denaturierte Proteine und/oder hydrophobe Peptidsegmente in den Test- und Kontrollproben bindet; und
(f) Vergleichen der in Schritt (e) durchgeführten Messung zwischen den Test- und Kontrollproben, wobei eine Abnahme des Signals von dem hydrophoben Farbstoff, der unspezifisch an aggregierte, entfaltete oder denaturierte Proteine und/oder hydrophobe Peptidsegmente in der Testprobe im Vergleich zur Kontrollprobe bindet, die Anwesenheit einer Target-Ligand-Interaktion anzeigt,
und wobei der biolumineszente Reporter 70 % oder mehr Sequenzidentität mit SEQ **ID** NO: 1 aufweist.

4. Verfahren nach Anspruch 1 oder 3, wobei die Fusion innerhalb einer Zelle, eines Zelllysats oder eines Reaktionsgemischs vorliegt; wobei die Fusion optional innerhalb der Zelle, des Zelllysats oder des Reaktionsgemischs exprimiert wird, und wobei der Ligand ferner optional der Zelle, dem Zelllysat oder dem Reaktionsgemisch exogen zugegeben wird.

5. Verfahren nach Anspruch 3, wobei der hydrophobe Farbstoff, der unspezifisch an aggregierte, entfaltete oder denaturierte Proteine und/oder hydrophobe Peptidsegmente bindet, der Zelle, dem Zelllysat oder dem Reaktionsgemisch exogen zugegeben wird.

6. Verfahren nach Anspruch 1 oder 3, wobei der biolumineszente Reporter ein Peptid oder ein Polypeptid-Tag ist, das bei Interaktion mit einem Komplementpolypeptid oder -peptid einen biolumineszenten Komplex bildet.

7. Verfahren nach Anspruch 2, wobei das Komplementpolypeptid oder -peptid vor Schritt (d) den Test- und Kontrollproben exogen zugegeben wird oder das Komplementpolypeptid oder -peptid in der Zelle, dem Zelllysat oder dem Reaktionsgemisch exprimiert wird.

8. Verfahren nach Anspruch 1 oder 3, wobei die Bedingungen, die veranlassen, dass sich das Zielprotein in einem entsprechenden Ausmaß entfaltet, eine erhöhte Temperatur und/oder ein Denaturierungsmittel umfassen; wobei optional eine erhöhte Temperatur eine oder mehrere Temperaturen entweder über der physiologischen Temperatur oder nahe der ungefähren Schmelztemperatur des Zielproteins umfasst.

9. Verfahren nach Anspruch 3, wobei der hydrophobe Farbstoff, der unspezifisch an aggregierte, ungefaltete oder denaturierte Proteine und/oder hydrophobe Peptidsegmente bindet, vorzugsweise an den gefalteten oder geschmolzenen Globuluszustand des Proteins bindet; wobei die Fluoreszenz des hydrophoben Farbstoffs, der unspezifisch an aggregierte, entfaltete oder denaturierte Proteine und/oder hydrophobe Peptidsegmente bindet, optional durch Wasser gequencht wird, und wobei der umweltempfindliche hydrophobe Farbstoff ferner optional SYPRO Orange, SYPRO Rot, Nilrot, ANS, Bis-ANS, SYPRO Rubin, SYPRO Mandarine und/oder Dapoxylsulfonsäure-Natriumsalz, Protein Thermal Shift^{™} Farbstoff (Life Technologies), Proteostat^{™} Farbstoff (Enzo), oder eine Kombination davon ist.

10. Verfahren zum Screening einer Gruppe von Testliganden auf Bindung an ein Zielprotein, umfassend:
(a) Erzeugen einer Vielzahl von Testproben, die jeweils mindestens einen Testliganden und eine Fusion eines Zielproteins und eines biolumineszenten Reporters umfassen;
(b) Erzeugen mindestens einer Kontrollprobe, die eine Fusion eines Zielproteins und eines biolumineszenten Reporters in Abwesenheit eines Testliganden umfasst;
(c) Inkontaktbringen der Test- und Kontrollproben mit einem hydrophoben Farbstoff, der unspezifisch an aggregierte, entfaltete oder denaturierte Proteine und/oder hydrophobe Peptidsegmente bindet, wobei sich das Emissionsspektrum des biolumineszenten Reporters bei Interaktion mit einem Substrat und das Anregungsspektrum des hydrophoben Farbstoffs, der unspezifisch an aggregierte, entfaltete oder denaturierte Proteine und/oder hydrophobe Peptidsegmente bindet, überlappen;
(d) Behandeln der Test- und Kontrollproben unter Bedingungen, die das Zielprotein veranlassen, sich in einem entsprechenden Ausmaß zu entfalten;
(e) Aussetzen der Test- und Kontrollproben gegenüber dem Substrat des biolumineszenten Reporters;
(f) Messen des Signals von dem hydrophoben Farbstoff, der unspezifisch an aggregierte, entfaltete oder denaturierte Proteine und/oder hydrophobe Peptidsegmente in den Test- und Kontrollproben bindet; und
(g) Vergleichen der in Schritt (f) durchgeführten Messung zwischen den Test- und Kontrollproben, wobei eine Abnahme des Signals von dem hydrophoben Farbstoff, der unspezifisch an aggregierte, entfaltete oder denaturierte Proteine und/oder hydrophobe Peptidsegmente in einer oder mehreren Testprobe im Vergleich zur Kontrollprobe bindet, die Anwesenheit einer Target-Ligand-Interaktion anzeigt,
und wobei der biolumineszente Reporter 70 % oder mehr Sequenzidentität mit SEQ ID NO: 1 aufweist.

11. Verfahren zum Nachweisen einer Target-Ligand-Interaktion, das die folgenden Schritte umfasst:
(a) Inkubieren einer Fusion eines Zielproteins und eines biolumineszenten Reporterpolypeptids in Anwesenheit eines Liganden, um eine Testprobe zu produzieren;
(b) Behandeln der Testprobe unter Bedingungen, die das Zielprotein veranlassen, sich in einem entsprechenden Ausmaß zu entfalten;
(c) Messen des Signals des Reporterpolypeptids in der Testprobe; und
(d) Nachweisen der Target-Ligand-Interaktion basierend auf dem Signal des Reporterpolypeptids in der Testprobe, und wobei die Differenz oder das Verhältnis des Signals mit einer Differenz oder einem Verhältnis des Signals in einer Kontrollprobe ohne den Liganden verglichen wird, und wobei der biolumineszente Reporter 70 % oder mehr Sequenzidentität mit SEQ ID NO: 1 aufweist.

12. Verfahren zum Nachweisen einer Target-Ligand-Interaktion, das die folgenden Schritte umfasst:
(a) Inkubieren einer Fusion eines Zielproteins und eines biolumineszenten Reporters in Anwesenheit eines Liganden, um eine Testprobe zu produzieren;
(b) Inkontaktbringen der Testprobe mit einem hydrophoben Farbstoff, der unspezifisch an aggregierte, entfaltete oder denaturierte Proteine und/oder hydrophobe Peptidsegmente bindet, wobei sich das Emissionsspektrum des biolumineszenten Reporters bei Interaktion mit einem Substrat und das Anregungsspektrum des hydrophoben Farbstoffs, der unspezifisch an aggregierte, entfaltete oder denaturierte Proteine und/oder hydrophobe Peptidsegmente bindet, überlappen;
(c) Behandeln der Testprobe unter Bedingungen, die das Zielprotein veranlassen, sich in einem entsprechenden Ausmaß zu entfalten;
(d) Aussetzen der Testprobe gegenüber dem Substrat des biolumineszenten Reporters;
(e) Messen des Signals von dem hydrophoben Farbstoff, der unspezifisch an aggregierte, entfaltete oder denaturierte Proteine und/oder hydrophobe Peptidsegmente in der Testprobe bindet; und
(f) Nachweisen der Target-Ligand-Interaktion basierend auf den Signalen des hydrophoben Farbstoffs, der unspezifisch an aggregierte, entfaltete oder denaturierte Proteine und/oder hydrophobe Peptidsegmente bindet, und des biolumineszenten Reporters in der Testprobe, und wobei die Differenz oder das Verhältnis des Signals mit einer Differenz oder einem Verhältnis des Signals in einer Kontrollprobe ohne den Liganden verglichen wird,
und wobei der biolumineszente Reporter 70 % oder mehr Sequenzidentität mit SEQ ID NO: 1 aufweist.

13. Verfahren zum Nachweisen einer Target-Ligand-Interaktion in einer lebenden, intakten Zelle, das die folgenden Schritte umfasst:
(a) Bereitstellen einer lebenden, intakten Zelle, die eine Fusion eines Zielproteins und eines biolumineszenten Reporters exprimiert;
(b) Einräumen von Zeit für das Zielprotein, um mit dem Liganden innerhalb der lebenden, intakten Zelle zu interagieren;
(c)(i)
(A) Inkontaktbringen der lebenden, intakten Zelle mit einem hydrophoben Farbstoff, der unspezifisch an aggregierte, entfaltete oder denaturierte Proteine und/oder hydrophobe Peptidsegmente bindet, wobei sich das Emissionsspektrum des biolumineszenten Reporters bei Interaktion mit einem Substrat und das Anregungsspektrum des hydrophoben Farbstoffs, der unspezifisch an aggregierte, entfaltete oder denaturierte Proteine und/oder hydrophobe Peptidsegmente bindet, überlappen;
oder
(c)(ii)
(A) Lysieren der Zelle, um ein Zelllysat zu produzieren;
(B) Inkontaktbringen des Zelllysats mit einem hydrophoben Farbstoff, der unspezifisch an aggregierte, entfaltete oder denaturierte Proteine und/oder hydrophobe Peptidsegmente bindet, wobei sich das Emissionsspektrum des biolumineszenten Reporters bei Interaktion mit einem Substrat und das Anregungsspektrum des hydrophoben Farbstoffs, der unspezifisch an aggregierte, entfaltete oder denaturierte Proteine und/oder hydrophobe Peptidsegmente bindet, überlappen;
oder
(c)(iii)
(A) Inkontaktbringen der lebenden, intakten Zelle mit einem hydrophoben Farbstoff, der unspezifisch an aggregierte, entfaltete oder denaturierte Proteine und/oder hydrophobe Peptidsegmente bindet, wobei sich das Emissionsspektrum des biolumineszenten Reporters bei Interaktion mit einem Substrat und das Anregungsspektrum des hydrophoben Farbstoffs, der unspezifisch an aggregierte, entfaltete oder denaturierte Proteine und/oder hydrophobe Peptidsegmente bindet, überlappen;
(B) Lysieren der Zelle, um ein Zelllysat zu produzieren;
(d) Behandeln des Zelllysats unter Bedingungen, die das Zielprotein veranlassen, sich in einem entsprechenden Ausmaß zu entfalten;
(e) Aussetzen des Zelllysats gegenüber dem Substrat des biolumineszenten Reporters;
(f) Messen des Signals von dem hydrophoben Farbstoff, der unspezifisch an aggregierte, entfaltete oder denaturierte Proteine und/oder hydrophobe Peptidsegmente und den biolumineszenten Reporter im Zelllysat bindet; und
(g) Nachweisen der Target-Ligand-Interaktion basierend auf den Signalen des hydrophoben Farbstoffs, der unspezifisch an aggregierte, entfaltete oder denaturierte Proteine und/oder hydrophobe Peptidsegmente bindet, und des biolumineszenten Reporters in dem Zelllysat, wobei das Signal des hydrophoben Farbstoffs, der unspezifisch an aggregierte, entfaltete oder denaturierte Proteine und/oder hydrophobe Peptidsegmente im Zelllysat bindet, mit einer Kontrollprobe ohne den Liganden verglichen wird;
optional ferner umfassend einen Schritt des Zugebens des Liganden zur lebenden, intakten Zelle,
und wobei der biolumineszente Reporter eine Sequenzidentität von 70 % oder mehr mit SEQ ID NO: 1 aufweist.

14. Verfahren zum Nachweisen einer Target-Ligand-Interaktion in einer lebenden, intakten Zelle, das die folgenden Schritte umfasst:
(a) Bereitstellen einer lebenden, intakten Zelle, die eine Fusion eines Zielproteins und eines biolumineszenten Reporters exprimiert;
(b)(i)
(A) Einräumen von Zeit für das Zielprotein, um mit dem Liganden innerhalb der lebenden, intakten Zelle zu interagieren;
(B) Behandeln der lebenden, intakten Zelle unter Bedingungen, die das Zielprotein veranlassen, sich in einem entsprechenden Ausmaß zu entfalten;
oder
(b)(ii)
(A) Einräumen von Zeit für das Zielprotein, um mit dem Liganden innerhalb der lebenden, intakten Zelle zu interagieren;
(B) Behandeln der lebenden, intakten Zelle unter Bedingungen, die das Zielprotein veranlassen, sich in einem entsprechenden Ausmaß zu entfalten;
(C) Lysieren der Zelle, um ein Zelllysat zu produzieren;
oder
(b)(iii)
(A) Lysieren der Zelle, um ein Zelllysat zu produzieren;
(B) Einräumen von Zeit für das Zielprotein, um mit dem Liganden innerhalb des Zelllysats zu interagieren;
(C) Behandeln des Zelllysats unter Bedingungen, die das Zielprotein veranlassen, sich in einem entsprechenden Ausmaß zu entfalten;
(c) Aussetzen der lebenden, intakten Zelle oder des Zelllysats gegenüber dem Substrat des biolumineszenten Reporters;
(d) Messen des Signals des biolumineszenten Reporters in der lebenden, intakten Zelle oder dem Zelllysat; und
(e) Nachweisen der Target-Ligand-Interaktion basierend auf dem Signal des biolumineszenten Reporters in der lebenden, intakten Zelle oder dem Zelllysat, und wobei die Differenz oder das Verhältnis des Signals mit einer Differenz oder einem Verhältnis des Signals in einer Kontrollprobe ohne den Liganden verglichen wird,
und wobei der biolumineszente Reporter 70 % oder mehr Sequenzidentität mit SEQ ID NO: 1 aufweist.

15. Verfahren zum Nachweisen einer Target-Ligand-Interaktion in einer lebenden, intakten Zelle, das die folgenden Schritte umfasst:
(a) Bereitstellen einer lebenden, intakten Zelle, die eine Fusion eines Zielproteins und eines biolumineszenten Reporters exprimiert;
(b) optional Lysieren der Zelle, um ein Zelllysat zu produzieren;
(c) Einräumen von Zeit für das Zielprotein, um mit dem Liganden innerhalb der lebenden, intakten Zelle oder dem Zelllysat zu interagieren;
(d) Aussetzen der lebenden, intakten Zelle oder des Zelllysats gegenüber dem Substrat des biolumineszenten Reporters;
(e) Messen des Signals des biolumineszenten Reporters in der lebenden, intakten Zelle oder dem Zelllysat unter Vordenaturierungsbedingungen;
(f) Behandeln der lebenden, intakten Zelle oder des Zelllysats unter Bedingungen, die das Zielprotein veranlassen, sich in einem entsprechenden Ausmaß zu entfalten;
(g) Messen des Signals des biolumineszenten Reporters in der lebenden, intakten Zelle oder dem Zelllysat unter Nachdenaturierungsbedingungen; und
(h) Nachweisen der Target-Ligand-Interaktion basierend auf der Differenz oder dem Verhältnis des Signals von dem biolumineszenten Reporter unter den Vordenaturierungs- und Nachdenaturierungsbedingungen in der lebenden, intakten Zelle oder dem Zelllysat, und wobei die Differenz oder das Verhältnis des Signals mit einer Differenz oder einem Verhältnis des Signals in einer Kontrollprobe ohne den Liganden verglichen wird,
und wobei der biolumineszente Reporter 70 % oder mehr Sequenzidentität mit SEQ ID NO: 1 aufweist.

16. Verfahren zum Nachweisen einer Target-Ligand-Interaktion in einer lebenden, intakten Zelle, das die folgenden Schritte umfasst:
(a) Bereitstellen einer lebenden, intakten Zelle, die eine Fusion eines Zielproteins und eines biolumineszenten Reporters exprimiert;
(b)(i)
(A) Einräumen von Zeit für das Zielprotein, um mit dem Liganden innerhalb der lebenden, intakten Zelle zu interagieren;
(B) Behandeln der lebenden, intakten Zelle unter Bedingungen, die das Zielprotein veranlassen, sich in einem entsprechenden Ausmaß zu entfalten;
(C) Inkontaktbringen der lebenden, intakten Zelle mit einem hydrophoben Farbstoff, der unspezifisch an aggregierte, entfaltete oder denaturierte Proteine und/oder hydrophobe Peptidsegmente bindet, wobei sich das Emissionsspektrum des biolumineszenten Reporters bei Interaktion mit einem Substrat und das Anregungsspektrum des hydrophoben Farbstoffs, der unspezifisch an aggregierte, entfaltete oder denaturierte Proteine und/oder hydrophobe Peptidsegmente bindet, überlappen;
(D) Aussetzen der lebenden, intakten Zelle oder des Zelllysats gegenüber dem Substrat des biolumineszenten Reporters;
oder
(b)(ii)
(A) Einräumen von Zeit für das Zielprotein, um mit dem Liganden innerhalb der lebenden, intakten Zelle zu interagieren;
(B) Behandeln der lebenden, intakten Zelle unter Bedingungen, die das Zielprotein veranlassen, sich in einem entsprechenden Ausmaß zu entfalten;
(C) Lysieren der Zelle, um ein Zelllysat zu produzieren;
(D) Inkontaktbringen des Zelllysats mit einem hydrophoben Farbstoff, der unspezifisch an aggregierte, entfaltete oder denaturierte Proteine und/oder hydrophobe Peptidsegmente bindet, wobei sich das Emissionsspektrum des biolumineszenten Reporters bei Interaktion mit einem Substrat und das Anregungsspektrum des umgebungsempfindlichen hydrophoben Farbstoffs überlappen;
(E) Aussetzen der lebenden, intakten Zelle oder des Zelllysats gegenüber dem Substrat des biolumineszenten Reporters;
oder
(b)(iii)
(A) Lysieren der Zelle, um ein Zelllysat zu produzieren;
(B) Inkontaktbringen des Zelllysats mit einem hydrophoben Farbstoff, der unspezifisch an aggregierte, entfaltete oder denaturierte Proteine und/oder hydrophobe Peptidsegmente bindet, wobei sich das Emissionsspektrum des biolumineszenten Reporters bei Interaktion mit einem Substrat und das Anregungsspektrum des hydrophoben Farbstoffs, der unspezifisch an aggregierte, entfaltete oder denaturierte Proteine und/oder hydrophobe Peptidsegmente bindet, überlappen;
(C) Einräumen von Zeit für das Zielprotein, um mit dem Liganden innerhalb des Zelllysats zu interagieren;
(D) Behandeln des Zelllysats unter Bedingungen, die das Zielprotein veranlassen, sich in einem entsprechenden Ausmaß zu entfalten;
(E) Aussetzen der lebenden, intakten Zelle oder des Zelllysats gegenüber dem Substrat des biolumineszenten Reporters;
oder
(b)(iv)
(A) Einräumen von Zeit für das Zielprotein, um mit dem Liganden innerhalb der lebenden, intakten Zelle zu interagieren;
(B) Inkontaktbringen der lebenden, intakten Zelle mit einem hydrophoben Farbstoff, der unspezifisch an aggregierte, entfaltete oder denaturierte Proteine und/oder hydrophobe Peptidsegmente bindet, wobei sich das Emissionsspektrum des biolumineszenten Reporters bei Interaktion mit einem Substrat und das Anregungsspektrum des hydrophoben Farbstoffs, der unspezifisch an aggregierte, entfaltete oder denaturierte Proteine und/oder hydrophobe Peptidsegmente bindet, überlappen;
(C) Aussetzen der lebenden, intakten Zelle gegenüber dem Substrat des biolumineszenten Reporters;
(D) Messen des Signals des hydrophoben Farbstoffs, der unspezifisch an aggregierte, entfaltete oder denaturierte Proteine und/oder hydrophobe Peptidsegmente in der lebenden, intakten Zelle bindet, unter Vordenaturierungsbedingungen;
(E) Behandeln der lebenden, intakten Zelle unter Bedingungen, die das Zielprotein veranlassen, sich in einem entsprechenden Ausmaß zu entfalten;
optional ferner umfassend einen Schritt des Zugebens des Liganden zur lebenden, intakten Zelle vor Schritt (D);
(c) Messen des Signals des hydrophoben Farbstoffs, der unspezifisch an aggregierte, entfaltete oder denaturierte Proteine und/oder hydrophobe Peptidsegmente bindet, und des biolumineszenten Reporters in der lebenden, intakten Zelle oder dem Zelllysat; und
(d)(i) wenn die Schritte (b)(i), (b)(ii) oder (b)(iii) durchgeführt werden, Nachweisen der Target-Ligand-Interaktion basierend auf dem ratiometrischen Signal von dem hydrophoben Farbstoff und dem biolumineszenten Reporter in der lebenden, intakten Zelle oder dem Zelllysat, wobei der hydrophobe Farbstoff unspezifisch an aggregierte, ungefaltete oder denaturierte Proteine und/oder hydrophobe Peptidsegmente bindet;
oder
(d)(ii) wenn Schritt (b)(iv) durchgeführt wird, Nachweisen der Target-Ligand-Interaktion basierend auf der Differenz oder dem Verhältnis der Signale des hydrophoben Farbstoffs und des biolumineszenten Reporters unter den Vordenaturierungs- und Nachdenaturierungsbedingungen in der lebenden, intakten Zelle, wobei der hydrophobe Farbstoff unspezifisch an aggregierte, entfaltete oder denaturierte Proteine und/oder hydrophobe Peptidsegmente bindet, und wobei die Differenz oder das Verhältnis des Signals mit einer Differenz oder einem Verhältnis des Signals in einer Kontrollprobe ohne den Liganden verglichen wird,
und wobei der biolumineszente Reporter 70 % oder mehr Sequenzidentität mit SEQ ID NO: 1 aufweist.

17. Verfahren nach Anspruch 13 oder 16, wobei der Ligand in der lebenden, intakten Zelle endogen ist.

## Revendications

1. Procédé de détection d'une interaction cible/ligand, comprenant les étapes consistant à :
(a) incuber une fusion d'une protéine cible et d'un polypeptide rapporteur bioluminescent :
(i) en présence d'un ligand pour produire un échantillon d'essai, et
(ii) en absence d'un ligand, pour produire un échantillon témoin ;
(b) traiter lesdits échantillons d'essai et témoin dans des conditions qui provoquent le dépliage de la protéine cible dans une mesure appropriée ;
(c) mesurer un signal provenant du polypeptide rapporteur dans lesdits échantillons d'essai et témoin ; et
(d) comparer la mesure effectuée à l'étape (c) entre les échantillons d'essai et témoin, dans lequel une augmentation du signal provenant dudit polypeptide rapporteur dans l'échantillon d'essai par comparaison à l'échantillon témoin indique la présence d'une interaction cible/ligand ;
et dans lequel le rapporteur bioluminescent a une identité de séquence de 70 % ou plus avec la SEQ ID NO : 1.

2. Procédé selon la revendication 1, dans lequel une pluralité d'échantillons d'essai sont produits à l'aide d'une pluralité de ligands d'essai.

3. Procédé de détection d'une interaction cible/ligand, comprenant les étapes consistant à :
(a) incuber une fusion d'une protéine cible et d'un rapporteur bioluminescent :
(i) en présence d'un ligand pour produire un échantillon d'essai, et
(ii) en absence d'un ligand, pour produire un échantillon témoin ;
(b) mettre en contact les échantillons d'essai et témoin avec un colorant hydrophobe qui se lie de manière non spécifique à des protéines agrégées, dépliées, ou dénaturées et/ou à des segments peptidiques hydrophobes, dans lequel le spectre d'émission du rapporteur bioluminescent lors de l'interaction avec un substrat et le spectre d'excitation du colorant hydrophobe qui se lie de manière non spécifique à des protéines agrégées, dépliées, ou dénaturées et/ou à des segments peptidiques hydrophobes se chevauchent ;
(c) traiter lesdits échantillons d'essai et témoin dans des conditions qui provoquent le dépliage de la protéine cible dans une mesure appropriée ;
(d) exposer les échantillons d'essai et témoin au substrat du rapporteur bioluminescent ;
(e) mesurer un signal provenant du colorant hydrophobe qui se lie de manière non spécifique à des protéines agrégées, dépliées, ou dénaturées et/ou à des segments peptidiques hydrophobes dans lesdits échantillons d'essai et témoin ; et
(f) comparer la mesure effectuée à l'étape (e) entre les échantillons d'essai et témoin, dans lequel une diminution du signal provenant du colorant hydrophobe qui se lie de manière non spécifique à des protéines agrégées, dépliées, ou dénaturées et/ou à des segments peptidiques hydrophobes dans l'échantillon d'essai par comparaison à l'échantillon témoin indique la présence d'une interaction cible/ligand,
et dans lequel le rapporteur bioluminescent a une identité de séquence de 70 % ou plus avec la SEQ ID NO : 1.

4. Procédé selon la revendication 1 ou 3, dans lequel la fusion se déroule à l'intérieur d'une cellule, d'un lysat cellulaire, ou d'un mélange réactionnel ; facultativement, dans lequel la fusion est exprimée à l'intérieur de la cellule, du lysat cellulaire, ou du mélange réactionnel, et en outre facultativement, dans lequel le ligand est ajouté de manière exogène à la cellule, au lysat cellulaire, ou au mélange réactionnel.

5. Procédé selon la revendication 3, dans lequel le colorant hydrophobe qui se lie de manière non spécifique à des protéines agrégées, dépliées, ou dénaturées et/ou à des segments peptidiques hydrophobes est ajouté de manière exogène à la cellule, au lysat cellulaire, ou au mélange réactionnel.

6. Procédé selon la revendication 1 ou 3, dans lequel le rapporteur bioluminescent est un marqueur peptidique ou polypeptidique qui forme un complexe bioluminescent lors de l'interaction avec un polypeptide ou un peptide complément.

7. Procédé selon la revendication 2, dans lequel le polypeptide ou le peptide complément est ajouté de manière exogène aux échantillons d'essai et témoin avant l'étape (d) ou le polypeptide ou le peptide complément est exprimé à l'intérieur de la cellule, du lysat cellulaire, ou du mélange réactionnel.

8. Procédé selon la revendication 1 ou 3, dans lequel les conditions qui provoquent le dépliage de la protéine cible dans une mesure appropriée comprennent une température élevée et/ou un dénaturant ; facultativement, dans lequel, une température élevée comprend une ou plusieurs températures supérieures à une température physiologique ou proches de la température de fusion approximative de la protéine cible.

9. Procédé selon la revendication 3, dans lequel le colorant hydrophobe qui se lie de manière non spécifique à des protéines agrégées, dépliées, ou dénaturées et/ou à des segments peptidiques hydrophobes se lie préférentiellement aux états de globule replié ou fondu de la protéine ; facultativement dans lequel la fluorescence du colorant hydrophobe qui se lie de manière non spécifique à des protéines agrégées, dépliées, ou dénaturées et/ou à des segments peptidiques hydrophobes est éteinte par de l'eau, et en outre facultativement dans lequel le colorant hydrophobe sensible à l'environnement est SYPRO Orange, SYPRO Red, Nile Red, ANS, Bis-ANS, SYPRO Ruby, SYPRO Tangerine et/ou sel de sodium d'acide Dapoxyl sulfonique, colorant Protein Thermal Shift^{™} (Life Technologies), colorant Proteostat^{™} (Enzo), ou une combinaison de ceux-ci.

10. Procédé de criblage d'un groupe de ligands d'essai quant à une liaison à une protéine cible, comprenant :
(a) la création d'une pluralité d'échantillons d'essai, chacun comprenant au moins un ligand d'essai et une fusion d'une protéine cible et d'un rapporteur bioluminescent ;
(b) la création d'au moins un échantillon témoin comprenant une fusion d'une protéine cible et d'un rapporteur bioluminescent en l'absence d'un ligand d'essai ;
(c) la mise en contact des échantillons d'essai et témoin avec un colorant hydrophobe qui se lie de manière non spécifique à des protéines agrégées, dépliées, ou dénaturées et/ou à des segments peptidiques hydrophobes, dans lequel le spectre d'émission du rapporteur bioluminescent lors de l'interaction avec un substrat et le spectre d'excitation du colorant hydrophobe qui se lie de manière non spécifique à des protéines agrégées, dépliées, ou dénaturées et/ou à des segments peptidiques hydrophobes se chevauchent ;
(d) le traitement des échantillons d'essai et témoin dans des conditions qui provoquent le dépliage de la protéine cible dans une mesure appropriée ;
(e) l'exposition des échantillons d'essai et témoin au substrat du rapporteur bioluminescent ;
(f) la mesure du signal provenant du colorant hydrophobe qui se lie de manière non spécifique à des protéines agrégées, dépliées, ou dénaturées et/ou à des segments peptidiques hydrophobes dans lesdits échantillons d'essai et témoin ; et
(g) la comparaison de la mesure effectuée à l'étape (f) entre les échantillons d'essai et témoin, dans lequel une diminution du signal provenant dudit colorant hydrophobe qui se lie de manière non spécifique à des protéines agrégées, dépliées, ou dénaturées et/ou à des segments peptidiques hydrophobes dans un ou plusieurs échantillons d'essai par comparaison à l'échantillon témoin indique la présence d'une interaction cible/ligand,
et dans lequel le rapporteur bioluminescent a une identité de séquence de 70 % ou plus avec la SEQ ID NO : 1.

11. Procédé de détection d'une interaction cible/ligand, comprenant les étapes consistant à :
(a) incuber une fusion d'une protéine cible et d'un polypeptide rapporteur bioluminescent en présence d'un ligand pour produire un échantillon d'essai ;
(b) traiter ledit échantillon d'essai dans des conditions qui provoquent le dépliage de la protéine cible dans une mesure appropriée ;
(c) mesurer un signal provenant du polypeptide rapporteur dans ledit échantillon d'essai ; et
(d) détecter l'interaction cible/ligand sur la base du signal provenant dudit polypeptide rapporteur dans l'échantillon d'essai, et dans lequel la différence ou le rapport du signal est comparé à une différence ou un rapport de signal dans un échantillon témoin sans le ligand,
et dans lequel le rapporteur bioluminescent a une identité de séquence de 70 % ou plus avec la SEQ ID NO : 1.

12. Procédé de détection d'une interaction cible/ligand, comprenant les étapes consistant à :
(a) incuber une fusion d'une protéine cible et d'un rapporteur bioluminescent en présence d'un ligand pour produire un échantillon d'essai ;
(b) mettre en contact l'échantillon d'essai avec un colorant hydrophobe qui se lie de manière non spécifique à des protéines agrégées, dépliées, ou dénaturées et/ou à des segments peptidiques hydrophobes, dans lequel le spectre d'émission du rapporteur bioluminescent lors de l'interaction avec un substrat et le spectre d'excitation du colorant hydrophobe qui se lie de manière non spécifique à des protéines agrégées, dépliées, ou dénaturées et/ou à des segments peptidiques hydrophobes se chevauchent ;
(c) traiter l'échantillon d'essai dans des conditions qui provoquent le dépliage de la protéine cible dans une mesure appropriée ;
(d) exposer l'échantillon d'essai au substrat du rapporteur bioluminescent ;
(e) mesurer un signal provenant du colorant hydrophobe qui se lie de manière non spécifique à des protéines agrégées, dépliées, ou dénaturées et/ou à des segments peptidiques hydrophobes et du rapporteur bioluminescent dans ledit échantillon d'essai ; et
(f) détecter une interaction cible/ligand sur la base des signaux provenant du colorant hydrophobe qui se lie de manière non spécifique à des protéines agrégées, dépliées, ou dénaturées et/ou à des segments peptidiques hydrophobes et du rapporteur bioluminescent dans l'échantillon d'essai, et dans lequel la différence ou le rapport du signal est comparé à une différence ou un rapport de signal dans un échantillon témoin sans le ligand,
et dans lequel le rapporteur bioluminescent a une identité de séquence de 70 % ou plus avec la SEQ ID NO : 1.

13. Procédé de détection d'une interaction cible/ligand à l'intérieur d'une cellule vivante et intacte, comprenant les étapes consistant à :
(a) fournir une cellule vivante et intacte exprimant une fusion d'une protéine cible et d'un rapporteur bioluminescent ;
(b) laisser du temps à la protéine cible pour interagir avec le ligand à l'intérieur de la cellule vivante et intacte ;
(c)(i)
(A) mettre en contact la cellule vivante et intacte avec un colorant hydrophobe qui se lie de manière non spécifique à des protéines agrégées, dépliées, ou dénaturées et/ou à des segments peptidiques hydrophobes, dans lequel le spectre d'émission du rapporteur bioluminescent lors de l'interaction avec un substrat et le spectre d'excitation du colorant hydrophobe qui se lie de manière non spécifique à des protéines agrégées, dépliées, ou dénaturées et/ou à des segments peptidiques hydrophobes se chevauchent ;
ou
(c)(ii)
(A) lyser la cellule pour produire un lysat cellulaire ;
(B) mettre en contact le lysat cellulaire avec un colorant hydrophobe qui se lie de manière non spécifique à des protéines agrégées, dépliées, ou dénaturées et/ou à des segments peptidiques hydrophobes, dans lequel le spectre d'émission du rapporteur bioluminescent lors de l'interaction avec un substrat et le spectre d'excitation du colorant hydrophobe qui se lie de manière non spécifique à des protéines agrégées, dépliées, ou dénaturées et/ou à des segments peptidiques hydrophobes se chevauchent ;
ou
(c)(iii)
(A) mettre en contact la cellule vivante et intacte avec un colorant hydrophobe qui se lie de manière non spécifique à des protéines agrégées, dépliées, ou dénaturées et/ou à des segments peptidiques hydrophobes, dans lequel le spectre d'émission du rapporteur bioluminescent lors de l'interaction avec un substrat et le spectre d'excitation du colorant hydrophobe qui se lie de manière non spécifique à des protéines agrégées, dépliées, ou dénaturées et/ou à des segments peptidiques hydrophobes se chevauchent ;
(B) lyser la cellule pour produire un lysat cellulaire ;
(d) traiter le lysat cellulaire dans des conditions qui provoquent le dépliage de la protéine cible dans une mesure appropriée ;
(e) exposer le lysat cellulaire au substrat du rapporteur bioluminescent ;
(f) mesurer un signal provenant du colorant hydrophobe qui se lie de manière non spécifique à des protéines agrégées, dépliées, ou dénaturées et/ou à des segments peptidiques hydrophobes et du rapporteur bioluminescent dans le lysat cellulaire ; et
(g) détecter une interaction cible/ligand sur la base de signaux provenant dudit colorant hydrophobe qui se lie de manière non spécifique à des protéines agrégées, dépliées, ou dénaturées et/ou à des segments peptidiques hydrophobes et du rapporteur bioluminescent dans le lysat cellulaire, dans lequel le signal dudit colorant hydrophobe qui se lie de manière non spécifique à des protéines agrégées, dépliées, ou dénaturées et/ou à des segments peptidiques hydrophobes dans le lysat cellulaire est comparé à un échantillon témoin sans le ligand ; comprenant en outre facultativement une étape d'ajout du ligand à la cellule vivante et intacte,
et dans lequel le rapporteur bioluminescent a une identité de séquence de 70 % ou plus avec la SEQ ID NO : 1.

14. Procédé de détection d'une interaction cible/ligand à l'intérieur d'une cellule vivante et intacte comprenant les étapes consistant à :
(a) fournir une cellule vivante et intacte exprimant une fusion d'une protéine cible et d'un rapporteur bioluminescent ;
(b)(i)
(A) laisser du temps à la protéine cible pour interagir avec le ligand à l'intérieur de la cellule vivante et intacte ;
(B) traiter la cellule vivante et intacte dans des conditions qui provoquent le dépliage de la protéine cible dans une mesure appropriée ;
ou
(b)(ii)
(A) laisser du temps à la protéine cible pour interagir avec le ligand à l'intérieur de la cellule vivante et intacte ;
(B) traiter la cellule vivante et intacte dans des conditions qui provoquent le dépliage de la protéine cible dans une mesure appropriée ;
(C) lyser la cellule pour produire un lysat cellulaire
ou
(b)(iii)
(A) lyser la cellule pour produire un lysat cellulaire
(B) laisser du temps à la protéine cible pour interagir avec le ligand à l'intérieur du lysat cellulaire ;
(C) traiter le lysat cellulaire dans des conditions qui provoquent le dépliage de la protéine cible dans une mesure appropriée ;
(c) exposer la cellule vivante et intacte ou le lysat cellulaire au substrat du rapporteur bioluminescent ;
(d) mesurer un signal provenant du rapporteur bioluminescent dans la cellule vivante et intacte ou le lysat cellulaire ; et
(e) détecter une interaction cible/ligand sur la base du signal provenant dudit rapporteur bioluminescent dans la cellule vivante et intacte ou le lysat cellulaire, et dans lequel la différence ou le rapport du signal est comparé à une différence ou un rapport de signal dans un échantillon témoin sans le ligand,
et dans lequel le rapporteur bioluminescent a une identité de séquence de 70 % ou plus avec la SEQ ID NO : 1.

15. Procédé de détection d'une interaction cible/ligand à l'intérieur d'une cellule vivante et intacte comprenant les étapes consistant à :
(a) fournir une cellule vivante et intacte exprimant une fusion d'une protéine cible et d'un rapporteur bioluminescent ;
(b) lyser facultativement la cellule pour produire un lysat cellulaire
(c) laisser du temps à la protéine cible pour interagir avec le ligand à l'intérieur de la cellule vivante et intacte ou du lysat cellulaire ;
(d) exposer la cellule vivante et intacte ou le lysat cellulaire au substrat du rapporteur bioluminescent ;
(e) mesurer un signal provenant du rapporteur bioluminescent dans la cellule vivante et intacte ou le lysat cellulaire dans des conditions pré-dénaturantes ;
(f) traiter la cellule vivante et intacte ou le lysat cellulaire dans des conditions qui provoquent le dépliage de la protéine cible dans une mesure appropriée ;
(g) mesurer un signal provenant du rapporteur bioluminescent dans la cellule vivante et intacte ou le lysat cellulaire dans des conditions post-dénaturantes ; et
(h) détecter une interaction cible/ligand sur la base de la différence ou du rapport de signal provenant du rapporteur bioluminescent dans les conditions pré-dénaturantes et post-dénaturantes dans la cellule vivante et intacte ou le lysat cellulaire, et dans lequel la différence ou le rapport du signal est comparé à une différence ou un rapport de signal dans un échantillon témoin sans le ligand,
et dans lequel le rapporteur bioluminescent a une identité de séquence de 70 % ou plus avec la SEQ ID NO : 1.

16. Procédé de détection d'une interaction cible/ligand à l'intérieur d'une cellule vivante et intacte, comprenant les étapes consistant à :
(a) fournir une cellule vivante et intacte exprimant une fusion d'une protéine cible et d'un rapporteur bioluminescent ;
(b)(i)
(A) laisser du temps à la protéine cible pour interagir avec le ligand à l'intérieur de la cellule vivante et intacte ;
(B) traiter la cellule vivante et intacte dans des conditions qui provoquent le dépliage de la protéine cible dans une mesure appropriée ;
(C) mettre en contact la cellule vivante et intacte avec un colorant hydrophobe qui se lie de manière non spécifique à des protéines agrégées, dépliées, ou dénaturées et/ou à des segments peptidiques hydrophobes, dans lequel le spectre d'émission du rapporteur bioluminescent lors de l'interaction avec un substrat et le spectre d'excitation du colorant hydrophobe qui se lie de manière non spécifique à des protéines agrégées, dépliées, ou dénaturées et/ou à des segments peptidiques hydrophobes se chevauchent ;
(D) exposer la cellule vivante et intacte ou le lysat cellulaire au substrat du rapporteur bioluminescent ;
ou
(b)(ii)
(A) laisser du temps à la protéine cible pour interagir avec le ligand à l'intérieur de la cellule vivante et intacte ;
(B) traiter la cellule vivante et intacte dans des conditions qui provoquent le dépliage de la protéine cible dans une mesure appropriée ;
(C) lyser la cellule pour produire un lysat cellulaire
(D) mettre en contact le lysat cellulaire avec un colorant hydrophobe qui se lie de manière non spécifique à des protéines agrégées, dépliées, ou dénaturées et/ou à des segments peptidiques hydrophobes, dans lequel le spectre d'émission du rapporteur bioluminescent lors de l'interaction avec un substrat et le spectre d'excitation du colorant hydrophobe sensible à l'environnement se chevauchent ;
(E) exposer la cellule vivante et intacte ou le lysat cellulaire au substrat du rapporteur bioluminescent ;
ou
(b)(iii)
(A) lyser la cellule pour produire un lysat cellulaire
(B) mettre en contact le lysat cellulaire avec un colorant hydrophobe qui se lie de manière non spécifique à des protéines agrégées, dépliées, ou dénaturées et/ou à des segments peptidiques hydrophobes, dans lequel le spectre d'émission du rapporteur bioluminescent lors de l'interaction avec un substrat et le spectre d'excitation du colorant hydrophobe qui se lie de manière non spécifique à des protéines agrégées, dépliées, ou dénaturées et/ou à des segments peptidiques hydrophobes se chevauchent ;
(C) laisser du temps à la protéine cible pour interagir avec le ligand à l'intérieur du lysat cellulaire ;
(D) traiter le lysat cellulaire dans des conditions qui provoquent le dépliage de la protéine cible dans une mesure appropriée ;
(E) exposer la cellule vivante et intacte ou le lysat cellulaire au substrat du rapporteur bioluminescent ;
ou
(b)(iv)
(A) laisser du temps à la protéine cible pour interagir avec le ligand à l'intérieur de la cellule vivante et intacte ;
(B) mettre en contact la cellule vivante et intacte avec un colorant hydrophobe qui se lie de manière non spécifique à des protéines agrégées, dépliées, ou dénaturées et/ou à des segments peptidiques hydrophobes, dans lequel le spectre d'émission du rapporteur bioluminescent lors de l'interaction avec un substrat et le spectre d'excitation du colorant hydrophobe qui se lie de manière non spécifique à des protéines agrégées, dépliées, ou dénaturées et/ou à des segments peptidiques hydrophobes se chevauchent ;
(C) exposer la cellule vivante et intacte au substrat du rapporteur bioluminescent ;
(D) mesurer le signal provenant du colorant hydrophobe qui se lie de manière non spécifique à des protéines agrégées, dépliées, ou dénaturées et/ou à des segments peptidiques hydrophobes dans la cellule vivante et intacte dans des conditions pré-dénaturantes ;
(E) traiter la cellule vivante et intacte dans des conditions qui provoquent le dépliage de la protéine cible dans une mesure appropriée ;
comprenant facultativement en outre une étape d'ajout du ligand à la cellule vivante et intacte avant l'étape (D) ;
(c) mesurer un signal provenant du colorant hydrophobe qui se lie de manière non spécifique à des protéines agrégées, dépliées, ou dénaturées et/ou à des segments peptidiques hydrophobes et du rapporteur bioluminescent dans la cellule vivante et intacte ou le lysat cellulaire ; et
(d)(i) lorsque les étapes (b)(i), (b)(ii) ou (b)(iii) sont effectuées, détecter une interaction cible/ligand sur la base du signal ratiométrique provenant dudit colorant hydrophobe et du rapporteur bioluminescent dans la cellule vivante et intacte ou le lysat cellulaire, dans lequel le colorant hydrophobe se lie de manière non spécifique à des protéines agrégées, dépliées, ou dénaturées et/ou à des segments peptidiques hydrophobes ;
ou
(d)(ii) lorsque l'étape (b)(iv) est effectuée, détecter une interaction cible/ligand sur la base de la différence ou du rapport de signaux provenant dudit colorant hydrophobe et du rapporteur bioluminescent dans les conditions pré-dénaturantes et post-dénaturantes dans la cellule vivante et intacte, dans lequel le colorant hydrophobe se lie de manière non spécifique à des protéines agrégées, dépliées, ou dénaturées et/ou à des segments peptidiques hydrophobes, et dans lequel la différence ou le rapport du signal est comparé à une différence ou un rapport de signal dans un échantillon témoin sans le ligand,
et dans lequel le rapporteur bioluminescent a une identité de séquence de 70 % ou plus avec la SEQ ID NO : 1.

17. Procédé selon la revendication 13 ou 16, dans lequel le ligand est endogène à la cellule vivante et intacte.
